# EUROPEAN PATENT SPECIFICATION

(11) **EP 4 242 324 B1**
(45) Date of publication and mention of the grant of the patent: **24.09.2025**
(21) Application number: 23166391.5
(22) Date of filing: 14.08.2019
(51) Int. Cl.: C12Q 1/6804, C12Q 1/6809

(54) **APTAMER BARCODING**
APTAMER-STRICHCODIERUNG
CODAGE À BARRES PAR APTAMÈRES

(30) Priority: 17.08.2018 US 201862719406 P
(43) Date of publication of application: 13.09.2023
(62) Divisional of application: 19762517.1
(73) Proprietor: Becton, Dickinson and Company, Franklin Lakes, NJ 07417-1880 (US)
(72) Inventor: CHANG, Christina, San Jose, 95131 (US); SHUM, Eleen, San Jose, 95131 (US); LI, Sixing, San Jose, 95131 (US); NAKAMOTO, Margaret, San Jose, 95131 (US)
(74) Representative: Carpmaels & Ransford LLP

(56) References cited:
- WO-A1-2013/137737
- WO-A1-2015/017586
- WO-A1-2018/226293
- WO-A2-2018/058073
- US-A1- 2018 208 975
- CYRILLE L DELLEY ET AL: "Combined aptamer and transcriptome sequencing of single cells", BIORXIV, 3 December 2017 (2017-12-03), XP055445009, Retrieved from the Internet <URL:https://www.biorxiv.org/content/biorxiv/early/2017/12/03/228338.full.pdf> DOI: 10.1101/228338

## Description

### RELATED APPLICATIONS

This application claims the benefit under 35 U.S.C. §119(e) of U.S. Provisional Patent Application Ser. 62/719,406, filed August 17, 2018.

### REFERENCE TO SEQUENCE LISTING

The present application is being filed along with a Sequence Listing in electronic format. The Sequence Listing is provided as a file entitled SequenceListing, created August 2, 2019, which is 4 kilobytes in size..

### BACKGROUND

### Field

The present disclosure relates generally to the field of molecular biology, for example identifying cells of different samples and determining protein expression profiles in cells using molecular barcoding.

### Description of the Related Art

Current technology allows measurement of gene expression of single cells in a massively parallel manner (e.g., >10000 cells) by attaching cell specific oligonucleotide barcodes to poly(A) mRNA molecules from individual cells as each of the cells is co-localized with a barcoded reagent bead in a compartment. Gene expression may affect protein expression. Protein-protein interaction may affect gene expression and protein expression. There is a need for systems and methods that can quantitatively analyze protein expression in cells, and simultaneously measure protein expression and gene expression in cells. Delley *et al.* (2017) describes a method to simultaneously characterize the transcriptome and proteome of single cells. US 2018/208975 A1 describes an assay for simultaneous genomic and proteomic analysis. WO 2018/226293 A1 describes systems, methods, compositions, and kits for sample identification using protein binding reagents associated with a sample indexing oligonucleotide. WO 2018/058073 A2 describes measurement of protein expression using reagents with barcoded oligonucleotide sequences. WO 2013/137737 A1 describes methods of creating and screening of DNA combinatorial libraries of chemical antibodies. WO 2015/017586 describes quantitative DNA-based imaging and super-resolution imaging.

### SUMMARY

The present invention is set out in the appended set of claims. The terminologies "embodiment" and "embodiments" are to be construed as embodiment(s) of the invention only in as far as they fall within the scope of the appended claims. Otherwise, they refer to embodiments of the disclosure only, for reference purposes. Disclosed herein include embodiments of a method for sample identification. In some embodiments, the method comprises: contacting each of a plurality of samples with a sample indexing composition of a plurality of sample indexing compositions, respectively, wherein each of the plurality of samples comprises one or more cells each comprising one or more protein targets, wherein the sample indexing composition comprises an aptamer composition comprising an aptamer and a sample indexing oligonucleotide, wherein the aptamer is capable of specifically binding to at least one of the one or more protein targets, wherein the sample indexing oligonucleotide comprises a sample indexing sequence, and wherein sample indexing sequences of at least two sample indexing compositions of the plurality of sample indexing compositions comprise different sequences; barcoding the sample indexing oligonucleotides using a plurality of barcodes to generate a plurality of barcoded sample indexing oligonucleotides; obtaining sequencing data of the plurality of barcoded sample indexing oligonucleotides; and identifying sample origin of at least one cell of the one or more cells based on the sample indexing sequence of at least one barcoded sample indexing oligonucleotide of the plurality of barcoded sample indexing oligonucleotides.

In some embodiments, the method comprises: contacting each of a plurality of samples with a sample indexing composition of a plurality of sample indexing compositions, respectively, wherein each of the plurality of samples comprises one or more cells each comprising one or more cellular component targets, wherein the sample indexing composition comprises an aptamer composition comprising an aptamer and a sample indexing oligonucleotide, wherein the aptamer is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; and identifying sample origin of at least one cell of the one or more cells based on the sample indexing sequence of at least one sample indexing oligonucleotide of the plurality of sample indexing compositions. In some embodiments, the cellular component target comprises a protein target. In some embodiments, identifying the sample origin of the at least one cell comprises: barcoding sample indexing oligonucleotides of the plurality of sample indexing compositions using a plurality of barcodes to generate a plurality of barcoded sample indexing oligonucleotides; obtaining sequencing data of the plurality of barcoded sample indexing oligonucleotides; and identifying the sample origin of the cell based on the sample indexing sequence of at least one barcoded sample indexing oligonucleotide of the plurality of barcoded sample indexing oligonucleotides in the sequencing data.

In some embodiments, identifying the sample origin of the at least one cell comprises identifying the presence or absence of the sample indexing sequence of at least one sample indexing oligonucleotide of the plurality of sample indexing compositions. Identifying the presence or absence of the sample indexing sequence can comprise: replicating the at least one sample indexing oligonucleotide to generate a plurality of replicated sample indexing oligonucleotides; obtaining sequencing data of the plurality of replicated sample indexing oligonucleotides; and identifying the sample origin of the cell based on the sample indexing sequence of a replicated sample indexing oligonucleotide of the plurality of sample indexing oligonucleotides that correspond to the least one barcoded sample indexing oligonucleotide in the sequencing data.

In some embodiments, replicating the at least one sample indexing oligonucleotide to generate the plurality of replicated sample indexing oligonucleotides comprises: prior to replicating the at least one barcoded sample indexing oligonucleotide, ligating a replicating adaptor to the at least one barcoded sample indexing oligonucleotide, and wherein replicating the at least one barcoded sample indexing oligonucleotide comprises replicating the at least one barcoded sample indexing oligonucleotide using the replicating adaptor ligated to the at least one barcoded sample indexing oligonucleotide to generate the plurality of replicated sample indexing oligonucleotides. In some embodiments, replicating the at least one sample indexing oligonucleotide to generate the plurality of replicated sample indexing oligonucleotides comprises: prior to replicating the at least one barcoded sample indexing oligonucleotide, contacting a capture probe with the at least one sample indexing oligonucleotide to generate a capture probe hybridized to the sample indexing oligonucleotide; and extending the capture probe hybridized to the sample indexing oligonucleotide to generate a sample indexing oligonucleotide associated with the capture probe. Replicating the at least one sample indexing oligonucleotide can comprise replicating the sample indexing oligonucleotide associated with the capture probe to generate the plurality of replicated sample indexing oligonucleotides.

In some embodiments, the sample indexing sequence is 6-60 nucleotides in length. The sample indexing oligonucleotide can be 50-500 nucleotides in length. Sample indexing sequences of at least 10, 100, or 1000 sample indexing compositions of the plurality of sample indexing compositions can comprise different sequences.

In some embodiments, a single polynucleotide comprises the sample indexing oligonucleotide and the aptamer. The aptamer can be 5' to the sample indexing oligonucleotide in the single polynucleotide. The aptamer can 3' to the sample indexing oligonucleotide in the single polynucleotide. The sample indexing oligonucleotide can be associated with the aptamer. The sample indexing oligonucleotide can be attached to the aptamer. The sample indexing oligonucleotide can be covalently attached to the aptamer. The sample indexing oligonucleotide can be conjugated to the aptamer. The sample indexing oligonucleotide can be conjugated to the aptamer through a chemical group selected from the group consisting of a UV photocleavable group, a streptavidin, a biotin, an amine, and a combination thereof. In some embodiments, the sample indexing oligonucleotide can be non-covalently attached to the aptamer. The sample indexing oligonucleotide can be associated with the aptamer through a linker.

In some embodiments, the aptamer comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The aptamer can comprise a peptide aptamer.

In some embodiments, the sample indexing composition can be associated with a first carrier. Different sample indexing compositions of the plurality of sample indexing compositions can be associated with different first carriers.

In some embodiments, the aptamer composition can be associated with a first carrier. The aptamer composition can comprise: a second aptamer capable of specifically binding to at least one of the one or more protein targets or one or more cellular component targets, and a second sample indexing oligonucleotide comprising a second sample indexing sequence. The aptamer and the second aptamer can be associated with a first carrier. The aptamer can be associated with a first carrier and the second aptamer is associated with a second carrier. The aptamer and the second aptamer can be at least 60%, 70%, 80%, 90%, or 95% identical in sequence. The aptamer and the second aptamer can be identical, for example, in sequence. The aptamer and the second aptamer can be different. The protein targets, or the cellular component targets, of the aptamer and the second aptamer can be identical. The aptamer and the second aptamer can be capable of binding to different regions of a protein target, or a cellular component target. The protein targets, or the cellular component targets, of the aptamer and the second aptamer can be different.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the aptamer can be attached to the first carrier. The aptamer can be covalently attached to the first carrier. The aptamer can be conjugated to the first carrier. The aptamer can be conjugated to the first carrier through a chemical group selected from the group consisting of a UV photocleavable group, a streptavidin, a biotin, an amine, and a combination thereof. The aptamer can be non-covalently linked to the first carrier. The aptamer can be associated with the first carrier through a linker. The aptamer can be immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the method comprises dissociating the aptamer from the first carrier. The dissociating can occur after barcoding the sample indexing oligonucleotides. The dissociating can occur before barcoding the sample indexing oligonucleotides.

In some embodiments, contacting each of the plurality of samples with the sample indexing composition comprises contacting a cell of the one or more cells of the sample with the sample indexing composition. The first carrier can be internalized into the cell. The first carrier can be internalized into the cell via endocytosis, pinocytosis, nanopinocytosis, micropinocytosis, phagocytosis, membrane fusion, or a combination thereof. The first carrier can be internalized into the cell via clathrin-medicated internalization, caveolin-mediated internalization, receptor dependent internalization, receptor independent internalization or a combination thereof.

In some embodiments, the method comprises removing unbound sample indexing compositions of the plurality of sample indexing compositions. Removing the unbound sample indexing compositions can comprise washing the one or more cells from each of the plurality of samples with a washing buffer. Removing the unbound sample indexing compositions can comprise selecting cells bound to at least one aptamer using flow cytometry. In some embodiments, the method comprises lysing the one or more cells from each of the plurality of samples.

In some embodiments, the sample indexing oligonucleotide is configured to be (or can be) non-detachable from the aptamer. The sample indexing oligonucleotide can be configured to be (or can be) detachable from the aptamer. The method can comprise detaching the sample indexing oligonucleotide from the aptamer. Detaching the sample indexing oligonucleotide can comprise detaching the sample indexing oligonucleotide from the aptamer by UV photocleaving, chemical treatment, heating, enzyme treatment, or any combination thereof.

In some embodiments, the sample indexing oligonucleotide is not homologous to genomic sequences of any of the one or more cells, is homologous to genomic sequences of a species, or a combination thereof. The species can be a non-mammalian species.

In some embodiments, a sample of the plurality of samples comprises a plurality of cells, a plurality of single cells, a tissue, a tumor sample, or any combination thereof. The plurality of samples can comprise a mammalian cell, a bacterial cell, a viral cell, a yeast cell, a fungal cell, or any combination thereof.

In some embodiments, the sample indexing oligonucleotide comprises a sequence complementary to a capture sequence configured to capture (or capable of capturing, hybridizing to, or binding to) the sequence of the sample indexing oligonucleotide. The barcode can comprise a target-binding region which comprises the capture sequence. The target-binding region can comprise a poly(dT) region. The sequence of the sample indexing oligonucleotide complementary to the capture sequence can comprise a poly(dA) region.

In some embodiments, the sample indexing oligonucleotide comprises an alignment sequence adjacent to the poly(dA) region. The alignment sequence can be 1 or more nucleotides in length. The alignment sequence can be 2 or more nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof. The sample indexing oligonucleotide can comprise a molecular label sequence, a binding site for a universal primer, or both. The molecular label sequence can be 2-20 nucleotides in length. The universal primer can be 5-50 nucleotides in length. The universal primer can comprise an amplification primer, a sequencing primer, or a combination thereof.

In some embodiments, the aptamer is associated with two or more sample indexing oligonucleotides with an identical sequence. The aptamer can be associated with two or more sample indexing oligonucleotides with different sample indexing sequences. In some embodiments, the sample indexing composition of the plurality of sample indexing compositions comprises a second aptamer not associated with the sample indexing oligonucleotide. The aptamer and the second aptamer can be identical. In some embodiments, each of the plurality of sample indexing compositions comprises the aptamer.

In some embodiments, the sample indexing composition of the plurality of sample indexing compositions comprises a second aptamer composition comprising a second protein-binding aptamer, and the second aptamer is capable of specifically binding to at least one of the one or more protein targets, or to at least one of the one or more cellular component targets. The aptamer and the second aptamer can be capable of binding to the same protein target of the one or more protein targets, or the one or more cellular component targets, and wherein the second aptamer is not associated with the sample indexing oligonucleotide. The second aptamer can be associated with a second sample indexing oligonucleotide comprising a second sample indexing sequence. The aptamer and the second aptamer can be at least 60%, 70%, 80%, 90%, or 95% identical in sequence. The aptamer and the second aptamer can be identical, for example, in sequence. The aptamer and the second aptamer can be different. The aptamer and the second aptamer can be capable of binding to different regions of the same protein target, or different regions of the same cellular component target. The aptamer and the second aptamer can be capable of binding to different protein targets of the one or more protein targets, or different cellular component targets of the one or more cellular component targets. The sample indexing sequence and the second sample indexing sequence can be identical. The sample indexing sequence and the second sample indexing sequence can be different.

In some embodiments, the method comprises: prior to barcoding the sample indexing oligonucleotides, pooling the plurality of samples contacted with the plurality of sample indexing compositions.

In sine embodiments, a barcode of the plurality of barcodes comprises a target-binding region and a molecular label sequence, and molecular label sequences of at least two barcodes of the plurality of barcodes comprise different molecule label sequences. The barcode can comprise a cell label sequence, a binding site for a universal primer, or any combination thereof. The target-binding region can comprise a poly(dT) region.

In some embodiments, the plurality of barcodes is associated with a particle. At least one barcode of the plurality of barcodes can be immobilized on the particle, partially immobilized on the particle, enclosed in the particle, partially enclosed in the particle, or a combination thereof. The particle can be disruptable. The particle can comprise a bead. The particle can comprise a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, a hydrogel bead, an anti-biotin microbead, an anti-fluorochrome microbead, or any combination thereof, or wherein the particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof. The particle can comprise a disruptable hydrogel bead.

In some embodiments, the barcodes of the particle comprise molecular label sequences selected from at least 1000, 10000, or a combination thereof, different molecular label sequences. The molecular label sequences of the barcodes can comprise random sequences. The particle can comprise at least 10000 barcodes.

In some embodiments, barcoding the sample indexing oligonucleotides using the plurality of barcodes comprises: contacting the plurality of barcodes with the sample indexing oligonucleotides to generate barcodes hybridized to the sample indexing oligonucleotides; and extending the barcodes hybridized to the sample indexing oligonucleotides to generate the plurality of barcoded sample indexing oligonucleotides.

In some embodiments, the method comprises, prior to extending the barcodes hybridized to the sample indexing oligonucleotides, pooling the barcodes hybridized to the sample indexing oligonucleotides, and wherein extending the barcodes hybridized to the sample indexing oligonucleotides comprises extending the pooled barcodes hybridized to the sample indexing oligonucleotides to generated a plurality of pooled barcoded sample indexing oligonucleotides. Extending the barcodes can comprise extending the barcodes using a DNA polymerase to generate the plurality of barcoded sample indexing oligonucleotides. Extending the barcodes can comprise extending the barcodes using a reverse transcriptase to generate the plurality of barcoded sample indexing oligonucleotides.

In some embodiments, the method comprises: amplifying the plurality of barcoded sample indexing oligonucleotides to produce a plurality of amplicons. Amplifying the plurality of barcoded sample indexing oligonucleotides can comprise amplifying, using polymerase chain reaction (PCR), at least a portion of the molecular label sequence and at least a portion of the sample indexing oligonucleotide. Obtaining the sequencing data of the plurality of barcoded sample indexing oligonucleotides can comprise obtaining sequencing data of the plurality of amplicons. Obtaining the sequencing data can comprise sequencing at least a portion of the molecular label sequence and at least a portion of the sample indexing oligonucleotide.

In some embodiments, barcoding the sample indexing oligonucleotides using the plurality of barcodes to generate the plurality of barcoded sample indexing oligonucleotides comprises stochastically barcoding the sample indexing oligonucleotides using a plurality of stochastic barcodes to generate a plurality of stochastically barcoded sample indexing oligonucleotides.

In some embodiments, the method comprises: barcoding a plurality of targets of the cell using the plurality of barcodes to generate a plurality of barcoded targets, wherein each of the plurality of barcodes comprises a cell label sequence, and wherein at least two barcodes of the plurality of barcodes comprise an identical cell label sequence; and obtaining sequencing data of the barcoded targets. Barcoding the plurality of targets using the plurality of barcodes to generate the plurality of barcoded targets can comprise: contacting copies of the targets with target-binding regions of the barcodes; and reverse transcribing the plurality targets using the plurality of barcodes to generate a plurality of reverse transcribed targets. The method can comprise: prior to obtaining the sequencing data of the plurality of barcoded targets, amplifying the barcoded targets to generate a plurality of amplified barcoded targets. Amplifying the barcoded targets to generate the plurality of amplified barcoded targets can comprise: amplifying the barcoded targets by polymerase chain reaction (PCR). Barcoding the plurality of targets of the cell using the plurality of barcodes to generate the plurality of barcoded targets can comprise stochastically barcoding the plurality of targets of the cell using a plurality of stochastic barcodes to generate a plurality of stochastically barcoded targets.

Disclosed herein are embodiments of a plurality of sample indexing compositions. In some embodiments, each of the plurality of sample indexing compositions comprises an aptamer composition comprising a first cellular component-binding aptamer and a sample indexing oligonucleotide, the cellular component-binding aptamer is capable of specifically binding to at least one cellular component target, the sample indexing oligonucleotide comprises a sample indexing sequence for identifying sample origin of one or more cells of a sample, and sample indexing sequences of at least two sample indexing compositions of the plurality of sample indexing compositions comprise different sequences.

In some embodiments, the sample indexing sequence is 6-60 nucleotides in length. The sample indexing oligonucleotide can be 50-500 nucleotides in length. Sample indexing sequences of at least 10, 100, or 1000 sample indexing compositions of the plurality of sample indexing compositions can comprise different sequences.

In some embodiments, a single polynucleotide comprises the sample indexing oligonucleotide and the cellular component-binding aptamer. The aptamer can be 5' to the sample indexing oligonucleotide in the single polynucleotide. The aptamer can be 3' to the sample indexing oligonucleotide in the single polynucleotide.

In some embodiment, the sample indexing oligonucleotide is associated with the aptamer. The sample indexing oligonucleotide can be attached to the cellular component-binding aptamer. The sample indexing oligonucleotide can be covalently attached to the cellular component-binding aptamer. The sample indexing oligonucleotide can be conjugated to the cellular component-binding aptamer. The sample indexing oligonucleotide can be conjugated to the cellular component-binding aptamer through a chemical group selected from the group consisting of a UV photocleavable group, a streptavidin, a biotin, an amine, and a combination thereof. The sample indexing oligonucleotide can be non-covalently attached the cellular component-binding aptamer. The sample indexing oligonucleotide can be associated with the protein-binding aptamer, or the cellular component-binding aptamer, through a linker.

In some embodiments, the protein-binding aptamer, or the cellular component-binding aptamer, comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The cellular component-binding aptamer can comprise a peptide aptamer.

In some embodiments, the sample indexing composition is associated with a first carrier. Different sample indexing compositions of the plurality of sample indexing compositions can be associated with different first carriers. In some embodiments, the aptamer composition is associated with a first carrier.

In some embodiments, the aptamer composition comprises: a second cellular component-binding aptamer capable of specifically binding to at least one of the one or more protein targets or one or more cellular component targets, and a second sample indexing oligonucleotide comprising a second sample indexing sequence. The cellular component-binding aptamer and the second cellular component-binding aptamer can be associated with a first carrier. The cellular component-binding aptamer can be associated with a first carrier, and the second cellular component-binding aptamer can be associated with a second carrier.

In some embodiments, the cellular component-binding aptamer and the second cellular component-binding aptamer have at least 60%, 70%, 80%, 90%, or 95% sequence identity. The cellular component-binding aptamer and the second cellular component-binding aptamer can be identical, for example, in sequence. The cellular component-binding aptamer and the second cellular component-binding aptamer can be different.

In some embodiments, the protein targets, or the cellular component targets, of the cellular component-binding aptamer and the second cellular component-binding aptamer are identical. The cellular component-binding aptamer and the second cellular component-binding aptamer can be capable of binding to different regions of a protein target, or a cellular component target. The protein targets, or the cellular component targets, of the cellular component-binding aptamer and the second cellular component-binding aptamer can be different.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the cellular component-binding aptamer is attached to the first carrier. The cellular component-binding aptamer can be covalently attached to the first carrier. The cellular component-binding aptamer can be conjugated to the first carrier. The cellular component-binding aptamer can be conjugated to the first carrier through a chemical group selected from the group consisting of a UV photocleavable group, a streptavidin, a biotin, an amine, and a combination thereof. The cellular component-binding aptamer can be non-covalently linked to the first carrier. The cellular component-binding aptamer can be associated with the first carrier through a linker.

In some embodiments, the cellular component-binding aptamer is immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the first carrier is configured to be internalized (or capable of being internalized) into the cell. The first carrier can be internalized into the cell via endocytosis, pinocytosis, nanopinocytosis, micropinocytosis, phagocytosis, membrane fusion, or a combination thereof. The first carrier can be internalized into the cell via clathrin-medicated internalization, caveolin-mediated internalization, receptor dependent internalization, receptor independent internalization or a combination thereof.

In some embodiments, the sample indexing oligonucleotide is not homologous to genomic sequences of any of the one or more cells. At least one sample of the plurality of samples can comprise one or more single cells, a plurality of cells, a tissue, a tumor sample, or any combination thereof. The sample can comprise a mammalian sample, a bacterial sample, a viral sample, a yeast sample, a fungal sample, or any combination thereof.

In some embodiments, the sample indexing oligonucleotide comprises a sequence complementary to a capture sequence configured to capture (or capable of capturing, hybridizing to, or binding to) the sequence of the sample indexing oligonucleotide. The target-binding region can comprise a poly(dT) region. The sequence of the sample indexing oligonucleotide complementary to the capture sequence can comprise a poly(dA) region.

In some embodiments, the sample indexing oligonucleotide comprises an alignment sequence adjacent to the poly(dA) region. The alignment sequence can be or more nucleotides in length. The alignment sequence can be 2 or more nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof.

In some embodiments, the sample indexing oligonucleotide comprises a molecular label sequence, a poly(dA) region, or a combination thereof. The molecular label sequence can be 2-20 nucleotides in length. The universal primer can be 5-50 nucleotides in length. The universal primer can comprise an amplification primer, a sequencing primer, or a combination thereof.

In some embodiments, the cellular component target comprises a cell-surface protein, a cell marker, a B-cell receptor, a T-cell receptor, a major histocompatibility complex, a tumor antigen, a receptor, or any combination thereof. The cellular component target can be selected from a group comprising 10-100 different cellular component targets. In some embodiments, the at least one of the one or more protein targets, or the one or more cellular component targets, is on a cell surface. In some embodiments, the protein target, or the cellular component target, comprises a carbohydrate, a lipid, a protein, an extracellular protein, a cell-surface protein, a cell marker, a B-cell receptor, a T-cell receptor, a major histocompatibility complex, a tumor antigen, a receptor, an intracellular protein, or any combination thereof. The protein target, or the cellular component target, can be selected from a group comprising 10-100 different protein targets, or cellular component targets.

In some embodiments, the cellular component-binding aptamer is associated with two or more sample indexing oligonucleotides with an identical sequence. The cellular component-binding aptamer can be associated with two or more sample indexing oligonucleotides with different sample indexing sequences.

In some embodiments, the sample indexing composition comprises a second cellular component-binding aptamer, and the second cellular component-binding aptamer is capable of specifically binding to at least one of the one or more cellular component targets.

The cellular component-binding aptamer and the second cellular component-binding aptamer can be capable of binding to the same cellular component target of the one or more cellular component targets, and the second cellular component-binding aptamer can be not associated with the sample indexing oligonucleotide. The second cellular component-binding aptamer can be associated with a second sample indexing oligonucleotide comprising a second sample indexing sequence. The sample indexing sequence and the second sample indexing sequence can be identical. The sample indexing sequence and the second sample indexing sequence can be different.

In some embodiments, the cellular component-binding aptamer and the second cellular component-binding aptamer are at least 60%, 70%, 80%, 90%, or 95% identical. The cellular component-binding aptamer and the second cellular component-binding aptamer can be identical. The cellular component-binding aptamer and the second cellular component-binding aptamer can be capable of binding to different regions of the same protein target. The cellular component-binding aptamer and the second cellular component-binding aptamer can be capable of binding to different protein targets of the one or more protein targets.

Disclosed herein are embodiments of a kit for sample identification. In some embodiments, the kit comprises a plurality of sample indexing compositions. In some embodiments, the kit comprises a plurality of barcodes. A barcode of the plurality of barcodes comprises a target-binding region. The target-binding region can comprise a capture sequence configured to capture (or capable of capturing, hybridizing to or binding to) a sequence of a sample indexing oligonucleotide. A cellular component-binding aptamer can comprise the sample indexing oligonucleotide and a first cellular component-binding aptamer.

Disclosed herein are embodiments of a method for measuring protein expression in cells. In some embodiments, the method comprises: contacting a plurality of protein-binding aptamers with a plurality of cells comprising a plurality of protein targets, wherein each of the plurality of protein-binding aptamers comprises an aptamer specific oligonucleotide comprising a unique identifier sequence for the cellular component-binding aptamer, and wherein the protein-binding aptamer is capable of specifically binding to at least one of the plurality of protein targets; partitioning the plurality of cells associated with the plurality of protein-binding aptamers to a plurality of partitions, wherein a partition of the plurality of partitions comprises a single cell from the plurality of cells associated with the protein-binding aptamers; in the partition comprising the single cell, contacting a barcoding particle with the aptamer specific oligonucleotides, wherein the barcoding particle comprises a plurality of oligonucleotide probes each comprising a target binding region and a barcode sequence selected from a diverse set of unique barcode sequences; extending oligonucleotide probes hybridized to the aptamer specific oligonucleotides to produce a plurality of labeled nucleic acids, wherein each of the labeled nucleic acid comprises a unique identifier sequence, or a complementary sequence thereof, and a barcode sequence; and obtaining sequence information of the plurality of labeled nucleic acids or a portion thereof to determine the quantity of one or more of the plurality of protein targets in one or more of the plurality of cells.

Disclosed herein include embodiments of a method of measuring cellular component expression in cells. In some embodiments, the method comprises: contacting a plurality of cellular component-binding aptamers with a plurality of cells comprising a plurality of cellular component targets, wherein each of the plurality of cellular component-binding aptamers comprises an aptamer specific oligonucleotide comprising a unique identifier sequence for the cellular component-binding aptamer, and wherein the cellular component-binding aptamer is capable of specifically binding to at least one of the plurality of cellular component targets; extending oligonucleotide probes hybridized to the aptamer specific oligonucleotides to produce a plurality of labeled nucleic acids, wherein each of the labeled nucleic acid comprises a unique identifier sequence, or a complementary sequence thereof, and a barcode sequence; and obtaining sequence information of the plurality of labeled nucleic acids or a portion thereof to determine the quantity of one or more of the plurality of cellular component targets in one or more of the plurality of cells. The method can comprise: prior to extending the oligonucleotide probes: partitioning the plurality of cells associated with the plurality of cellular component-binding aptamers to a plurality of partitions, wherein a partition of the plurality of partitions comprises a single cell from the plurality of cells associated with the cellular component-binding aptamers; in the partition comprising the single cell, contacting a barcoding particle with the aptamer specific oligonucleotides, wherein the barcoding particle comprises a plurality of oligonucleotide probes each comprising a target binding region and a barcode sequence selected from a can diverse set of unique barcode sequences. The plurality of cellular component targets comprise a plurality of protein targets, and wherein the cellular component-binding aptamer is capable of specifically binding to at least one of the plurality of protein targets.

In some embodiments, the aptamer specific oligonucleotide and the aptamer form a single polynucleotide. The aptamer can be 5' to the aptamer specific oligonucleotide in the single polynucleotide. The aptamer can be 3' to the aptamer specific oligonucleotide in the single polynucleotide. The aptamer specific oligonucleotide can be associated with the aptamer. The aptamer specific oligonucleotide can be attached to the aptamer. The aptamer specific oligonucleotide can be covalently attached to the aptamer. The aptamer specific oligonucleotide can be non-covalently attached to the aptamer. The aptamer specific oligonucleotide can be conjugated to the aptamer. The aptamer specific oligonucleotide can be conjugated to the aptamer through a chemical group selected from the group consisting of a UV photocleavable group, a streptavidin, a biotin, an amine, and a combination thereof. The aptamer specific oligonucleotide can be covalently attached to the aptamer. In some embodiments, the method comprises detaching the aptamer specific oligonucleotides from the aptamer.

In some embodiments, the aptamer comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The aptamer can comprise a peptide aptamer.

In some embodiments, the plurality of protein-binding aptamers comprises a second protein-binding aptamer, or the plurality of cellular component-binding aptamers comprises a second cellular component-binding aptamer. The aptamer and the second aptamer can be associated with a first carrier. The aptamer can be associated with a first carrier, and the second aptamer can be associated with a second carrier. The aptamer and the second aptamer can be at least 60%, 70%, 80%, 90%, or 95% identical in sequence3. The aptamer and the second protein-binding aptamer can be identical. The aptamer and the second aptamer can be different.

In some embodiments, the protein targets, or the cellular component targets, of the aptamer and the second aptamer are identical. The aptamer and the second aptamer can be capable of binding to different regions of a protein target, or a cellular component target. The protein targets, or the cellular component targets, of the aptamer and the second aptamer can be different.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the aptamer is attached to the first carrier. The aptamer can be covalently attached to the first carrier. The aptamer can be conjugated to the first carrier. The aptamer can be conjugated to the first carrier through a chemical group selected from the group consisting of a UV photocleavable group, a streptavidin, a biotin, an amine, and a combination thereof. The aptamer can be non-covalently linked to the first carrier. The aptamer can be associated with the first carrier through a linker. The aptamer can be immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the method comprises dissociating the aptamer from the first carrier. The dissociating can occur after the contacting in the partition comprising the single cells. The dissociating can occur before the contacting in the partition comprising the single cells.

In some embodiments, contacting the plurality of aptamers with the plurality of cells comprises: contacting the first carrier comprising the aptamer with a cell of the plurality of cells comprising the plurality of protein targets. The first carrier can be internalized into the cell. The first carrier can be internalized into the cell via endocytosis, pinocytosis, nanopinocytosis, micropinocytosis, phagocytosis, membrane fusion, or a combination thereof. The first carrier can be internalized into the cell via clathrin-medicated internalization, caveolin-mediated internalization, receptor dependent internalization, receptor independent internalization or a combination thereof.

In some embodiments, the aptamer specific oligonucleotide comprises a sequence complementary to a capture sequence configured to capture (or capable of capturing, hybridizing to, or binding to) the sequence of the aptamer specific oligonucleotide. The barcode can comprise a target-binding region which comprises the capture sequence. The target-binding region can comprise a poly(dT) region. The sequence of the aptamer specific oligonucleotide complementary to the capture sequence can comprise a poly(dA) region.

In some embodiments, the aptamer specific oligonucleotide changes from a first conformation in which the poly(dA) region is inaccessible to a second conformation in which the poly(dA) region is accessible when the aptamer contacts the at least one of the plurality of protein targets, or cellular component targets. The poly(dA) region of the aptamer specific oligonucleotide in the first conformation can comprise a hairpin structure. The poly(dA) region with the hairpin structure can be accessible to the poly(dT) region of the target-binding region of the barcode. The oligonucleotide probes can be hybridized to the aptamer specific oligonucleotides via the hybridization between the poly(dA) regions of the aptamer specific oligonucleotides and the poly(dT) regions of the oligonucleotide probes.

In some embodiments, the aptamer specific oligonucleotide comprises an alignment sequence adjacent to the poly(dA) region. The alignment sequence can be 1 or more nucleotides in length. The alignment sequence can be 2 nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof.

In some embodiments, the aptamer specific oligonucleotide comprises a molecular label sequence, a binding site for a universal primer, or both. The molecular label sequence can be 2-20 nucleotides in length. The universal primer can be 5-50 nucleotides in length. The universal primer can comprise an amplification primer, a sequencing primer, or a combination thereof.

In some embodiments, the method comprises after contacting the plurality of aptamers with the plurality of cells, removing the aptamers that are not contacted with the plurality of cells. Removing the aptamers not contacted with the plurality of cells can comprise: removing the aptamers not contacted with the respective at least one of the plurality of protein targets. Removing the aptamers not contacted with the respective at least one of the plurality of protein targets can comprise: removing the aptamers not contacted with the respective at least one of the plurality of protein targets comprises using an aptamer sponge. The aptamer sponge can comprise a plurality of sequences complementary to the sequences of the aptamers. The aptamers not contacted with the respective at least one of the plurality of protein targets can hybridize to the plurality of sequences of the aptamer sponge.

In some embodiments, the aptamers comprise complementary sequences. The aptamers not contacted with the respective at least one of the plurality of protein targets can hybridize to one another to form an aggregate. The aggregate can be targeted for degradation in the cell.

In some embodiments, removing the aptamers not contacted with the respective at least one of the plurality of protein targets comprises: removing the aptamers not contacted with the respective at least one of the plurality of protein targets comprises using a plurality of antisense aptamers, wherein each anti-sense aptamer of the plurality of antisense aptamer comprises a second aptamer comprising an anti-sense aptamer specific oligonucleotide comprising the complementary sequence, or a portion thereof, of the aptamer specific oligonucleotide of one of the aptamers not contacted with the respective at least one of the plurality of protein targets, and wherein the aptamer specific oligonucleotide and the anti-sense aptamer specific oligonucleotide form a double stranded, or a partially double stranded, deoxyribonucleic acid (DNA) molecule.

In some embodiments, partitioning the plurality of cells comprises partitioning the plurality of cells associated with the plurality of aptamers and a plurality of barcoding particles comprising the barcoding particle to the plurality of partitions, wherein the partition of the plurality of partitions comprises the single cell from the plurality of cells associated with the oligonucleotide-conjugated aptamer and the barcoding particle. In some embodiments, the partition is a well or a droplet. In some embodiments, obtaining sequencing information of the plurality of labeled nucleic acids or a portion thereof comprises subjecting the labeled nucleic acids to one or more reactions to generate a set of nucleic acids for nucleic acid sequencing.

In some embodiments, the plurality of protein targets comprises a cell-surface protein, an intracellular protein, a cell marker, a B-cell receptor, a T-cell receptor, an antibody, a major histocompatibility complex, a tumor antigen, a receptor, or a combination thereof. In some embodiments, the plurality of cells comprises T cells, B cells, tumor cells, myeloid cells, blood cells, normal cells, fetal cells, maternal cells, or a mixture thereof.

In some embodiments, each of the oligonucleotide probes comprises a cell label, a binding site for a universal primer, an amplification adaptor, a sequencing adaptor, or a combination thereof. In some embodiments, the barcoding particle is a Sepharose bead, a streptavidin bead, an agarose bead, a magnetic bead, a silica bead, a silica-like bead, a hydrogel bead, an anti-biotin microbead, an anti-fluorochrome microbead, or a hydrogel bead.

Disclosed herein include composition comprising: a plurality of cellular component-binding aptamers, wherein each of the plurality of cellular component-binding aptamers comprises an aptamer specific oligonucleotide comprising a unique identifier sequence for the cellular component-binding aptamer, and wherein the cellular component-binding aptamer is capable of specifically binding to at least one of a plurality of cellular component targets.

In some embodiments, the aptamer specific oligonucleotide and the aptamer form a single polynucleotide. The cellular component-binding aptamer can be 5' to the aptamer specific oligonucleotide in the single polynucleotide. The cellular component-binding aptamer can be 3' to the aptamer specific oligonucleotide in the single polynucleotide.

In some embodiments, the aptamer specific oligonucleotide is associated with the cellular component-binding aptamer. The aptamer specific oligonucleotide can be attached to the cellular component-binding aptamer. The aptamer specific oligonucleotide can be covalently attached to the cellular component-binding aptamer. The aptamer specific oligonucleotide can be non-covalently attached to the cellular component-binding aptamer. The aptamer specific oligonucleotide can be conjugated to the cellular component-binding aptamer. The aptamer specific oligonucleotide can be conjugated to the cellular component-binding aptamer through a chemical group selected from the group consisting of a UV photocleavable group, a streptavidin, a biotin, an amine, and a combination thereof. The aptamer specific oligonucleotide can be covalently attached to the cellular component-binding aptamer.

In some embodiments, the cellular component-binding aptamer comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The cellular component-binding aptamer can comprise a peptide aptamer.

In some embodiments, the plurality of cellular component-binding aptamers comprises a second cellular component-binding aptamer. The cellular component-binding aptamer and the second cellular component-binding aptamer can be associated with a first carrier. The cellular component-binding aptamer can be associated with a first carrier, and the second cellular component-binding aptamer can be associated with a second carrier.

In some embodiments, the cellular component-binding aptamer and the second cellular component-binding aptamer can have at least 60%, 70%, 80%, 90%, or 95% sequence identity. The cellular component-binding aptamer and the second protein-binding aptamer can be identical. The cellular component-binding aptamer and the second cellular component-binding aptamer can be different.

In some embodiments, the protein targets, or the cellular component targets, of the cellular component-binding aptamer and the second cellular component-binding aptamer are identical. The cellular component-binding aptamer and the second cellular component-binding aptamer can be capable of binding to different regions of a protein target, or a cellular component target. The protein targets, or the cellular component targets, of the cellular component-binding aptamer and the second cellular component-binding aptamer can be different.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the cellular component-binding aptamer is attached to the first carrier. The cellular component-binding aptamer can be covalently attached to the first carrier. The cellular component-binding aptamer can be conjugated to the first carrier. The cellular component-binding aptamer can be conjugated to the first carrier through a chemical group selected from the group consisting of a UV photocleavable group, a streptavidin, a biotin, an amine, and a combination thereof. The cellular component-binding aptamer can be non-covalently linked to the first carrier. The cellular component-binding aptamer can be associated with the first carrier through a linker. The cellular component-binding aptamer can be immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the first carrier is configured to be internalized (or capable of being internalized) into a cell. The first carrier can be configured to be internalized (or capable of being internalized) into the cell via endocytosis, pinocytosis, nanopinocytosis, micropinocytosis, phagocytosis, membrane fusion, or a combination thereof. The first carrier can be configured to be internalized (or capable of being internalized) into the cell via clathrin-medicated internalization, caveolin-mediated internalization, receptor dependent internalization, receptor independent internalization or a combination thereof.

In some embodiments, the aptamer specific oligonucleotide comprises a sequence complementary to a capture sequence configured to capture (or capable of capturing, binding to, or hybridizing to) the sequence of the aptamer specific oligonucleotide. The barcode can comprise a target-binding region which comprises the capture sequence. The target-binding region can comprise a poly(dT) region. The sequence of the aptamer specific oligonucleotide complementary to the capture sequence can comprise a poly(dA) region.

In some embodiments, the aptamer specific oligonucleotide changes from a first conformation in which the poly(dA) region is inaccessible to a second conformation in which the poly(dA) region is accessible when the aptamer contacts the at least one of the plurality of protein targets, or cellular component targets. The poly(dA) region of the aptamer specific oligonucleotide in the first conformation can comprise a hairpin structure. The poly(dA) region with the hairpin structure can be accessible to the poly(dT) region of the target-binding region of the barcode. The oligonucleotide probes can be hybridized to the aptamer specific oligonucleotides via the hybridization between the poly(dA) regions of the aptamer specific oligonucleotides and the poly(dT) regions of the oligonucleotide probes.

In some embodiments, the aptamer specific oligonucleotide comprises an alignment sequence adjacent to the poly(dA) region. The alignment sequence can be 1 or more nucleotides in length. The alignment sequence can be 2 nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof.

In some embodiments, the aptamer specific oligonucleotide can comprise a molecular label sequence, a binding site for a universal primer, or both. The molecular label sequence can be 2-20 nucleotides in length. The universal primer can be 5-50 nucleotides in length. The universal primer can comprise an amplification primer, a sequencing primer, or a combination thereof.

In some embodiments, the composition comprises an aptamer sponge. The aptamer sponge can comprise a plurality of sequences complementary to the sequences of the aptamers.

In some embodiments, the aptamers comprise complementary sequences. The aptamers can be configured to hybridize to (or capable of hybridizing to) one another to form an aggregate. The aggregate can be targeted for degradation in the cell.

In some embodiments, the composition comprises: a plurality of antisense aptamers, wherein each anti-sense aptamer of the plurality of antisense aptamer comprises a second aptamer comprising an anti-sense aptamer specific oligonucleotide comprising the complementary sequence, or a portion thereof, of the aptamer specific oligonucleotide of one of the aptamers, and wherein the aptamer specific oligonucleotide and the anti-sense aptamer specific oligonucleotide form a double stranded, or a partially double stranded, deoxyribonucleic acid (DNA) molecule.

### BRIEF DESCRIPTION OF THE DRAWINGS

FIG. 1 illustrates a non-limiting exemplary stochastic barcode.
FIG. 2 shows a non-limiting exemplary workflow of stochastic barcoding and digital counting.
FIG. 3 is a schematic illustration showing a non-limiting exemplary process for generating an indexed library of the stochastically barcoded targets from a plurality of targets.
FIG. 4 shows a schematic illustration of an exemplary protein binding reagent (antibody illustrated here) associated with an oligonucleotide comprising a unique identifier for the protein binding reagent.
FIG. 5 shows a schematic illustration of an exemplary binding reagent (antibody illustrated here) associated with an oligonucleotide comprising a unique identifier for sample indexing to determine cells from the same or different samples.
FIG. 6 shows a schematic illustration of an exemplary workflow of using oligonucleotide-associated antibodies to determine cellular component expression (e.g., protein expression) and gene expression simultaneously in a high throughput manner.
FIG. 7 shows a schematic illustration of an exemplary workflow of using oligonucleotide-associated antibodies for sample indexing.
FIGS. 8A-8E show a schematic illustration of an exemplary workflow of using oligonucleotide-associated (e.g., conjugated) aptamers to determine cellular component expression (e.g., protein expression).
FIG. 9 is a non-limiting exemplary aptamer and associated sequences.
FIGS. 10A-10B show a schematic illustration of an exemplary workflow of using oligonucleotide-associated aptamer for sample indexing.
FIGS. 11A-11D show non-limiting exemplary designs of oligonucleotides for determining protein expression and gene expression simultaneously and for sample indexing.
FIG. 12 shows a schematic illustration of a non-limiting exemplary oligonucleotide sequence for determining protein expression and gene expression simultaneously and for sample indexing.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

Quantifying small numbers of nucleic acids, for example messenger ribonucleotide acid (mRNA) molecules, is clinically important for determining, for example, the genes that are expressed in a cell at different stages of development or under different environmental conditions. However, it can also be very challenging to determine the absolute number of nucleic acid molecules (e.g., mRNA molecules), especially when the number of molecules is very small. One method to determine the absolute number of molecules in a sample is digital polymerase chain reaction (PCR). Ideally, PCR produces an identical copy of a molecule at each cycle. However, PCR can have disadvantages such that each molecule replicates with a stochastic probability, and this probability varies by PCR cycle and gene sequence, resulting in amplification bias and inaccurate gene expression measurements. Stochastic barcodes with unique molecular labels (also referred to as molecular indexes (MIs)) can be used to count the number of molecules and correct for amplification bias. Stochastic barcoding such as the Precise^{™} assay (Cellular Research, Inc. (Palo Alto, CA)) can correct for bias induced by PCR and library preparation steps by using molecular labels (MLs) to label mRNAs during reverse transcription (RT).

The Precise^{™} assay can utilize a non-depleting pool of stochastic barcodes with large number, for example 6561 to 65536, unique molecular labels on poly(T) oligonucleotides to hybridize to all poly(A) mRNAs in a sample during the RT step. A stochastic barcode can comprise a universal PCR priming site. During RT, target gene molecules react randomly with stochastic barcodes. Each target molecule can hybridize to a stochastic barcode resulting to generate stochastically barcoded complementary ribonucleotide acid (cDNA) molecules). After labeling, stochastically barcoded cDNA molecules from microwells of a microwell plate can be pooled into a single tube for PCR amplification and sequencing. Raw sequencing data can be analyzed to produce the number of reads, the number of stochastic barcodes with unique molecular labels, and the numbers of mRNA molecules.

Methods for determining mRNA expression profiles of single cells can be performed in a massively parallel manner. For example, the Precise^{™} assay can be used to determine the mRNA expression profiles of more than 10000 cells simultaneously. The number of single cells (e.g., 100s or 1000s of singles) for analysis per sample can be lower than the capacity of the current single cell technology. Pooling of cells from different samples enables improved utilization of the capacity of the current single technology, thus lowering reagents wasted and the cost of single cell analysis. The disclosure provides methods of sample indexing for distinguishing cells of different samples for cDNA library preparation for cell analysis, such as single cell analysis. Pooling of cells from different samples can minimize the variations in cDNA library preparation of cells of different samples, thus enabling more accurate comparisons of different samples.

Disclosed herein include embodiments of a method for sample identification. In some embodiments, the method comprises: contacting each of a plurality of samples with a sample indexing composition of a plurality of sample indexing compositions, respectively, wherein each of the plurality of samples comprises one or more cells each comprising one or more protein targets, wherein the sample indexing composition comprises an aptamer composition comprising an aptamer and a sample indexing oligonucleotide, wherein the aptamer is capable of specifically binding to at least one of the one or more protein targets, wherein the sample indexing oligonucleotide comprises a sample indexing sequence, and wherein sample indexing sequences of at least two sample indexing compositions of the plurality of sample indexing compositions comprise different sequences; barcoding the sample indexing oligonucleotides using a plurality of barcodes to generate a plurality of barcoded sample indexing oligonucleotides; obtaining sequencing data of the plurality of barcoded sample indexing oligonucleotides; and identifying sample origin of at least one cell of the one or more cells based on the sample indexing sequence of at least one barcoded sample indexing oligonucleotide of the plurality of barcoded sample indexing oligonucleotides.

Disclosed herein are embodiments of a plurality of sample indexing compositions. In some embodiments, each of the plurality of sample indexing compositions comprises an aptamer composition comprising a first cellular component-binding aptamer and a sample indexing oligonucleotide, the cellular component-binding aptamer is capable of specifically binding to at least one cellular component target, the sample indexing oligonucleotide comprises a sample indexing sequence for identifying sample origin of one or more cells of a sample, and sample indexing sequences of at least two sample indexing compositions of the plurality of sample indexing compositions comprise different sequences.

Disclosed herein are embodiments of a method for measuring protein expression in cells. In some embodiments, the method comprises: contacting a plurality of protein-binding aptamers with a plurality of cells comprising a plurality of protein targets, wherein each of the plurality of protein-binding aptamers comprises an aptamer specific oligonucleotide comprising a unique identifier sequence for the cellular component-binding aptamer, and wherein the protein-binding aptamer is capable of specifically binding to at least one of the plurality of protein targets; partitioning the plurality of cells associated with the plurality of protein-binding aptamers to a plurality of partitions, wherein a partition of the plurality of partitions comprises a single cell from the plurality of cells associated with the protein-binding aptamers; in the partition comprising the single cell, contacting a barcoding particle with the aptamer specific oligonucleotides, wherein the barcoding particle comprises a plurality of oligonucleotide probes each comprising a target binding region and a barcode sequence selected from a diverse set of unique barcode sequences; extending oligonucleotide probes hybridized to the aptamer specific oligonucleotides to produce a plurality of labeled nucleic acids, wherein each of the labeled nucleic acid comprises a unique identifier sequence, or a complementary sequence thereof, and a barcode sequence; and obtaining sequence information of the plurality of labeled nucleic acids or a portion thereof to determine the quantity of one or more of the plurality of protein targets in one or more of the plurality of cells.

Disclosed herein include embodiments of a method of measuring cellular component expression in cells. In some embodiments, the method comprises: contacting a plurality of cellular component-binding aptamers with a plurality of cells comprising a plurality of cellular component targets, wherein each of the plurality of cellular component-binding aptamers comprises an aptamer specific oligonucleotide comprising a unique identifier sequence for the cellular component-binding aptamer, and wherein the cellular component-binding aptamer is capable of specifically binding to at least one of the plurality of cellular component targets; extending oligonucleotide probes hybridized to the aptamer specific oligonucleotides to produce a plurality of labeled nucleic acids, wherein each of the labeled nucleic acid comprises a unique identifier sequence, or a complementary sequence thereof, and a barcode sequence; and obtaining sequence information of the plurality of labeled nucleic acids or a portion thereof to determine the quantity of one or more of the plurality of cellular component targets in one or more of the plurality of cells.

Disclosed herein include composition comprising: a plurality of cellular component-binding aptamers, wherein each of the plurality of cellular component-binding aptamers comprises an aptamer specific oligonucleotide comprising a unique identifier sequence for the cellular component-binding aptamer, and wherein the cellular component-binding aptamer is capable of specifically binding to at least one of a plurality of cellular component targets.

### Definitions

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence to facilitate amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adapters can be linear. The adaptors can be pre-adenylated adapters. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adapter can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adapters can comprise identical and/or universal nucleic acid sequences and the 3' adapters can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adapters (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein, an antibody can be a full-length (e.g., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

In some embodiments, an antibody is a functional antibody fragment. For example, an antibody fragment can be a portion of an antibody such as F(ab')2, Fab', Fab, Fv, sFv and the like. An antibody fragment can bind with the same antigen that is recognized by the full-length antibody. An antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (for example, CD8, CD34, and CD45), and therapeutic antibodies.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, the terms "complement", "complementary", and "reverse complement" can be used interchangeably. It is understood from the disclosure that if a molecule can hybridize to another molecule it may be the complement of the molecule that is hybridizing.

As used herein, the term "digital counting" can refer to a method for estimating a number of target molecules in a sample. Digital counting can include the step of determining a number of unique labels that have been associated with targets in a sample. This methodology, which can be stochastic in nature, transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode. The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH₂), group bridging the 2' oxygen atom and the 4' carbon atom wherein n is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of barcodes (e.g., stochastic barcodes) may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

As used herein, the term "stochastic barcode" can refer to a polynucleotide sequence comprising labels of the present disclosure. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "stochastic labeling."

As used here, the term "target" can refer to a composition which can be associated with a barcode (e.g., a stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transciptases, and group II intron reverse transcriptases. Examples of group II intron reverse transcriptases include the *Lactococcus lactis* LI.LtrB intron reverse transcriptase, the *Thermosynechococcus* elongatus TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

The terms "universal adaptor primer," "universal primer adaptor" or "universal adaptor sequence" are used interchangeably to refer to a nucleotide sequence that can be used to hybridize to barcodes (e.g., stochastic barcodes) to generate gene-specific barcodes. A universal adaptor sequence can, for example, be a known sequence that is universal across all barcodes used in methods of the disclosure. For example, when multiple targets are being labeled using the methods disclosed herein, each of the target-specific sequences may be linked to the same universal adaptor sequence. In some embodiments, more than one universal adaptor sequences may be used in the methods disclosed herein. For example, when multiple targets are being labeled using the methods disclosed herein, at least two of the target-specific sequences are linked to different universal adaptor sequences. A universal adaptor primer and its complement may be included in two oligonucleotides, one of which comprises a target-specific sequence and the other comprises a barcode. For example, a universal adaptor sequence may be part of an oligonucleotide comprising a target-specific sequence to generate a nucleotide sequence that is complementary to a target nucleic acid. A second oligonucleotide comprising a barcode and a complementary sequence of the universal adaptor sequence may hybridize with the nucleotide sequence and generate a target-specific barcode (e.g., a target-specific stochastic barcode). In some embodiments, a universal adaptor primer has a sequence that is different from a universal PCR primer used in the methods of this disclosure.

### Barcodes

Barcoding, such as stochastic barcoding, has been described in, for example, US20150299784, WO2015031691, and Fu et al, Proc Natl Acad Sci U.S.A. 2011 May 31;108(22):9026-31. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

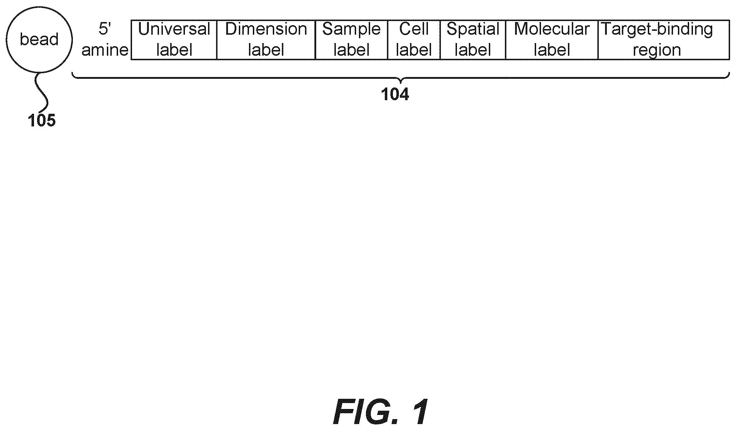

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. FIG. 1 illustrates an exemplary barcode 104 with a spatial label. The barcode 104 can comprise a 5'amine that may link the barcode to a solid support 105. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, as shown in FIG. 1, the universal label may be the 5'-most label, and the molecular label may be the 3'-most label. The spatial label, dimension label, and the cell label may be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs. In some instances, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

The barcode can comprise a target-binding region. The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, or any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the barcode (e.g., the universal label, the dimension label, the spatial label, the cell label, and the barcode sequences (e.g., molecular label)) can be separated by a spacer from another one or two of the remaining labels of the barcode. The spacer can be, for example, 1, 2, 3, 4, 5, 6, 7, 8, 9, 10, 11, 12, 13, 14, 15, 16, 17, 18, 19, or 20, or more nucleotides. In some embodiments, none of the labels of the barcode is separated by spacer.

### Universal Labels

A barcode can comprise one or more universal labels. In some embodiments, the one or more universal labels can be the same for all barcodes in the set of barcodes attached to a given solid support. In some embodiments, the one or more universal labels can be the same for all barcodes attached to a plurality of beads. In some embodiments, a universal label can comprise a nucleic acid sequence that is capable of hybridizing to a sequencing primer. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or be at most, 1, 2, 3, 4, 5, 10, 15, 20, 25, 30, 35, 40, 45, 50, 100, 200, or 300 nucleotides in length. In some embodiments, a cleavable linker or modified nucleotide can be part of the universal label sequence to enable the barcode to be cleaved off from the support.

### Dimension Labels

A barcode can comprise one or more dimension labels. In some embodiments, a dimension label can comprise a nucleic acid sequence that provides information about a dimension in which the labeling (e.g., stochastic labeling) occurred. For example, a dimension label can provide information about the time at which a target was barcoded. A dimension label can be associated with a time of barcoding (e.g., stochastic barcoding) in a sample. A dimension label can be activated at the time of labeling. Different dimension labels can be activated at different times. The dimension label provides information about the order in which targets, groups of targets, and/or samples were barcoded. For example, a population of cells can be barcoded at the G0 phase of the cell cycle. The cells can be pulsed again with barcodes (e.g., stochastic barcodes) at the G1 phase of the cell cycle. The cells can be pulsed again with barcodes at the S phase of the cell cycle, and so on. Barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, 10, or more times. The dimension label can be reversibly activatable, for example, at least 1, 2, 3, 4, 5, 6, 7, 8, 9,, 10 or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all barcodes (e.g., stochastic barcodes) attached to a given solid support (e.g., a bead), but different for different solid supports (e.g., beads). In some embodiments, at least 60%, 70%, 80%, 85%, 90%, 95%, 97%, 99% or 100%, of barcodes on the same solid support can comprise the same dimension label. In some embodiments, at least 60% of barcodes on the same solid support can comprise the same dimension label. In some embodiments, at least 95% of barcodes on the same solid support can comprise the same dimension label.

There can be as many as 10⁶ or more unique dimension label sequences represented in a plurality of solid supports (e.g., beads). A dimension label can be, or be about 1, 2, 3, 4, 5, 10, 15, 20, 25, 30, 35, 40, 45, 50, or a number or a range between any two of these values, nucleotides in length. A dimension label can be at least, or be at most, 1, 2, 3, 4, 5, 10, 15, 20, 25, 30, 35, 40, 45, 50, 100, 200, or 300, nucleotides in length. A dimension label can comprise between about 5 to about 200 nucleotides. A dimension label can comprise between about 10 to about 150 nucleotides. A dimension label can comprise between about 20 to about 125 nucleotides in length.

### Spatial Labels

A barcode can comprise one or more spatial labels. In some embodiments, a spatial label can comprise a nucleic acid sequence that provides information about the spatial orientation of a target molecule which is associated with the barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g., A well, a container, or a droplet). In some embodiments, multiple spatial labels are used together to encode one or more positions in space.

The spatial label can be identical for all barcodes attached to a given solid support (e.g., a bead), but different for different solid supports (e.g., beads). In some embodiments, the percentage of barcodes on the same solid support comprising the same spatial label can be, or be about, 60%, 70%, 80%, 85%, 90%, 95%, 97%, 99%, 100%, or a number or a range between any two of these values. In some embodiments, the percentage of barcodes on the same solid support comprising the same spatial label can be at least, or be at most, 60%, 70%, 80%, 85%, 90%, 95%, 97%, 99%, or 100%. In some embodiments, at least 60% of barcodes on the same solid support can comprise the same spatial label. In some embodiments, at least 95% of barcodes on the same solid support can comprise the same spatial label.

There can be as many as 10⁶ or more unique spatial label sequences represented in a plurality of solid supports (e.g., beads). A spatial label can be, or be about, 1, 2, 3, 4, 5, 10, 15, 20, 25, 30, 35, 40, 45, 50, or a number or a range between any two of these values, nucleotides in length. A spatial label can be at least or at most 1, 2, 3, 4, 5, 10, 15, 20, 25, 30, 35, 40, 45, 50, 100, 200, or 300 nucleotides in length. A spatial label can comprise between about 5 to about 200 nucleotides. A spatial label can comprise between about 10 to about 150 nucleotides. A spatial label can comprise between about 20 to about 125 nucleotides in length.

### Cell labels

A barcode (e.g., a stochastic barcode) can comprise one or more cell labels. In some embodiments, a cell label can comprise a nucleic acid sequence that provides information for determining which target nucleic acid originated from which cell. In some embodiments, the cell label is identical for all barcodes attached to a given solid support (e.g., a bead), but different for different solid supports (e.g., beads). In some embodiments, the percentage of barcodes on the same solid support comprising the same cell label can be, or be about 60%, 70%, 80%, 85%, 90%, 95%, 97%, 99%, 100%, or a number or a range between any two of these values. In some embodiments, the percentage of barcodes on the same solid support comprising the same cell label can be, or be about 60%, 70%, 80%, 85%, 90%, 95%, 97%, 99%, or 100%. For example, at least 60% of barcodes on the same solid support can comprise the same cell label. As another example, at least 95% of barcodes on the same solid support can comprise the same cell label.

There can be as many as 10⁶ or more unique cell label sequences represented in a plurality of solid supports (e.g., beads). A cell label can be, or be about, 1, 2, 3, 4, 5, 10, 15, 20, 25, 30, 35, 40, 45, 50, or a number or a range between any two of these values, nucleotides in length. A cell label can be at least, or be at most, 1, 2, 3, 4, 5, 10, 15, 20, 25, 30, 35, 40, 45, 50, 100, 200, or 300 nucleotides in length. For example, a cell label can comprise between about 5 to about 200 nucleotides. As another example, a cell label can comprise between about 10 to about 150 nucleotides. As yet another example, a cell label can comprise between about 20 to about 125 nucleotides in length.

### Barcode Sequences

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., a bead). In some embodiments, there can be, or be about, 10², 10³, 10⁴, 10⁵, 10⁶, 10⁷, 10⁸, 10⁹, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or be at most, 10², 10³, 10⁴, 10⁵, 10⁶, 10⁷, 10⁸, or 10⁹, unique barcode sequences. The unique molecular label sequences can be attached to a given solid support (e.g., a bead).

The length of a barcode can be different in different implementations. For example, a barcode can be, or be about, 1, 2, 3, 4, 5, 10, 15, 20, 25, 30, 35, 40, 45, 50, or a number or a range between any two of these values, nucleotides in length. As another example, a barcode can be at least, or be at most, 1, 2, 3, 4, 5, 10, 15, 20, 25, 30, 35, 40, 45, 50, 100, 200, or 300 nucleotides in length.

### Molecular Labels

A barcode (e.g., a stochastic barcode) can comprise one or more molecular labels. Molecular labels can include barcode sequences. In some embodiments, a molecular label can comprise a nucleic acid sequence that provides identifying information for the specific type of target nucleic acid species hybridized to the barcode. A molecular label can comprise a nucleic acid sequence that provides a counter for the specific occurrence of the target nucleic acid species hybridized to the barcode (e.g., target-binding region).

In some embodiments, a diverse set of molecular labels are attached to a given solid support (e.g., a bead). In some embodiments, there can be, or be about, 10², 10³, 10⁴, 10⁵, 10⁶, 10⁷, 10⁸, 10⁹, or a number or a range between any two of these values, of unique molecular label sequences. For example, a plurality of barcodes can comprise about 6561 molecular labels with distinct sequences. As another example, a plurality of barcodes can comprise about 65536 molecular labels with distinct sequences. In some embodiments, there can be at least, or be at most, 10², 10³, 10⁴, 10⁵, 10⁶, 10⁷, 10⁸, or 10⁹, unique molecular label sequences. Barcodes with unique molecular label sequences can be attached to a given solid support (e.g., a bead).

For stochastic barcoding using a plurality of stochastic barcodes, the ratio of the number of different molecular label sequences and the number of occurrence of any of the targets can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. In some embodiments, the ratio of the number of different molecular label sequences and the number of occurrence of any of the targets is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1.

A molecular label can be, or be about, 1, 2, 3, 4, 5, 10, 15, 20, 25, 30, 35, 40, 45, 50, or a number or a range between any two of these values, nucleotides in length. A molecular label can be at least, or be at most, 1, 2, 3, 4, 5, 10, 15, 20, 25, 30, 35, 40, 45, 50, 100, 200, or 300 nucleotides in length.

### Target-Binding Region

A barcode can comprise one or more target binding regions, such as capture probes. In some embodiments, a target-binding region can hybridize with a target of interest. In some embodiments, the target binding regions can comprise a nucleic acid sequence that hybridizes specifically to a target (e.g., target nucleic acid, target molecule, e.g., a cellular nucleic acid to be analyzed), for example to a specific gene sequence. In some embodiments, a target binding region can comprise a nucleic acid sequence that can attach (e.g., hybridize) to a specific location of a specific target nucleic acid. In some embodiments, the target binding region can comprise a nucleic acid sequence that is capable of specific hybridization to a restriction enzyme site overhang (e.g., an EcoRI sticky-end overhang). The barcode can then ligate to any nucleic acid molecule comprising a sequence complementary to the restriction site overhang.

In some embodiments, a target binding region can comprise a non-specific target nucleic acid sequence. A non-specific target nucleic acid sequence can refer to a sequence that can bind to multiple target nucleic acids, independent of the specific sequence of the target nucleic acid. For example, target binding region can comprise a random multimer sequence, or an oligo(dT) sequence that hybridizes to the poly(A) tail on mRNA molecules. A random multimer sequence can be, for example, a random dimer, trimer, quatramer, pentamer, hexamer, septamer, octamer, nonamer, decamer, or higher multimer sequence of any length. In some embodiments, the target binding region is the same for all barcodes attached to a given bead. In some embodiments, the target binding regions for the plurality of barcodes attached to a given bead can comprise two or more different target binding sequences. A target binding region can be, or be about, 5, 10, 15, 20, 25, 30, 35, 40, 45, 50, or a number or a range between any two of these values, nucleotides in length. A target binding region can be at most about 5, 10, 15, 20, 25, 30, 35, 40, 45, 50 or more nucleotides in length.

In some embodiments, a target-binding region can comprise an oligo(dT) which can hybridize with mRNAs comprising polyadenylated ends. A target-binding region can be gene-specific. For example, a target-binding region can be configured to hybridize to a specific region of a target. A target-binding region can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. A target-binding region can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30, nucleotides in length. A target-binding region can be about 5-30 nucleotides in length. When a barcode comprises a gene-specific target-binding region, the barcode can be referred to herein as a gene-specific barcode.

### Orientation Property

A stochastic barcode (e.g., a stochastic barcode) can comprise one or more orientation properties which can be used to orient (e.g., align) the barcodes. A barcode can comprise a moiety for isoelectric focusing. Different barcodes can comprise different isoelectric focusing points. When these barcodes are introduced to a sample, the sample can undergo isoelectric focusing in order to orient the barcodes into a known way. In this way, the orientation property can be used to develop a known map of barcodes in a sample. Exemplary orientation properties can include, electrophoretic mobility (e.g., based on size of the barcode), isoelectric point, spin, conductivity, and/or self-assembly. For example, barcodes with an orientation property of self-assembly, can self-assemble into a specific orientation (e.g., nucleic acid nanostructure) upon activation.

### Affinity Property

A barcode (e.g., a stochastic barcode) can comprise one or more affinity properties. For example, a spatial label can comprise an affinity property. An affinity property can include a chemical and/or biological moiety that can facilitate binding of the barcode to another entity (e.g., cell receptor). For example, an affinity property can comprise an antibody, for example, an antibody specific for a specific moiety (e.g., receptor) on a sample. In some embodiments, the antibody can guide the barcode to a specific cell type or molecule. Targets at and/or near the specific cell type or molecule can be labeled (e.g., stochastically labeled). The affinity property can, in some embodiments, provide spatial information in addition to the nucleotide sequence of the spatial label because the antibody can guide the barcode to a specific location. The antibody can be a therapeutic antibody, for example a monoclonal antibody or a polyclonal antibody. The antibody can be humanized or chimeric. The antibody can be a naked antibody or a fusion antibody.

The antibody can be a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

The antibody fragment can be, for example, a portion of an antibody such as F(ab')2, Fab', Fab, Fv, sFv and the like. In some embodiments, the antibody fragment can bind with the same antigen that is recognized by the full-length antibody. The antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (CD8, CD34, CD45), and therapeutic antibodies.

### Universal Adaptor Primer

A barcode can comprise one or more universal adaptor primers. For example, a gene-specific barcode, such as a gene-specific stochastic barcode, can comprise a universal adaptor primer. A universal adaptor primer can refer to a nucleotide sequence that is universal across all barcodes. A universal adaptor primer can be used for building gene-specific barcodes. A universal adaptor primer can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length. A universal adaptor primer can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 nucleotides in length. A universal adaptor primer can be from 5-30 nucleotides in length.

### Linker

When a barcode comprises more than one of a type of label (*e.g.*, more than one cell label or more than one barcode sequence, such as one molecular label), the labels may be interspersed with a linker label sequence. A linker label sequence can be at least about 5, 10, 15, 20, 25, 30, 35, 40, 45, 50 or more nucleotides in length. A linker label sequence can be at most about 5, 10, 15, 20, 25, 30, 35, 40, 45, 50 or more nucleotides in length. In some instances, a linker label sequence is 12 nucleotides in length. A linker label sequence can be used to facilitate the synthesis of the barcode. The linker label can comprise an error-correcting (e.g., Hamming) code.

### Solid Supports

Barcodes, such as stochastic barcodes, disclosed herein can, in some embodiments, be associated with a solid support. The solid support can be, for example, a synthetic particle. In some embodiments, some or all of the barcode sequences, such as molecular labels for stochastic barcodes (e.g., the first barcode sequences) of a plurality of barcodes (e.g., the first plurality of barcodes) on a solid support differ by at least one nucleotide. The cell labels of the barcodes on the same solid support can be the same. The cell labels of the barcodes on different solid supports can differ by at least one nucleotide. For example, first cell labels of a first plurality of barcodes on a first solid support can have the same sequence, and second cell labels of a second plurality of barcodes on a second solid support can have the same sequence. The first cell labels of the first plurality of barcodes on the first solid support and the second cell labels of the second plurality of barcodes on the second solid support can differ by at least one nucleotide. A cell label can be, for example, about 5-20 nucleotides long. A barcode sequence can be, for example, about 5-20 nucleotides long. The synthetic particle can be, for example, a bead.

The bead can be, for example, a silica gel bead, a controlled pore glass bead, a magnetic bead, a Dynabead, a Sephadex/Sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, or any combination thereof.

In some embodiments, the bead can be a polymeric bead, for example a deformable bead or a gel bead, functionalized with barcodes or stochastic barcodes (such as gel beads from 10X Genomics (San Francisco, CA). In some implementation, a gel bead can comprise a polymer based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the particle can be degradable. For example, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some embodiments, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some embodiments, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte. In some embodiments, at least one barcode of the plurality of barcodes can be immobilized on the particle, partially immobilized on the particle, enclosed in the particle, partially enclosed in the particle, or any combination thereof.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), poly(lactic-co-glycolic acid) (PLGA).

Numerous chemical stimuli can be used to trigger the disruption, dissolution, or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity of the bead wall.

Biological stimuli may also be used to trigger disruption, dissolution, or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat may cause melting of a bead such that the bead wall disintegrates. In other cases, the heat may increase the internal pressure of the inner components of the bead such that the bead ruptures or explodes. In still other cases, the heat may transform the bead into a shrunken dehydrated state. The heat may also act upon heat-sensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. In one example, incorporation of Fe₃O₄ nanoparticles into polyelectrolyte containing beads triggers rupture in the presence of an oscillating magnetic field stimulus.

A bead may also be disrupted, dissolved, or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus may also be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with SiO₂ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches result in a bead wall that may disintegrate or become more porous upon the application of a light trigger.

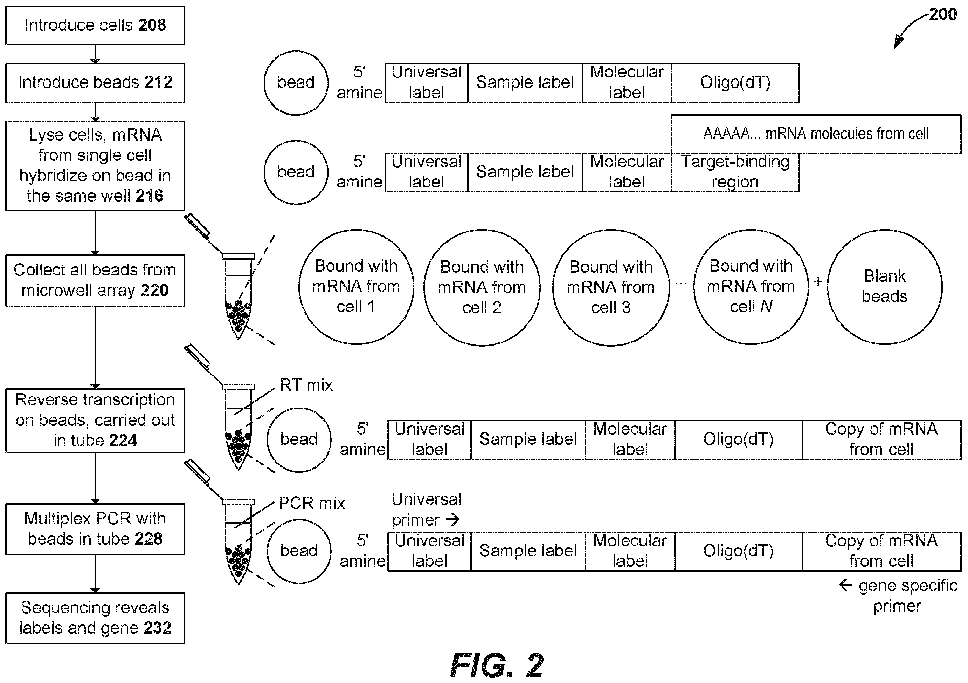

For example, in a non-limiting example of barcoding (e.g., stochastic barcoding) illustrated in FIG. 2, after introducing cells such as single cells onto a plurality of microwells of a microwell array at block 208, beads can be introduced onto the plurality of microwells of the microwell array at block 212. Each microwell can comprise one bead. The beads can comprise a plurality of barcodes. A barcode can comprise a 5' amine region attached to a bead. The barcode can comprise a universal label, a barcode sequence (e.g., a molecular label), a target-binding region, or any combination thereof.

The barcodes disclosed herein can be associated with (e.g., attached to) a solid support (e.g., a bead). The barcodes associated with a solid support can each comprise a barcode sequence selected from a group comprising at least 100 or 1000 barcode sequences with unique sequences. In some embodiments, different barcodes associated with a solid support can comprise barcode with different sequences. In some embodiments, a percentage of barcodes associated with a solid support comprises the same cell label. For example, the percentage can be, or be about 60%, 70%, 80%, 85%, 90%, 95%, 97%, 99%, 100%, or a number or a range between any two of these values. As another example, the percentage can be at least, or be at most 60%, 70%, 80%, 85%, 90%, 95%, 97%, 99%, or 100%. In some embodiments, barcodes associated with a solid support can have the same cell label. The barcodes associated with different solid supports can have different cell labels selected from a group comprising at least 100 or 1000 cell labels with unique sequences.

The barcodes disclosed herein can be associated to (e.g., attached to) a solid support (e.g., a bead). In some embodiments, barcoding the plurality of targets in the sample can be performed with a solid support including a plurality of synthetic particles associated with the plurality of barcodes. In some embodiments, the solid support can include a plurality of synthetic particles associated with the plurality of barcodes. The spatial labels of the plurality of barcodes on different solid supports can differ by at least one nucleotide. The solid support can, for example, include the plurality of barcodes in two dimensions or three dimensions. The synthetic particles can be beads. The beads can be silica gel beads, controlled pore glass beads, magnetic beads, Dynabeads, Sephadex/Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The barcodes may not be associated with solid supports. The barcodes can be individual nucleotides. The barcodes can be associated with a substrate.

As used herein, the terms "tethered," "attached," and "immobilized," are used interchangeably, and can refer to covalent or non-covalent means for attaching barcodes to a solid support. Any of a variety of different solid supports can be used as solid supports for attaching pre-synthesized barcodes or for *in situ* solid-phase synthesis of barcode.

In some embodiments, the solid support is a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite (Fe₃O₄; magnetite) nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, Sepharose, agarose, hydrogel, polymer, cellulose, nylon, or any combination thereof.

In some embodiments, the bead (e.g., the bead to which the labels are attached) is a hydrogel bead. In some embodiments, the bead comprises hydrogel.

Some embodiments disclosed herein include one or more particles (for example, beads). Each of the particles can comprise a plurality of oligonucleotides (e.g., barcodes). Each of the plurality of oligonucleotides can comprise a barcode sequence (e.g., a molecular label sequence), a cell label, and a target-binding region (e.g., an oligo(dT) sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸ 10⁹, a number or a range between any two of these values, or more. In some embodiments, the number of cell label sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. In some embodiments, no more than 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence. In some embodiment, the plurality of particles that include oligonucleotides with the same cell sequence can be at most 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more. In some embodiments, none of the plurality of the particles has the same cell label sequence.

The plurality of oligonucleotides on each particle can comprise different barcode sequences (e.g., molecular labels). In some embodiments, the number of barcode sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, or a number or a range between any two of these values. In some embodiments, the number of barcode sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. For example, at least 100 of the plurality of oligonucleotides comprise different barcode sequences. As another example, in a single particle, at least 100, 500, 1000, 5000, 10000, 15000, 20000, 50000, a number or a range between any two of these values, or more of the plurality of oligonucleotides comprise different barcode sequences. Some embodiments provide a plurality of the particles comprising barcodes. In some embodiments, the ratio of an occurrence (or a copy or a number) of a target to be labeled and the different barcode sequences can be at least 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

The size of the beads can vary. For example, the diameter of the bead can range from 0.1 micrometer to 50 micrometers. In some embodiments, the diameter of the bead can be, or be about, 0.1, 0.5, 1, 2, 3, 4, 5, 6, 7, 8, 9, 10, 20, 30, 40, 50 micrometers, or a number or a range between any two of these values.

The diameter of the bead can be related to the diameter of the wells of the substrate. In some embodiments, the diameter of the bead can be, or be about, 10%, 20%, 30%, 40%, 50%, 60%, 70%, 80%, 90%, 100%, or a number or a range between any two of these values, longer or shorter than the diameter of the well. The diameter of the beads can be related to the diameter of a cell (e.g., a single cell entrapped by a well of the substrate). In some embodiments, the diameter of the bead can be at least, or be at most, 10%, 20%, 30%, 40%, 50%, 60%, 70%, 80%, 90%, or 100% longer or shorter than the diameter of the well. The diameter of the beads can be related to the diameter of a cell (e.g., a single cell entrapped by a well of the substrate). In some embodiments, the diameter of the bead can be, or be about, 10%, 20%, 30%, 40%, 50%, 60%, 70%, 80%, 90%, 100%, 150%, 200%, 250%, 300%, or a number or a range between any two of these values, longer or shorter than the diameter of the cell. In some embodiments, the diameter of the beads can be at least, or be at most, 10%, 20%, 30%, 40%, 50%, 60%, 70%, 80%, 90%, 100%, 150%, 200%, 250%, or 300% longer or shorter than the diameter of the cell.

A bead can be attached to and/or embedded in a substrate. A bead can be attached to and/or embedded in a gel, hydrogel, polymer and/or matrix. The spatial position of a bead within a substrate (e.g., gel, matrix, scaffold, or polymer) can be identified using the spatial label present on the barcode on the bead which can serve as a location address.

Examples of beads can include, but are not limited to, streptavidin beads, agarose beads, magnetic beads, Dynabeads^{®}, MACS^{®} microbeads, antibody conjugated beads (e.g., anti-immunoglobulin microbeads), protein A conjugated beads, protein G conjugated beads, protein A/G conjugated beads, protein L conjugated beads, oligo(dT) conjugated beads, silica beads, silica-like beads, anti-biotin microbeads, anti-fluorochrome microbeads, and BcMag^{™} Carboxyl-Terminated Magnetic Beads.

A bead can be associated with (e.g., impregnated with) quantum dots or fluorescent dyes to make it fluorescent in one fluorescence optical channel or multiple optical channels. A bead can be associated with iron oxide or chromium oxide to make it paramagnetic or ferromagnetic. Beads can be identifiable. For example, a bead can be imaged using a camera. A bead can have a detectable code associated with the bead. For example, a bead can comprise a barcode. A bead can change size, for example, due to swelling in an organic or inorganic solution. A bead can be hydrophobic. A bead can be hydrophilic. A bead can be biocompatible.

A solid support (e.g., a bead) can be visualized. The solid support can comprise a visualizing tag (e.g., fluorescent dye). A solid support (e.g., a bead) can be etched with an identifier (e.g., a number). The identifier can be visualized through imaging the beads.

A solid support can comprise an insoluble, semi-soluble, or insoluble material. A solid support can be referred to as "functionalized" when it includes a linker, a scaffold, a building block, or other reactive moiety attached thereto, whereas a solid support may be "nonfunctionalized" when it lack such a reactive moiety attached thereto. The solid support can be employed free in solution, such as in a microtiter well format; in a flow-through format, such as in a column; or in a dipstick.

The solid support can comprise a membrane, paper, plastic, coated surface, flat surface, glass, slide, chip, or any combination thereof. A solid support can take the form of resins, gels, microspheres, or other geometric configurations. A solid support can comprise silica chips, microparticles, nanoparticles, plates, arrays, capillaries, flat supports such as glass fiber filters, glass surfaces, metal surfaces (steel, gold silver, aluminum, silicon and copper), glass supports, plastic supports, silicon supports, chips, filters, membranes, microwell plates, slides, plastic materials including multiwell plates or membranes (e.g., formed of polyethylene, polypropylene, polyamide, polyvinylidenedifluoride), and/or wafers, combs, pins or needles (e.g., arrays of pins suitable for combinatorial synthesis or analysis) or beads in an array of pits or nanoliter wells of flat surfaces such as wafers (e.g., silicon wafers), wafers with pits with or without filter bottoms.

The solid support can comprise a polymer matrix (e.g., gel, hydrogel). The polymer matrix may be able to permeate intracellular space (e.g., around organelles). The polymer matrix may able to be pumped throughout the circulatory system.

### Substrates and Microwell Array

As used herein, a substrate can refer to a type of solid support. A substrate can refer to a solid support that can comprise barcodes or stochastic barcodes of the disclosure. A substrate can, for example, comprise a plurality of microwells. For example, a substrate can be a well array comprising two or more microwells. In some embodiments, a microwell can comprise a small reaction chamber of defined volume. In some embodiments, a microwell can entrap one or more cells. In some embodiments, a microwell can entrap only one cell. In some embodiments, a microwell can entrap one or more solid supports. In some embodiments, a microwell can entrap only one solid support. In some embodiments, a microwell entraps a single cell and a single solid support (e.g., a bead). A microwell can comprise barcode reagents of the disclosure.

### Methods of Barcoding

The disclosure provides for methods for estimating the number of distinct targets at distinct locations in a physical sample (e.g., tissue, organ, tumor, cell). The methods can comprise placing barcodes (e.g., stochastic barcodes) in close proximity with the sample, lysing the sample, associating distinct targets with the barcodes, amplifying the targets and/or digitally counting the targets. The method can further comprise analyzing and/or visualizing the information obtained from the spatial labels on the barcodes. In some embodiments, a method comprises visualizing the plurality of targets in the sample. Mapping the plurality of targets onto the map of the sample can include generating a two dimensional map or a three dimensional map of the sample. The two dimensional map and the three dimensional map can be generated prior to or after barcoding (e.g., stochastically barcoding) the plurality of targets in the sample. Visualizing the plurality of targets in the sample can include mapping the plurality of targets onto a map of the sample. Mapping the plurality of targets onto the map of the sample can include generating a two dimensional map or a three dimensional map of the sample. The two dimensional map and the three dimensional map can be generated prior to or after barcoding the plurality of targets in the sample. in some embodiments, the two dimensional map and the three dimensional map can be generated before or after lysing the sample. Lysing the sample before or after generating the two dimensional map or the three dimensional map can include heating the sample, contacting the sample with a detergent, changing the pH of the sample, or any combination thereof.

In some embodiments, barcoding the plurality of targets comprises hybridizing a plurality of barcodes with a plurality of targets to create barcoded targets (e.g., stochastically barcoded targets). Barcoding the plurality of targets can comprise generating an indexed library of the barcoded targets. Generating an indexed library of the barcoded targets can be performed with a solid support comprising the plurality of barcodes (e.g., stochastic barcodes).

### Contacting a Sample and a Barcode

The disclosure provides for methods for contacting a sample (e.g., cells) to a substrate of the disclosure. A sample comprising, for example, a cell, organ, or tissue thin section, can be contacted to barcodes (e.g., stochastic barcodes). The cells can be contacted, for example, by gravity flow wherein the cells can settle and create a monolayer. The sample can be a tissue thin section. The thin section can be placed on the substrate. The sample can be one-dimensional (e.g., formsa planar surface). The sample (e.g., cells) can be spread across the substrate, for example, by growing/culturing the cells on the substrate.

When barcodes are in close proximity to targets, the targets can hybridize to the barcode. The barcodes can be contacted at a non-depletable ratio such that each distinct target can associate with a distinct barcode of the disclosure. To ensure efficient association between the target and the barcode, the targets can be cross-linked to barcode.

### Cell Lysis

Following the distribution of cells and barcodes, the cells can be lysed to liberate the target molecules. Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, 10, or more. The pH of the lysis buffer can be at most about 1, 2, 3, 4, 5, 6, 7, 8, 9, 10, or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about 0.0001%, 0.0005%, 0.001%, 0.005%, 0.01%, 0.05%, 0.1%, 0.5%, 1%, 2%, 3%, 4%, 5%, 6%, or 7%, or more. The concentration of the detergent in the lysis buffer can be at most about 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., beta-mercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

### Attachment of Barcodes to Target Nucleic Acid Molecules

Following lysis of the cells and release of nucleic acid molecules therefrom, the nucleic acid molecules can randomly associate with the barcodes of the co-localized solid support. Association can comprise hybridization of a barcode's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule. For example, in a non-limiting example of barcoding illustrated in FIG. 2, at block 216, mRNA molecules can hybridize to barcodes on beads. For example, single-stranded nucleotide fragments can hybridize to the target-binding regions of barcodes.

Attachment can further comprise ligation of a barcode's target recognition region and a portion of the target nucleic acid molecule. For example, the target binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The barcode can then be ligated to any nucleic acid molecule comprising a sequence complementary to the restriction site overhang. A ligase (e.g., T4 DNA ligase) can be used to join the two fragments.

For example, in a non-limiting example of barcoding illustrated in FIG. 2, at block 220, the labeled targets from a plurality of cells (or a plurality of samples) (e.g., target-barcode molecules) can be subsequently pooled, for example, into a tube. The labeled targets can be pooled by, for example, retrieving the barcodes and/or the beads to which the target-barcode molecules are attached.

The retrieval of solid support-based collections of attached target-barcode molecules can be implemented by use of magnetic beads and an externally-applied magnetic field. Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

### Reverse Transcription

The disclosure provides for a method to create a target-barcode conjugate using reverse transcription (e.g., at block 224 of FIG. 2). The target-barcode conjugate can comprise the barcode and a complementary sequence of all or a portion of the target nucleic acid (i.e., a barcoded cDNA molecule, such as a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of the labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

Reverse transcription can occur repeatedly to produce multiple labeled-cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

### Amplification

One or more nucleic acid amplification reactions (e.g., at block 228 of FIG. 2) can be performed to create multiple copies of the labeled target nucleic acid molecules. Amplification can be performed in a multiplexed manner, wherein multiple target nucleic acid sequences are amplified simultaneously. The amplification reaction can be used to add sequencing adaptors to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeledamplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule. The single-stranded molecule can comprise DNA, RNA, or a combination thereof. The nucleic acids of the disclosure can comprise synthetic or altered nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can comprise less than 12-15 nucleotides. The one or more primers can anneal to at least a portion of the plurality of labeled targets (e.g., stochastically labeled targets). The one or more primers can anneal to the 3' end or 5' end of the plurality of labeled targets. The one or more primers can anneal to an internal region of the plurality of labeled targets. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers. The one or more primers can comprise at least 960 or more custom primers. The one or more primers can comprise at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adaptors to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, or 20 amplification reactions. Alternatively, the method comprises conducting at least about 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adaptors can be added for sequencing using, for example, next generation sequencing (NGS). The sequencing results can be used to determine cell labels, molecular labels, and sequences of nucleotide fragments of the one or more copies of the targets, for example at block 232 of FIG. 2.

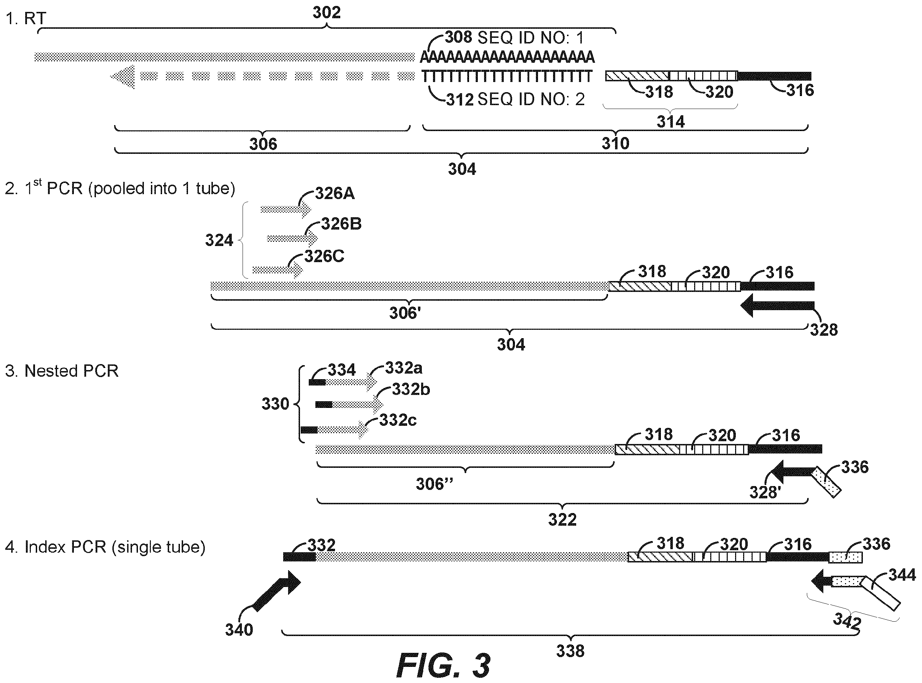

FIG. 3 is a schematic illustration showing a non-limiting exemplary process of generating an indexed library of the barcoded targets (e.g., stochastically barcoded targets), such as barcoded mRNAs or fragments thereof. As shown in step 1, the reverse transcription process can encode each mRNA molecule with a unique molecular label, a cell label, and a universal PCR site. In particular, RNA molecules 302 can be reverse transcribed to produce labeled cDNA molecules 304, including a cDNA region 306, by hybridization (e.g., stochastic hybridization) of a set of barcodes (e.g., stochastic barcodes) 310 to the poly(A) tail region 308 of the RNA molecules 302. Each of the barcodes 310 can comprise a target-binding region, for example a poly(dT) region 312, a label region 314 (e.g., a barcode sequence or a molecule), and a universal PCR region 316.

In some embodiments, the cell label can include 3 to 20 nucleotides. In some embodiments, the molecular label can include 3 to 20 nucleotides. In some embodiments, each of the plurality of stochastic barcodes further comprises one or more of a universal label and a cell label, wherein universal labels are the same for the plurality of stochastic barcodes on the solid support and cell labels are the same for the plurality of stochastic barcodes on the solid support. In some embodiments, the universal label can include 3 to 20 nucleotides. In some embodiments, the cell label comprises 3 to 20 nucleotides.

In some embodiments, the label region 314 can include a barcode sequence or a molecular label 318 and a cell label 320. In some embodiments, the label region 314 can include one or more of a universal label, a dimension label, and a cell label. The barcode sequence or molecular label 318 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The cell label 320 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The universal label can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. Universal labels can be the same for the plurality of stochastic barcodes on the solid support and cell labels are the same for the plurality of stochastic barcodes on the solid support. The dimension label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length.

In some embodiments, the label region 314 can comprise, comprise about, comprise at least, or comprise at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different labels, such as a barcode sequence or a molecular label 318 and a cell label 320. Each label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length. A set of barcodes or stochastic barcodes 310 can contain, contain about, contain at least, or can be at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹¹, 10¹⁴, 10¹⁵, 10²⁰, or a number or a range between any of these values, barcodes or stochastic barcodes 310. And the set of barcodes or stochastic barcodes 310 can, for example, each contain a unique label region 314. The labeled cDNA molecules 304 can be purified to remove excess barcodes or stochastic barcodes 310. Purification can comprise Ampure bead purification.

As shown in step 2, products from the reverse transcription process in step 1 can be pooled into 1 tube and PCR amplified with a 1^{st} PCR primer pool and a 1^{st} universal PCR primer. Pooling is possible because of the unique label region 314. In particular, the labeled cDNA molecules 304 can be amplified to produce nested PCR labeled amplicons 322. Amplification can comprise multiplex PCR amplification. Amplification can comprise a multiplex PCR amplification with 96 multiplex primers in a single reaction volume. In some embodiments, multiplex PCR amplification can utilize, utilize about, utilize at least, or utilize at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹¹, 10¹⁴, 10¹⁵, 10²⁰, or a number or a range between any of these values, multiplex primers in a single reaction volume. Amplification can comprise using a 1^{st} PCR primer pool 324 comprising custom primers 326A-C targeting specific genes and a universal primer 328. The custom primers 326 can hybridize to a region within the cDNA portion 306' of the labeled cDNA molecule 304. The universal primer 328 can hybridize to the universal PCR region 316 of the labeled cDNA molecule 304.

As shown in step 3 of FIG. 3, products from PCR amplification in step 2 can be amplified with a nested PCR primers pool and a 2^{nd} universal PCR primer. Nested PCR can minimize PCR amplification bias. In particular, the nested PCR labeled amplicons 322 can be further amplified by nested PCR. The nested PCR can comprise multiplex PCR with nested PCR primers pool 330 of nested PCR primers 332a-c and a 2^{nd} universal PCR primer 328' in a single reaction volume. The nested PCR primer pool 328 can contain, contain about, contain at least, or contain at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different nested PCR primers 330. The nested PCR primers 332 can contain an adaptor 334 and hybridize to a region within the cDNA portion 306" of the labeled amplicon 322. The universal primer 328' can contain an adaptor 336 and hybridize to the universal PCR region 316 of the labeled amplicon 322. Thus, step 3 produces adaptor-labeled amplicon 338. In some embodiments, nested PCR primers 332 and the 2^{nd} universal PCR primer 328' may not contain the adaptors 334 and 336. The adaptors 334 and 336 can instead be ligated to the products of nested PCR to produce adaptor-labeled amplicon 338.

As shown in step 4, PCR products from step 3 can be PCR amplified for sequencing using library amplification primers. In particular, the adaptors 334 and 336 can be used to conduct one or more additional assays on the adaptor-labeled amplicon 338. The adaptors 334 and 336 can be hybridized to primers 340 and 342. The one or more primers 340 and 342 can be PCR amplification primers. The one or more primers 340 and 342 can be sequencing primers. The one or more adaptors 334 and 336 can be used for further amplification of the adaptor-labeled amplicons 338. The one or more adaptors 334 and 336 can be used for sequencing the adaptor-labeled amplicon 338. The primer 342 can contain a plate index 344 so that amplicons generated using the same set of barcodes or stochastic barcodes 310 can be sequenced in one sequencing reaction using next generation sequencing (NGS).

### Compositions Comprising Cellular Component Binding Reagents Associated with Oligonucleotides

Some embodiments disclosed herein provide a plurality of compositions each comprising a cellular component binding reagent (such as a protein binding reagent) that is conjugated with an oligonucleotide, wherein the oligonucleotide comprises a unique identifier for the cellular component binding reagent that it is conjugated with. Cellular component binding reagents (such as barcoded antibodies) and their uses (such as sample indexing of cells) have been described in U.S. Patent Application Publication No. 2018/0088112 and U.S. Patent Application No. 15/937,713.

In some embodiments, the cellular component binding reagent is capable of specifically binding to a cellular component target. For example, a binding target of the cellular component binding reagent can be, or comprise, a carbohydrate, a lipid, a protein, an extracellular protein, a cell-surface protein, a cell marker, a B-cell receptor, a T-cell receptor, a major histocompatibility complex, a tumor antigen, a receptor, an integrin, an intracellular protein, or any combination thereof. In some embodiments, the cellular component binding reagent (e.g., a protein binding reagent) is capable of specifically binding to an antigen target or a protein target. In some embodiments, each of the oligonucleotides can comprise a barcode, such as a stochastic barcode. A barcode can comprise a barcode sequence (e.g., a molecular label), a cell label, a sample label, or any combination thereof. In some embodiments, each of the oligonucleotides can comprise a linker. In some embodiments, each of the oligonucleotides can comprise a binding site for an oligonucleotide probe, such as a poly(A) tail. For example, the poly(A) tail can be, e.g., unanchored to a solid support or anchored to a solid support. The poly(A) tail can be from about 10 to 50 nucleotides in length. In some embodiments, the poly(A) tail can be 18 nucleotides in length. The oligonucleotides can comprise deoxyribonucleotides, ribonucleotides, or both.

The unique identifiers can be, for example, a nucleotide sequence having any suitable length, for example, from about 4 nucleotides to about 200 nucleotides. In some embodiments, the unique identifier is a nucleotide sequence of 25 nucleotides to about 45 nucleotides in length. In some embodiments, the unique identifier can have a length that is, is about, is less than, is greater than, 4 nucleotides, 5 nucleotides, 6 nucleotides, 7 nucleotides, 8 nucleotides, 9 nucleotides, 10 nucleotides, 15 nucleotides, 20 nucleotides, 25 nucleotides, 30 nucleotides, 35 nucleotides, 40 nucleotides, 45 nucleotides, 50 nucleotides, 55 nucleotides, 60 nucleotides, 70 nucleotides, 80 nucleotides, 90 nucleotides, 100 nucleotides, 200 nucleotides, or a range that is between any two of the above values.

In some embodiments, the unique identifiers are selected from a diverse set of unique identifiers. The diverse set of unique identifiers can comprise, or comprise about, 20, 30, 40, 50, 60, 70, 80, 90, 100, 200, 300, 400, 500, 600, 700, 800, 900, 1000, 2000, 5000, or a number or a range between any two of these values, different unique identifiers. The diverse set of unique identifiers can comprise at least, or comprise at most, 20, 30, 40, 50, 60, 70, 80, 90, 100, 200, 300, 400, 500, 600, 700, 800, 900, 1000, 2000, or 5000, different unique identifiers. In some embodiments, the set of unique identifiers is designed to have minimal sequence homology to the DNA or RNA sequences of the sample to be analyzed. In some embodiments, the sequences of the set of unique identifiers are different from each other, or the complement thereof, by, or by about, 1 nucleotide, 2 nucleotides, 3 nucleotides, 4 nucleotides, 5 nucleotides, 6 nucleotides, 7 nucleotides, 8 nucleotides, 9 nucleotides, 10 nucleotides, or a number or a range between any two of these values. In some embodiments, the sequences of the set of unique identifiers are different from each other, or the complement thereof, by at least, or by at most, 1 nucleotide, 2 nucleotides, 3 nucleotides, 4 nucleotides, 5 nucleotides, 6 nucleotides, 7 nucleotides, 8 nucleotides, 9 nucleotides, 10 nucleotides. In some embodiments, the sequences of the set of unique identifiers are different from each other, or the complement thereof, by at least 3%, at least 5%, at least 8%, at least 10%, at least 15%, at least 20%, or more.

In some embodiments, the unique identifiers can comprise a binding site for a primer, such as universal primer. In some embodiments, the unique identifiers can comprise at least two binding sites for a primer, such as a universal primer. In some embodiments, the unique identifiers can comprise at least three binding sites for a primer, such as a universal primer. The primers can be used for amplification of the unique identifiers, for example, by PCR amplification. In some embodiments, the primers can be used for nested PCR reactions.

Any suitable cellular component binding reagents are contemplated in this disclosure, such as protein binding reagents, antibodies or fragments thereof, aptamers, small molecules, ligands, peptides, oligonucleotides, etc., or any combination thereof. In some embodiments, the cellular component binding reagents can be polyclonal antibodies, monoclonal antibodies, recombinant antibodies, single chain antibody (sc-Ab), or fragments thereof, such as Fab, Fv, etc. In some embodiments, the plurality of cellular component binding reagents can comprise, or comprise about, 20, 30, 40, 50, 60, 70, 80, 90, 100, 200, 300, 400, 500, 600, 700, 800, 900, 1000, 2000, 5000, or a number or a range between any two of these values, different cellular component reagents. In some embodiments, the plurality of cellular component binding reagents can comprise at least, or comprise at most, 20, 30, 40, 50, 60, 70, 80, 90, 100, 200, 300, 400, 500, 600, 700, 800, 900, 1000, 2000, or 5000, different cellular component reagents.

The oligonucleotide can be conjugated with the cellular component binding reagent through various mechanism. In some embodiments, the oligonucleotide can be conjugated with the cellular component binding reagent covalently. In some embodiment, the oligonucleotide can be conjugated with the cellular component binding reagent non-covalently. In some embodiments, the oligonucleotide is conjugated with the cellular component binding reagent through a linker. The linker can be, for example, cleavable or detachable from the cellular component binding reagent and/or the oligonucleotide. In some embodiments, the linker can comprise a chemical group that reversibly attaches the oligonucleotide to the cellular component binding reagents. The chemical group can be conjugated to the linker, for example, through an amine group. In some embodiments, the linker can comprise a chemical group that forms a stable bond with another chemical group conjugated to the cellular component binding reagent. For example, the chemical group can be a UV photocleavable group, a disulfide bond, a streptavidin, a biotin, an amine, etc. In some embodiments, the chemical group can be conjugated to the cellular component binding reagent through a primary amine on an amino acid, such as lysine, or the N-terminus. Commercially available conjugation kits, such as the Protein-Oligo Conjugation Kit (Solulink, Inc., San Diego, California), the Thunder-Link^{®} oligo conjugation system (Innova Biosciences, Cambridge, United Kingdom), etc., can be used to conjugate the oligonucleotide to the cellular component binding reagent.

The oligonucleotide can be conjugated to any suitable site of the cellular component binding reagent (e.g., a protein binding reagent), as long as it does not interfere with the specific binding between the cellular component binding reagent and its cellular component target. In some embodiments, the cellular component binding reagent is a protein, such as an antibody. In some embodiments, the cellular component binding reagent is not an antibody. In some embodiments, the oligonucleotide can be conjugated to the antibody anywhere other than the antigen-binding site, for example, the Fc region, the C_{H}1 domain, the C_{H}2 domain, the C_{H}3 domain, the C_{L} domain, etc. Methods of conjugating oligonucleotides to cellular component binding reagents (e.g., antibodies) have been previously disclosed, for example, in U.S. Patent. No. 6,531,283. Stoichiometry of oligonucleotide to cellular component binding reagent can be varied. To increase the sensitivity of detecting the cellular component binding reagent specific oligonucleotide in sequencing, it may be advantageous to increase the ratio of oligonucleotide to cellular component binding reagent during conjugation. In some embodiments, each cellular component binding reagent can be conjugated with a single oligonucleotide molecule. In some embodiments, each cellular component binding reagent can be conjugated with more than one oligonucleotide molecule, for example, at least, or at most, 2, 3, 4, 5, 10, 20, 30, 40, 50, 100, 1000, or a number or a range between any two of these values, oligonucleotide molecules wherein each of the oligonucleotide molecule comprises the same, or different, unique identifiers. In some embodiments, each cellular component binding reagent can be conjugated with more than one oligonucleotide molecule, for example, at least, or at most, 2, 3, 4, 5, 10, 20, 30, 40, 50, 100, 1000, oligonucleotide molecules, wherein each of the oligonucleotide molecule comprises the same, or different, unique identifiers.

In some embodiments, the plurality of cellular component binding reagents are capable of specifically binding to a plurality of cellular component targets in a sample, such as a single cell, a plurality of cells, a tissue sample, a tumor sample, a blood sample, or the like. In some embodiments, the plurality of cellular component targets comprises a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular cellular components. In some embodiments, the plurality of cellular component targets can comprise intracellular cellular components. In some embodiments, the plurality of cellular components can be, or be about, 1%, 2%, 3%, 4%, 5%, 6%, 7%, 8%, 9%, 10%, 20%, 30%, 40%, 50%, 60%, 70%, 80%, 90%, 95%, 98%, 99%, or a number or a range between any two of these values, of all the cellular components (e.g., proteins) in a cell or an organism. In some embodiments, the plurality of cellular components can be at least, or be at most, 1%, 2%, 3%, 4%, 5%, 6%, 7%, 8%, 9%, 10%, 20%, 30%, 40%, 50%, 60%, 70%, 80%, 90%, 95%, 98%, or 99%, of all the cellular components (e.g., proteins) in a cell or an organism. In some embodiments, the plurality of cellular component targets can comprise, or comprise about, 2, 3, 4, 5, 10, 20, 30, 40, 50, 100, 1000, 10000, or a number or a range between any two of these values, different cellular component targets. In some embodiments, the plurality of cellular component targets can comprise at least, or comprise at most, 2, 3, 4, 5, 10, 20, 30, 40, 50, 100, 1000, 10000, different cellular component targets.

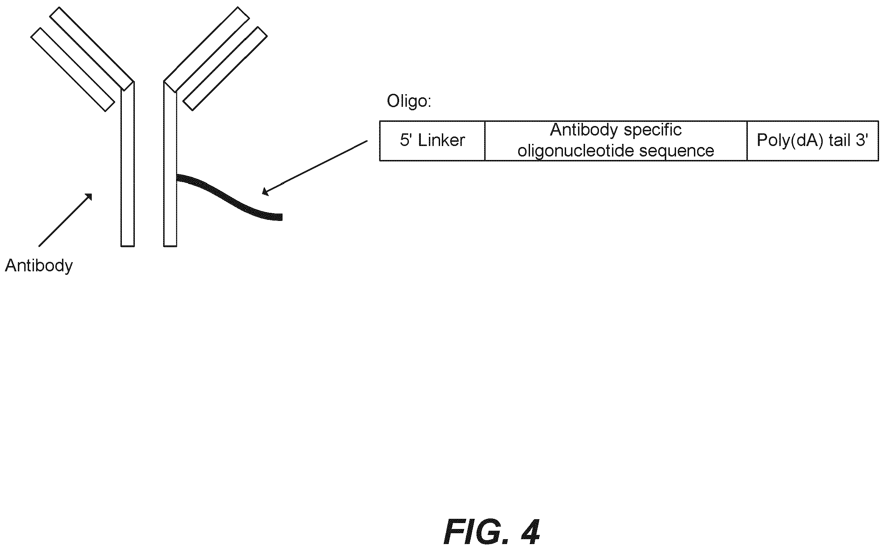

FIG. 4 shows a schematic illustration of an exemplary cellular component binding reagent (e.g., an antibody) that is associated (e.g., conjugated) with an oligonucleotide comprising a unique identifier sequence for the antibody. An oligonucleotide-conjugated with a cellular component binding reagent, an oligonucleotide for conjugation with a cellular component binding reagent, or an oligonucleotide previously conjugated with a cellular component binding reagent can be referred to herein as an antibody oligonucleotide (abbreviated as a binding reagent oligonucleotide). An oligonucleotide-conjugated with an antibody, an oligonucleotide for conjugation with an antibody, or an oligonucleotide previously conjugated with an antibody can be referred to herein as an antibody oligonucleotide (abbreviated as an "AbOligo" or "AbO"). The oligonucleotide can also comprise additional components, including but not limited to, one or more linker, one or more unique identifier for the antibody, optionally one or more barcode sequences (e.g., molecular labels), and a poly(dA) tail. In some embodiments, the oligonucleotide can comprise, from 5' to 3', a linker, a unique identifier, a barcode sequence (e.g., a molecular label), and a poly(dA) tail. An antibody oligonucleotide can be an mRNA mimic.

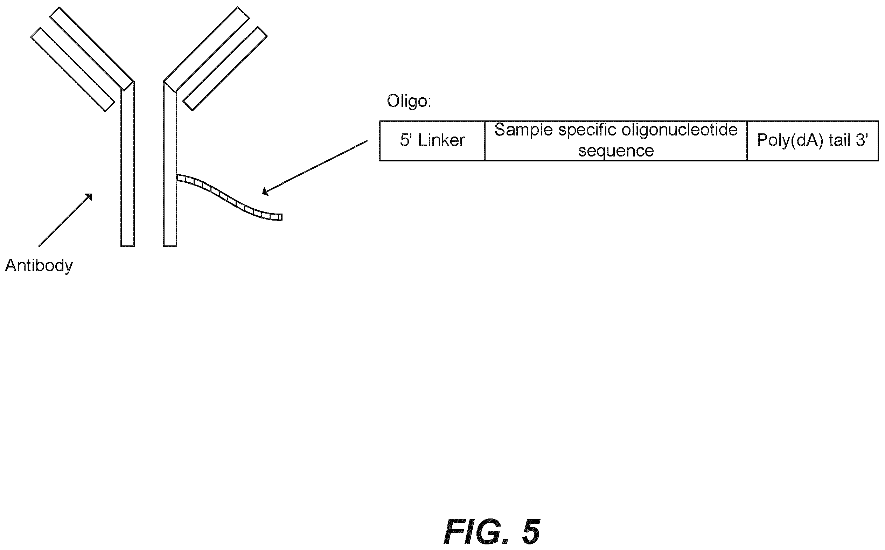

FIG. 5 shows a schematic illustration of an exemplary cellular component binding reagent (e.g., an antibody) that is associated (e.g., conjugated) with an oligonucleotide comprising a unique identifier sequence for the antibody. The cellular component binding reagent can be capable of specifically binding to at least one cellular component target, such as an antigen target or a protein target. A binding reagent oligonucleotide (e.g., a sample indexing oligonucleotide, or an antibody oligonucleotide) can comprise a sequence (e.g., a sample indexing sequence) for performing the methods of the disclosure. For example, a sample indexing oligonucleotide can comprise a sample indexing sequence for identifying sample origin of one or more cells of a sample. Indexing sequences (e.g., sample indexing sequences) of at least two compositions comprising two cellular component binding reagents (e.g., sample indexing compositions) of the plurality of compositions comprising cellular component binding reagents can comprise different sequences. In some embodiments, the binding reagent oligonucleotide is not homologous to genomic sequences of a species. The binding reagent oligonucleotide can be configured to be (or can be) detachable or non-detachable from the cellular component binding reagent.

The oligonucleotide conjugated to a cellular component binding reagent can, for example, comprise a barcode sequence (e.g., a molecular label sequence), a poly(dA) tail, or a combination thereof. An oligonucleotide conjugated to a cellular component binding reagent can be an mRNA mimic. In some embodiments, the sample indexing oligonucleotide comprises a sequence complementary to a capture sequence of at least one barcode of the plurality of barcodes. A target binding region of the barcode can comprise the capture sequence. The target binding region can, for example, comprise a poly(dT) region. In some embodiments, the sequence of the sample indexing oligonucleotide complementary to the capture sequence of the barcode can comprise a poly(dA) tail. The sample indexing oligonucleotide can comprise a molecular label.

In some embodiments, the binding reagent oligonucleotide (e.g., the sample oligonucleotide) comprises a nucleotide sequence of, or a nucleotide sequence of about, 6, 7, 8, 9, 10, 11, 12, 13, 14, 15, 16, 17, 18, 19, 20, 25, 30, 35, 40, 45, 50, 60, 70, 80, 90, 100, 110, 120, 128, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values, nucleotides in length. In some embodiments, the binding reagent oligonucleotide comprises a nucleotide sequence of at least, or of at most, 6, 7, 8, 9, 10, 11, 12, 13, 14, 15, 16, 17, 18, 19, 20, 25, 30, 35, 40, 45, 50, 60, 70, 80, 90, 100, 110, 120, 128, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000, nucleotides in length.

In some embodiments, the cellular component binding reagent comprises an antibody, a tetramer, an aptamers, a protein scaffold, or a combination thereof. The binding reagent oligonucleotide can be conjugated to the cellular component binding reagent, for example, through a linker. The binding reagent oligonucleotide can comprise the linker. The linker can comprise a chemical group. The chemical group can be reversibly, or irreversibly, attached to the molecule of the cellular component binding reagent. The chemical group can be selected from the group consisting of a UV photocleavable group, a disulfide bond, a streptavidin, a biotin, an amine, and any combination thereof.

In some embodiments, the cellular component binding reagent can bind to ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2, or any combination thereof.

In some embodiments, the protein target is, or comprises, an extracellular protein, an intracellular protein, or any combination thereof. In some embodiments, the antigen or protein target is, or comprises, a cell-surface protein, a cell marker, a B-cell receptor, a T-cell receptor, a major histocompatibility complex, a tumor antigen, a receptor, an integrin, or any combination thereof. The antigen or protein target can be, or comprise, a lipid, a carbohydrate, or any combination thereof. The protein target can be selected from a group comprising a number of protein targets. The number of antigen target or protein targets can be, or be about, 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, or a number or a range between any two of these values. The number of protein targets can be at least, or be at most, 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, or 10000.

The cellular component binding reagent (e.g., a protein binding reagent) can be associated with two or more binding reagent oligonucleotide (e.g., sample indexing oligonucleotides) with an identical sequence. The cellular component binding reagent can be associated with two or more binding reagent oligonucleotides with different sequences. The number of binding reagent oligonucleotides associated with the cellular component binding reagent can be different in different implementations. In some embodiments, the number of binding reagent oligonucleotides , whether having an identical sequence, or different sequences, can be, or be about, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any two of these values. In some embodiments, the number of binding reagent oligonucleotides can be at least, or be at most, 1, 2, 3, 4, 5, 6, 7, 8, 9, 10, 20, 30, 40, 50, 60, 70, 80, 90, 100, 200, 300, 400, 500, 600, 700, 800, 900, or 1000.

The plurality of compositions comprising cellular component binding reagents (e.g., the plurality of sample indexing compositions) can comprise one or more additional cellular component binding reagents not conjugated with the binding reagent oligonucleotide (such as sample indexing oligonucleotide), which is also referred to herein as the binding reagent oligonucleotide-free cellular component binding reagent (such as sample indexing oligonucleotide-free cellular component binding reagent). The number of additional cellular component binding reagents in the plurality of compositions can be different in different implementations. In some embodiments, the number of additional cellular component binding reagents can be, or be about, 1, 2, 3, 4, 5, 6, 7, 8, 9, 10, 20, 30, 40, 50, 60, 70, 80, 90, 100, or a number or a range between any two of these values. In some embodiments, the number of additional cellular component binding reagents can be at least, or be at most, 1, 2, 3, 4, 5, 6, 7, 8, 9, 10, 20, 30, 40, 50, 60, 70, 80, 90, or 100. The cellular component binding reagent and any of the additional cellular component binding reagents can be identical, in some embodiments.

In some embodiments, a mixture comprising cellular component binding reagent(s) that is conjugated with one or more binding reagent oligonucleotides (e.g., sample indexing oligonucleotides) and cellular component binding reagent(s) that is not conjugated with binding reagent oligonucleotides is provided. The mixture can be used in some embodiments of the methods disclosed herein, for example, to contact the sample(s) and/or cell(s). The ratio of (1) the number of a cellular component binding reagent conjugated with a binding reagent oligonucleotide and (2) the number of another cellular component binding reagent (e.g., the same cellular component binding reagent) not conjugated with the binding reagent oligonucleotide (e.g., sample indexing oligonucleotide) or other binding reagent oligonucleotide(s) in the mixture can be different in different implementations. In some embodiments, the ratio can be, or be about, 1:1, 1:1.1, 1:1.2, 1:1.3, 1:1.4, 1:1.5, 1:1.6, 1:1.7, 1:1.8, 1:1.9, 1:2, 1:2.5, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, 1:41, 1:42, 1:43, 1:44, 1:45, 1:46, 1:47, 1:48, 1:49, 1:50, 1:51, 1:52, 1:53, 1:54, 1:55, 1:56, 1:57, 1:58, 1:59, 1:60, 1:61, 1:62, 1:63, 1:64, 1:65, 1:66, 1:67, 1:68, 1:69, 1:70, 1:71, 1:72, 1:73, 1:74, 1:75, 1:76, 1:77, 1:78, 1:79, 1:80, 1:81, 1:82, 1:83, 1:84, 1:85, 1:86, 1:87, 1:88, 1:89, 1:90, 1:91, 1:92, 1:93, 1:94, 1:95, 1:96, 1:97, 1:98, 1:99, 1:100, 1:200, 1:300, 1:400, 1:500, 1:600, 1:700, 1:800, 1:900, 1:1000, 1:2000, 1:3000, 1:4000, 1:5000, 1:6000, 1:7000, 1:8000, 1:9000, 1:10000, or a number or a range between any two of the values. In some embodiments, the ratio can be at least, or be at most, 1:1, 1:1.1, 1:1.2, 1:1.3, 1:1.4, 1:1.5, 1:1.6, 1:1.7, 1:1.8, 1:1.9, 1:2, 1:2.5, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, 1:41, 1:42, 1:43, 1:44, 1:45, 1:46, 1:47, 1:48, 1:49, 1:50, 1:51, 1:52, 1:53, 1:54, 1:55, 1:56, 1:57, 1:58, 1:59, 1:60, 1:61, 1:62, 1:63, 1:64, 1:65, 1:66, 1:67, 1:68, 1:69, 1:70, 1:71, 1:72, 1:73, 1:74, 1:75, 1:76, 1:77, 1:78, 1:79, 1:80, 1:81, 1:82, 1:83, 1:84, 1:85, 1:86, 1:87, 1:88, 1:89, 1:90, 1:91, 1:92, 1:93, 1:94, 1:95, 1:96, 1:97, 1:98, 1:99, 1:100, 1:200, 1:300, 1:400, 1:500, 1:600, 1:700, 1:800, 1:900, 1:1000, 1:2000, 1:3000, 1:4000, 1:5000, 1:6000, 1:7000, 1:8000, 1:9000, or 1:10000.

In some embodiments, the ratio can be, or be about, 1:1, 1.1:1, 1.2:1, 1.3:1, 1.4:1, 1.5:1, 1.6:1, 1.7:1, 1.8:1, 1.9:1, 2:1, 2.5:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1, 200:1, 300:1, 400:1, 500:1, 600:1, 700:1, 800:1, 900:1, 1000:1, 2000:1, 3000:1, 4000:1, 5000:1, 6000:1, 7000:1, 8000:1, 9000:1, 10000:1, or a number or a range between any two of the values. In some embodiments, the ratio can be at least, or be at most, 1:1, 1.1:1, 1.2:1, 1.3:1, 1.4:1, 1.5:1, 1.6:1, 1.7:1, 1.8:1, 1.9:1, 2:1, 2.5:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1, 200:1, 300:1, 400:1, 500:1, 600:1, 700:1, 800:1, 900:1, 1000:1, 2000:1, 3000:1, 4000:1, 5000:1, 6000:1, 7000:1, 8000:1, 9000:1, or 10000:1.

A cellular component binding reagent can be conjugated with a binding reagent oligonucleotide (e.g., a sample indexing oligonucleotide), or not. In some embodiments, the percentage of the cellular component binding reagent conjugated with a binding reagent oligonucleotide (e.g., a sample indexing oligonucleotide) in a mixture comprising the cellular component binding reagent that is conjugated with the binding reagent oligonucleotide and the cellular component binding reagent(s) that is not conjugated with the binding reagent oligonucleotide can be, or be about, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values. In some embodiments, the percentage of the cellular component binding reagent conjugated with a sample indexing oligonucleotide in a mixture can be at least, or be at most, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

In some embodiments, the percentage of the cellular component binding reagent not conjugated with a binding reagent oligonucleotide (e.g., a sample indexing oligonucleotide) in a mixture comprising a cellular component binding reagent conjugated with a binding reagent oligonucleotide (e.g., a sample indexing oligonucleotide) and the cellular component binding reagent that is not conjugated with the sample indexing oligonucleotide can be, or be about, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values. In some embodiments, the percentage of the cellular component binding reagent not conjugated with a binding reagent oligonucleotide in a mixture can be at least, or be at most, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

### Cellular Component Cocktails

In some embodiments, a cocktail of cellular component binding reagents (e.g., an antibody cocktail) can be used to increase labeling sensitivity in the methods disclosed herein. Without being bound by any particular theory, it is believed that this may be because cellular component expression or protein expression can vary between cell types and cell states, making finding a universal cellular component binding reagent or antibody that labels all cell types challenging. For example, cocktail of cellular component binding reagents can be used to allow for more sensitive and efficient labeling of more sample types. The cocktail of cellular component binding reagents can include two or more different types of cellular component binding reagents, for example a wider range of cellular component binding reagents or antibodies. Cellular component binding reagents that label different cellular component targets can be pooled together to create a cocktail that sufficiently labels all cell types, or one or more cell types of interest.

In some embodiments, each of the plurality of compositions (e.g., sample indexing compositions) comprises a cellular component binding reagent. In some embodiments, a composition of the plurality of compositions comprises two or more cellular component binding reagents, wherein each of the two or more cellular component binding reagents is associated with a binding reagent oligonucleotide (e.g., a sample indexing oligonucleotide), wherein at least one of the two or more cellular component binding reagents is capable of specifically binding to at least one of the one or more cellular component targets. The sequences of the binding reagent oligonucleotides associated with the two or more cellular component binding reagents can be identical. The sequences of the binding reagent oligonucleotides associated with the two or more cellular component binding reagents can comprise different sequences. Each of the plurality of compositions can comprise the two or more cellular component binding reagents.

The number of different types of cellular component binding reagents (e.g., a CD147 antibody and a CD47 antibody) in a composition can be different in different implementations. A composition with two or more different types of cellular component binding reagents can be referred to herein as a cellular component binding reagent cocktail (e.g., a sample indexing composition cocktail). The number of different types of cellular component binding reagents in a cocktail can vary. In some embodiments, the number of different types of cellular component binding reagents in cocktail can be, or be about 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, or a number or a range between any two of these values. In some embodiments, the number of different types of cellular component binding reagents in cocktail can be at least, or be at most, 2, 3, 4, 5, 6, 7, 8, 9, 10, 20, 30, 40, 50, 60, 70, 80, 90, 100, 200, 300, 400, 500, 600, 700, 800, 900, 1000, 10000, or 100000. The different types of cellular component binding reagents can be conjugated to binding reagent oligonucleotides with the same or different sequences (e.g., sample indexing sequences).

### Methods of Quantitative Analysis of Cellular Component Targets

In some embodiments, the methods disclosed herein can also be used for quantitative analysis of a plurality of cellular component targets (for example, protein targets) in a sample using the compositions disclosed herein and oligonucleotide probes that can associate a barcode sequence (e.g., a molecular label sequence) to the oligonucleotides of the cellular component binding reagents (e.g., protein binding reagents). The oligonucleotides of the cellular component binding reagents can be, or comprise, an antibody oligonucleotide, a sample indexing oligonucleotide, a cell identification oligonucleotide, a control particle oligonucleotide, a control oligonucleotide, an interaction determination oligonucleotide, etc. In some embodiments, the sample can be a single cell, a plurality of cells, a tissue sample, a tumor sample, a blood sample, or the like. In some embodiments, the sample can comprise a mixture of cell types, such as normal cells, tumor cells, blood cells, B cells, T cells, maternal cells, fetal cells, etc., or a mixture of cells from different subjects.

In some embodiments, the sample can comprise a plurality of single cells separated into individual compartments, such as microwells in a microwell array.

In some embodiments, the binding target of the plurality of cellular component target (i.e., the cellular component target) can be, or comprise, a carbohydrate, a lipid, a protein, an extracellular protein, a cell-surface protein, a cell marker, a B-cell receptor, a T-cell receptor, a major histocompatibility complex, a tumor antigen, a receptor, an integrin, an intracellular protein, or any combination thereof. In some embodiments, the cellular component target is a protein target. In some embodiments, the plurality of cellular component targets comprises a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular cellular components. In some embodiments, the plurality of cellular components can be at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90%, at least 95%, at least 98%, at least 99%, or more, of all the encoded cellular components in an organism. In some embodiments, the plurality of cellular component targets can comprise at least 2, at least 3, at least 4, at least 5, at least 10, at least 20, at least 30, at least 40, at least 50, at least 100, at least 1000, at least 10000, or more different cellular component targets.

In some embodiments, the plurality of cellular component binding reagents is contacted with the sample for specific binding with the plurality of cellular component targets. Unbound cellular component binding reagents can be removed, for example, by washing. In embodiments where the sample comprises cells, any cellular component binding reagents not specifically bound to the cells can be removed.

In some instances, cells from a population of cells can be separated (e.g., isolated) into wells of a substrate of the disclosure. The population of cells can be diluted prior to separating. The population of cells can be diluted such that at least 1%, 5%, 10%, 15%, 20%, 25%, 30%, 35%, 40%, 45%, 50%, 55%, 60%, 65%, 70%, 75%, 80%, 85%, 90%, 95% or 100%, of wells of the substrate receive a single cell. The population of cells can be diluted such that at most 1%, 5%, 10%, 15%, 20%, 25%, 30%, 35%, 40%, 45%, 50%, 55%, 60%, 65%, 70%, 75%, 80%, 85%, 90%, 95%, or 100%, of wells of the substrate receive a single cell. The population of cells can be diluted such that the number of cells in the diluted population is, or is at least, 1%, 5%, 10%, 15%, 20%, 25%, 30%, 35%, 40%, 45%, 50%, 55%, 60%, 65%, 70%, 75%, 80%, 85%, 90%, 95%, or 100%, of the number of wells on the substrate. The population of cells can be diluted such that the number of cells in the diluted population is, or is at least, 1%, 5%, 10%, 15%, 20%, 25%, 30%, 35%, 40%, 45%, 50%, 55%, 60%, 65%, 70%, 75%, 80%, 85%, 90%, 95%, or 100%, of the number of wells on the substrate. In some instances, the population of cells is diluted such that the number of cell is about 10% of the number of wells in the substrate.

Distribution of single cells into wells of the substrate can follow a Poisson distribution. For example, there can be at least a 0.1%, 0.5%, 1%, 2%, 3%, 4%, 5%, 6%, 7%, 8%, 9%, or 10%, or more probability that a well of the substrate has more than one cell. There can be at least a 0.1%, 0.5%, 1%, 2%, 3%, 4%, 5%, 6%, 7%, 8%, 9%, or 10%, or more probability that a well of the substrate has more than one cell. Distribution of single cells into wells of the substrate can be random. Distribution of single cells into wells of the substrate can be non-random. The cells can be separated such that a well of the substrate receives only one cell.

In some embodiments, the cellular component binding reagents can be additionally conjugated with fluorescent molecules to enable flow sorting of cells into individual compartments.

In some embodiments, the methods disclosed herein provide contacting a plurality of compositions with the sample for specific binding with the plurality of cellular component targets. It would be appreciated that the conditions used may allow specific binding of the cellular component binding reagents, e.g., antibodies, to the cellular component targets. Following the contacting step, unbound compositions can be removed. For example, in embodiments where the sample comprises cells, and the compositions specifically bind to cellular component targets are cell-surface cellular components, such as cell-surface proteins, unbound compositions can be removed by washing the cells with buffer such that only compositions that specifically bind to the cellular component targets remain with the cells.

In some embodiments, the methods disclosed herein can comprise associating an oligonucleotide (e.g., a barcode, or a stochastic barcode), including a barcode sequence (such as a molecular label), a cell label, a sample label, etc., or any combination thereof, to the plurality of oligonucleotides associated with the cellular component binding reagents. For example, a plurality of oligonucleotide probes comprising a barcode can be used to hybridize to the plurality of oligonucleotides of the compositions.

In some embodiments, the plurality of oligonucleotide probes can be immobilized on solid supports. The solid supports can be free floating, e.g., beads in a solution. The solid supports can be embedded in a semi-solid or solid array. In some embodiments, the plurality of oligonucleotide probes may not be immobilized on solid supports. When the plurality of oligonucleotide probes are in close proximity to the plurality associated with oligonucleotides of the cellular component binding reagents, the plurality of oligonucleotides of the cellular component binding reagents can hybridize to the oligonucleotide probes. The oligonucleotide probes can be contacted at a non-depletable ratio such that each distinct oligonucleotide of the cellular component binding reagents can associate with oligonucleotide probes having different barcode sequences (e.g., molecular labels) of the disclosure.

In some embodiments, the methods disclosed herein provide detaching the oligonucleotides from the cellular component binding reagents that are specifically bound to the cellular component targets. Detachment can be performed in a variety of ways to separate the chemical group from the cellular component binding reagent, such as UV photocleaving, chemical treatment (e.g., dithiothreitol treatment), heating, enzyme treatment, or any combination thereof. Detaching the oligonucleotide from the cellular component binding reagent can be performed either before, after, or during the step of hybridizing the plurality of oligonucleotide probes to the plurality of oligonucleotides of the compositions.

### Methods of Simultaneous Quantitative Analysis of Cellular Component and Nucleic Acid Targets

In some embodiments, the methods disclosed herein can also be used for simultaneous quantitative analysis of a plurality of cellular component targets (e.g., protein targets) and a plurality of nucleic acid target molecules in a sample using the compositions disclosed herein and oligonucleotide probes that can associate a barcode sequence (e.g., a molecular label sequence) to both the oligonucleotides of the cellular component binding reagents and nucleic acid target molecules. Other methods of simultaneous quantitative analysis of a plurality of cellular component targets and a plurality of nucleic acid target molecules are described in U.S. Patent Application Number 15/715028, filed on September 25, 2017. In some embodiments, the sample can be a single cell, a plurality of cells, a tissue sample, a tumor sample, a blood sample, or the like. In some embodiments, the sample can comprise a mixture of cell types, such as normal cells, tumor cells, blood cells, B cells, T cells, maternal cells, fetal cells, or a mixture of cells from different subjects.

In some embodiments, the sample can comprise a plurality of single cells separated into individual compartments, such as microwells in a microwell array.

In some embodiments, the plurality of cellular component targets comprises a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular cellular components. In some embodiments, the plurality of cellular components can be, or be about, 1%, 2%, 3%, 4%, 5%, 6%, 7%, 8%, 9%, 10%, 20%, 30%, 40%, 50%, 60%, 70%, 80%, 90%, 95%, 98%, 99%, or a number or a range between any two of these values, of all the cellular components, such as expressed proteins, in an organism, or one or more cells of the organism. In some embodiments, the plurality of cellular components can be at least, or be at most, 1%, 2%, 3%, 4%, 5%, 6%, 7%, 8%, 9%, 10%, 20%, 30%, 40%, 50%, 60%, 70%, 80%, 90%, 95%, 98%, or 99%, of all the cellular components, such as proteins could be expressed, in an organism, or one or more cells of the organism. In some embodiments, the plurality of cellular component targets can comprise, or comprise about, 2, 3, 4, 5, 10, 20, 30, 40, 50, 100, 1000, 10000, or a number or a range between any two of these values, different cellular component targets. In some embodiments, the plurality of cellular component targets can comprise at least, or comprise at most, 2, 3, 4, 5, 10, 20, 30, 40, 50, 100, 1000, or 10000, different cellular component targets.

In some embodiments, the plurality of cellular component binding reagents is contacted with the sample for specific binding with the plurality of cellular component targets. Unbound cellular component binding reagents can be removed, for example, by washing. In embodiments where the sample comprises cells, any cellular component binding reagents not specifically bound to the cells can be removed.

In some instances, cells from a population of cells can be separated (e.g., isolated) into wells of a substrate of the disclosure. The population of cells can be diluted prior to separating. The population of cells can be diluted such that at least 1%, 5%, 10%, 15%, 20%, 25%, 30%, 35%, 40%, 45%, 50%, 55%, 60%, 65%, 70%, 75%, 80%, 85%, 90%, 95%, or 100% of wells of the substrate receive a single cell. The population of cells can be diluted such that at most 1%, 5%, 10%, 15%, 20%, 25%, 30%, 35%, 40%, 45%, 50%, 55%, 60%, 65%, 70%, 75%, 80%, 85%, 90%, 95%, or 100% of wells of the substrate receive a single cell. The population of cells can be diluted such that the number of cells in the diluted population is, or is at least, 1%, 5%, 10%, 15%, 20%, 25%, 30%, 35%, 40%, 45%, 50%, 55%, 60%, 65%, 70%, 75%, 80%, 85%, 90%, 95%, or 100% of the number of wells on the substrate. The population of cells can be diluted such that the number of cells in the diluted population is, or is at least, 1%, 5%, 10%, 15%, 20%, 25%, 30%, 35%, 40%, 45%, 50%, 55%, 60%, 65%, 70%, 75%, 80%, 85%, 90%, 95%, or 100% of the number of wells on the substrate. In some instances, the population of cells is diluted such that the number of cell is about 10% of the number of wells in the substrate.

Distribution of single cells into wells of the substrate can follow a Poisson distribution. For example, there can be at least a 0.1%, 0.5%, 1%, 2%, 3%, 4%, 5%, 6%, 7%, 8%, 9%, or 10%, or more probability that a well of the substrate has more than one cell. There can be at least a 0.1%, 0.5%, 1%, 2%, 3%, 4%, 5%, 6%, 7%, 8%, 9%, or 10%, or more probability that a well of the substrate has more than one cell. Distribution of single cells into wells of the substrate can be random. Distribution of single cells into wells of the substrate can be non-random. The cells can be separated such that a well of the substrate receives only one cell.

In some embodiments, the cellular component binding reagents can be additionally conjugated with fluorescent molecules to enable flow sorting of cells into individual compartments.

In some embodiments, the methods disclosed herein provide contacting a plurality of compositions with the sample for specific binding with the plurality of cellular component targets. It would be appreciated that the conditions used may allow specific binding of the cellular component binding reagents, e.g., antibodies, to the cellular component targets. Following the contacting step, unbound compositions can be removed. For example, in embodiments where the sample comprises cells, and the compositions specifically bind to cellular component targets are on the cell surface, such as cell-surface proteins, unbound compositions can be removed by washing the cells with buffer such that only compositions that specifically bind to the cellular component targets remain with the cells.

In some embodiments, the methods disclosed herein can provide releasing the plurality of nucleic acid target molecules from the sample, e.g., cells. For example, the cells can be lysed to release the plurality of nucleic acid target molecules. Cell lysis may be accomplished by any of a variety of means, for example, by chemical treatment, osmotic shock, thermal treatment, mechanical treatment, optical treatment, or any combination thereof. Cells may be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof.

It would be appreciated by one of ordinary skill in the art that the plurality of nucleic acid molecules can comprise a variety of nucleic acid molecules. In some embodiments, the plurality of nucleic acid molecules can comprise, DNA molecules, RNA molecules, genomic DNA molecules, mRNA molecules, rRNA molecules, siRNA molecules, or a combination thereof, and can be double-stranded or single-stranded. In some embodiments, the plurality of nucleic acid molecules comprise, or comprise about, 100, 1000, 10000, 20000, 30000, 40000, 50000, 100000, 1000000, or a number or a range between any two of these values, species. In some embodiments, the plurality of nucleic acid molecules comprise at least, or comprise at most, 100, 1000, 10000, 20000, 30000, 40000, 50000, 100000, or 1000000, species. In some embodiments, the plurality of nucleic acid molecules can be from a sample, such as a single cell, or a plurality of cells. In some embodiments, the plurality of nucleic acid molecules can be pooled from a plurality of samples, such as a plurality of single cells.

In some embodiments, the methods disclosed herein can comprise associating a barcode (e.g., a stochastic barcode), which can include a barcode sequence (such as a molecular label), a cell label, a sample label, etc., or any combination thereof, to the plurality of nucleic acid target molecules and the plurality of oligonucleotides of the cellular component binding reagents. For example, a plurality of oligonucleotide probes comprising a stochastic barcode can be used to hybridize to the plurality of nucleic acid target molecules and the plurality of oligonucleotides of the compositions.

In some embodiments, the plurality of oligonucleotide probes can be immobilized on solid supports. The solid supports can be free floating, e.g., beads in a solution. The solid supports can be embedded in a semi-solid or solid array. In some embodiments, the plurality of oligonucleotide probes may not be immobilized on solid supports. When the plurality of oligonucleotide probes are in close proximity to the plurality of nucleic acid target molecules and the plurality of oligonucleotides of the cellular component binding reagents, the plurality of nucleic acid target molecules and the plurality of oligonucleotides of the cellular component binding reagents can hybridize to the oligonucleotide probes. The oligonucleotide probes can be contacted at a non-depletable ratio such that each distinct nucleic acid target molecules and oligonucleotides of the cellular component binding reagents can associate with oligonucleotide probes having different barcode sequences (e.g., molecular labels) of the disclosure.

In some embodiments, the methods disclosed herein provide detaching the oligonucleotides from the cellular component binding reagents that are specifically bound to the cellular component targets. Detachment can be performed in a variety of ways to separate the chemical group from the cellular component binding reagent, such as UV photocleaving, chemical treatment (e.g., dithiothreitol treatment), heating, enzyme treatment, or any combination thereof. Detaching the oligonucleotide from the cellular component binding reagent can be performed either before, after, or during the step of hybridizing the plurality of oligonucleotide probes to the plurality of nucleic acid target molecules and the plurality of oligonucleotides of the compositions.

### Simultaneous Quantitative Analysis of Protein and Nucleic Acid Targets

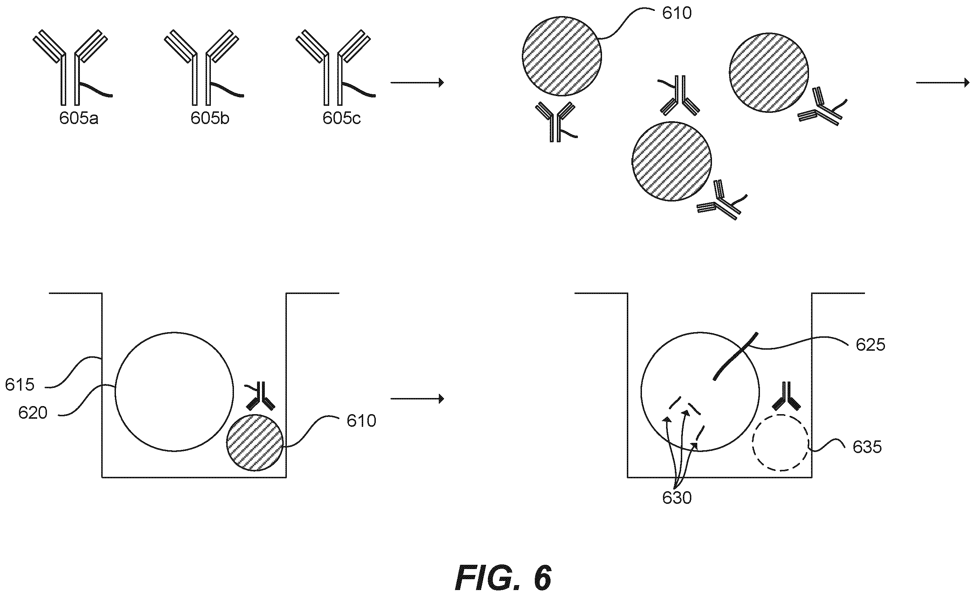

In some embodiments, the methods disclosed herein also can be used for simultaneous quantitative analysis of multiple types of target molecules, for example protein and nucleic acid targets. For example, the target molecules can be, or comprise, cellular components. FIG. 6 shows a schematic illustration of an exemplary method of simultaneous quantitative analysis of both nucleic acid targets and other cellular component targets (e.g., proteins) in single cells. In some embodiments, a plurality of compositions 605, 605b, 605c, etc., each comprising a cellular component binding reagent, such as an antibody, is provided. Different cellular component binding reagents, such as antibodies, which bind to different cellular component targets are conjugated with different unique identifiers. Next, the cellular component binding reagents can be incubates with a sample containing a plurality of cells 610. The different cellular component binding reagents can specifically bind to cellular components on the cell surface, such as a cell marker, a B-cell receptor, a T-cell receptor, an antibody, a major histocompatibility complex, a tumor antigen, a receptor, or any combination thereof. Unbound cellular component binding reagents can be removed, e.g., by washing the cells with a buffer. The cells with the cellular component binding reagents can be then separated into a plurality of compartments, such as a microwell array, wherein a single compartment 615 is sized to fit a single cell and a single bead 620. Each bead can comprise a plurality of oligonucleotide probes, which can comprise a cell label that is common to all oligonucleotide probes on a bead, and barcode sequences (e.g., molecular label sequences). In some embodiments, each oligonucleotide probe can comprise a target binding region, for example, a poly(dT) sequence. The oligonucleotides 625 conjugated to the cellular component binding reagent can be detached from the cellular component binding reagent using chemical, optical or other means. The cell can be lysed 635 to release nucleic acids within the cell, such as genomic DNA or cellular mRNA 630. Cellular mRNA 630, oligonucleotides 625 or both can be captured by the oligonucleotide probes on bead 620, for example, by hybridizing to the poly(dT) sequence. A reverse transcriptase can be used to extend the oligonucleotide probes hybridized to the cellular mRNA 630 and the oligonucleotides 625 using the cellular mRNA 630 and the oligonucleotides 625 as templates. The extension products produced by the reverse transcriptase can be subject to amplification and sequencing. Sequencing reads can be subject to demultiplexing of sequences or identifies of cell labels, barcodes (e.g., molecular labels), genes, cellular component binding reagent specific oligonucleotides (e.g., antibody specific oligonucleotides), etc., which can give rise to a digital representation of cellular components and gene expression of each single cell in the sample.

### Association of Barcodes

The oligonucleotides associated with the cellular component binding reagents (e.g., antigen binding reagents or protein binding reagents) and/or the nucleic acid molecules may randomly associate with the oligonucleotide probes (e.g., barcodes, such as stochastic barcodes). The oligonucleotides associated with the cellular component binding reagents, referred to herein as binding reagent oligonucleotides, can be, or comprise oligonucleotides of the disclosure, such as an antibody oligonucleotide, a sample indexing oligonucleotide, a cell identification oligonucleotide, a control particle oligonucleotide, a control oligonucleotide, an interaction determination oligonucleotide, etc. Association can, for example, comprise hybridization of an oligonucleotide probe's target binding region to a complementary portion of the target nucleic acid molecule and/or the oligonucleotides of the protein binding reagents. For example, a oligo(dT) region of a barcode (e.g., a stochastic barcode) can interact with a poly(A) tail of a target nucleic acid molecule and/or a poly(dA) tail of an oligonucleotide of a protein binding reagent. The assay conditions used for hybridization (e.g., buffer pH, ionic strength, temperature, etc.) can be chosen to promote formation of specific, stable hybrids.

The disclosure provides for methods of associating a molecular label with a target nucleic acid and/or an oligonucleotide associated with a cellular component binding reagent using reverse transcription. As a reverse transcriptase can use both RNA and DNA as template. For example, the oligonucleotide originally conjugated on the cellular component binding reagent can be either RNA or DNA bases, or both. A binding reagent oligonucleotide can be copied and linked (e.g., covalently linked) to a cell label and a barcode sequence (e.g., a molecular label) in addition to the sequence, or a portion thereof, of the binding reagent sequence. As another example, an mRNA molecule can be copied and linked (e.g., covalently linked) to a cell label and a barcode sequence (e.g., a molecular label) in addition to the sequence of the mRNA molecule, or a portion thereof.

In some embodiments, molecular labels can be added by ligation of an oligonucleotide probe target binding region and a portion of the target nucleic acid molecule and/or the oligonucleotides associated with (e.g., currently, or previously, associated with) with cellular component binding reagents. For example, the target binding region may comprise a nucleic acid sequence that can be capable of specific hybridization to a restriction site overhang (e.g., an EcoRI sticky-end overhang). The methods can further comprise treating the target nucleic acids and/or the oligonucleotides associated with cellular component binding reagents with a restriction enzyme (e.g., EcoRI) to create a restriction site overhang. A ligase (e.g., T4 DNA ligase) may be used to join the two fragments.

### Determining the Number or Presence of Unique Molecular Label Sequences

In some embodiments, the methods disclosed herein comprise determining the number or presence of unique molecular label sequences for each unique identifier, each nucleic acid target molecule, and/or each binding reagent oligonucleotides (e.g., antibody oligonucleotides). For example, the sequencing reads can be used to determine the number of unique molecular label sequences for each unique identifier, each nucleic acid target molecule, and/or each binding reagent oligonucleotide. As another example, the sequencing reads can be used to determine the presence or absence of a molecular label sequence (such as a molecular label sequence associated with a target, a binding reagent oligonucleotide, an antibody oligonucleotide, a sample indexing oligonucleotide, a cell identification oligonucleotide, a control particle oligonucleotide, a control oligonucleotide, an interaction determination oligonucleotide, etc. in the sequencing reads).

In some embodiments, the number of unique molecular label sequences for each unique identifier, each nucleic acid target molecule, and/or each binding reagent oligonucleotide indicates the quantity of each cellular component target (e.g., an antigen target or a protein target) and/or each nucleic acid target molecule in the sample. In some embodiments, the quantity of a cellular component target and the quantity of its corresponding nucleic acid target molecules, e.g., mRNA molecules, can be compared to each other. In some embodiments, the ratio of the quantity of a cellular component target and the quantity of its corresponding nucleic acid target molecules, e.g., mRNA molecules, can be calculated. The cellular component targets can be, for example, cell surface protein markers. In some embodiments, the ratio between the protein level of a cell surface protein marker and the level of the mRNA of the cell surface protein marker is low.

The methods disclosed herein can be used for a variety of applications. For example, the methods disclosed herein can be used for proteome and/or transcriptome analysis of a sample. In some embodiments, the methods disclosed herein can be used to identify a cellular component target and/or a nucleic acid target, i.e., a biomarker, in a sample. In some embodiments, the cellular component target and the nucleic acid target correspond to each other, i.e., the nucleic acid target encodes the cellular component target. In some embodiments, the methods disclosed herein can be used to identify cellular component targets that have a desired ratio between the quantity of the cellular component target and the quantity of its corresponding nucleic acid target molecule in a sample, e.g., mRNA molecule. In some embodiments, the ratio is, or is about, 0.001, 0.01, 0.1, 1, 10, 100, 1000, or a number or a range between any two of the above values. In some embodiments, the ratio is at least, or is at most, 0.001, 0.01, 0.1, 1, 10, 100, or 1000. In some embodiments, the methods disclosed herein can be used to identify cellular component targets in a sample that the quantity of its corresponding nucleic acid target molecule in the sample is, or is about, 1000, 100, 10, 5, 2 1, 0, or a number or a range between any two of these values. In some embodiments, the methods disclosed herein can be used to identify cellular component targets in a sample that the quantity of its corresponding nucleic acid target molecule in the sample is more than, or less than, 1000, 100, 10, 5, 2 1, or 0.

### Compositions and Kits

Some embodiments disclosed herein provide kits and compositions for simultaneous quantitative analysis of a plurality of cellular components (e.g., proteins) and/or a plurality of nucleic acid target molecules in a sample. The kits and compositions can, in some embodiments, comprise a plurality of cellular component binding reagents (e.g., a plurality of protein binding reagents) each conjugated with an oligonucleotide, wherein the oligonucleotide comprises a unique identifier for the cellular component binding reagent, and a plurality of oligonucleotide probes, wherein each of the plurality of oligonucleotide probes comprises a target binding region, a barcode sequence (e.g., a molecular label sequence), wherein the barcode sequence is from a diverse set of unique barcode sequences. In some embodiments, each of the oligonucleotides can comprise a molecular label, a cell label, a sample label, or any combination thereof. In some embodiments, each of the oligonucleotides can comprise a linker. In some embodiments, each of the oligonucleotides can comprise a binding site for an oligonucleotide probe, such as a poly(A) tail. For example, the poly(A) tail can be, e.g., oligodA₁₈ (unanchored to a solid support) or oligoA₁₈V (anchored to a solid support). The oligonucleotides can comprise DNA residues, RNA residues, or both.

Disclosed herein include a plurality of sample indexing compositions. Each of the plurality of sample indexing compositions can comprise two or more cellular component binding reagents. Each of the two or more cellular component binding reagents can be associated with a sample indexing oligonucleotide. At least one of the two or more cellular component binding reagents can be capable of specifically binding to at least one cellular component target. The sample indexing oligonucleotide can comprise a sample indexing sequence for identifying sample origin of one or more cells of a sample. Sample indexing sequences of at least two sample indexing compositions of the plurality of sample indexing compositions can comprise different sequences.

Disclosed herein include kits comprising sample indexing compositions for cell identification. In some embodiments. Each of two sample indexing compositions comprises a **cellular component binding reagent (e.g., a protein binding reagent)** associated with a sample indexing oligonucleotide, wherein the cellular component binding reagent is capable of specifically binding to at least one of one or more cellu.ar component targets (e.g., one or more protein targets), wherein the sample indexing oligonucleotide comprises a sample indexing sequence, and wherein sample indexing sequences of the two sample indexing compositions comprise different sequences. In some embodiments, the sample indexing oligonucleotide comprises a molecular label sequence, a binding site for a universal primer, or a combination thereof.

Disclosed herein include kits for cell identification. In some embodiments, the kit comprises: two or more sample indexing compositions. Each of the two or more sample indexing compositions can comprise a cellular component binding reagent (e.g., an antigen binding reagent) associated with a sample indexing oligonucleotide, wherein the cellular component binding reagent is capable of specifically binding to at least one of one or more cellular component targets, wherein the sample indexing oligonucleotide comprises a sample indexing sequence, and wherein sample indexing sequences of the two sample indexing compositions comprise different sequences. In some embodiments, the sample indexing oligonucleotide comprises a molecular label sequence, a binding site for a universal primer, or a combination thereof. Disclosed herein include kits for multiplet identification. In some embodiments, the kit comprises two sample indexing compositions. Each of two sample indexing compositions can comprise a cellular component binding reagent (e.g., an antigen binding reagent) associated with a sample indexing oligonucleotide, wherein the antigen binding reagent is capable of specifically binding to at least one of one or more cellular component targets (e.g., antigen targets), wherein the sample indexing oligonucleotide comprises a sample indexing sequence, and wherein sample indexing sequences of the two sample indexing compositions comprise different sequences.

The unique identifiers (or oligonucleotides associated with cellular component binding reagents, such as binding reagent oligonucleotides, antibody oligonucleotides, sample indexing oligonucleotides, cell identification oligonucleotides, control particle oligonucleotides, control oligonucleotides, or interaction determination oligonucleotides) can have any suitable length, for example, from about 25 nucleotides to about 45 nucleotides long. In some embodiments, the unique identifier can have a length that is, is about, is less than, is greater than, 4 nucleotides, 5 nucleotides, 6 nucleotides, 7 nucleotides, 8 nucleotides, 9 nucleotides, 10 nucleotides, 15 nucleotides, 20 nucleotides, 25 nucleotides, 30 nucleotides, 35 nucleotides, 40 nucleotides, 45 nucleotides, 50 nucleotides, 55 nucleotides, 60 nucleotides, 70 nucleotides, 80 nucleotides, 90 nucleotides, 100 nucleotides, 200 nucleotides, or a range that is between any two of the above values.

In some embodiments, the unique identifiers are selected from a diverse set of unique identifiers. The diverse set of unique identifiers can comprise, or comprise about, 20, 30, 40, 50, 60, 70, 80, 90, 100, 200, 300, 400, 500, 600, 700, 800, 900, 1000, 2000, 5000, or a number or a range between any two of these values, different unique identifiers. The diverse set of unique identifiers can comprise at least, or comprise at most, 20, 30, 40, 50, 60, 70, 80, 90, 100, 200, 300, 400, 500, 600, 700, 800, 900, 1000, 2000, or 5000, different unique identifiers. In some embodiments, the set of unique identifiers is designed to have minimal sequence homology to the DNA or RNA sequences of the sample to be analyzed. In some embodiments, the sequences of the set of unique identifiers are different from each other, or the complement thereof, by, or by about, 1 nucleotide, 2 nucleotides, 3 nucleotides, 4 nucleotides, 5 nucleotides, 6 nucleotides, 7 nucleotides, 8 nucleotides, 9 nucleotides, 10 nucleotides, or a number or a range between any two of these values. In some embodiments, the sequences of the set of unique identifiers are different from each other, or the complement thereof, by at least, or by at most, 1 nucleotide, 2 nucleotides, 3 nucleotides, 4 nucleotides, 5 nucleotides, 6 nucleotides, 7 nucleotides, 8 nucleotides, 9 nucleotides, or 10 nucleotides.

In some embodiments, the unique identifiers can comprise a binding site for a primer, such as universal primer. In some embodiments, the unique identifiers can comprise at least two binding sites for a primer, such as a universal primer. In some embodiments, the unique identifiers can comprise at least three binding sites for a primer, such as a universal primer. The primers can be used for amplification of the unique identifiers, for example, by PCR amplification. In some embodiments, the primers can be used for nested PCR reactions.

Any suitable cellular component binding reagents are contemplated in this disclosure, such as any protein binding reagents (e.g., antibodies or fragments thereof, aptamers, small molecules, ligands, peptides, oligonucleotides, etc., or any combination thereof). In some embodiments, the cellular component binding reagents can be polyclonal antibodies, monoclonal antibodies, recombinant antibodies, single-chain antibody (scAb), or fragments thereof, such as Fab, Fv, etc. In some embodiments, the plurality of protein binding reagents can comprise, or comprise about, 20, 30, 40, 50, 60, 70, 80, 90, 100, 200, 300, 400, 500, 600, 700, 800, 900, 1000, 2000, 5000, or a number or a range between any two of these values, different protein binding reagents. In some embodiments, the plurality of protein binding reagents can comprise at least, or comprise at most, 20, 30, 40, 50, 60, 70, 80, 90, 100, 200, 300, 400, 500, 600, 700, 800, 900, 1000, 2000, or 5000, different protein binding reagents.

In some embodiments, the oligonucleotide is conjugated with the cellular component binding reagent through a linker. In some embodiments, the oligonucleotide can be conjugated with the protein binding reagent covalently. In some embodiment, the oligonucleotide can be conjugated with the protein binding reagent non-covalently. In some embodiments, the linker can comprise a chemical group that reversibly or irreversibly attached the oligonucleotide to the protein binding reagents. The chemical group can be conjugated to the linker, for example, through an amine group. In some embodiments, the linker can comprise a chemical group that forms a stable bond with another chemical group conjugated to the protein binding reagent. For example, the chemical group can be a UV photocleavable group, a disulfide bond, a streptavidin, a biotin, an amine, etc. In some embodiments, the chemical group can be conjugated to the protein binding reagent through a primary amine on an amino acid, such as lysine, or the N-terminus. The oligonucleotide can be conjugated to any suitable site of the protein binding reagent, as long as it does not interfere with the specific binding between the protein binding reagent and its protein target. In embodiments where the protein binding reagent is an antibody, the oligonucleotide can be conjugated to the antibody anywhere other than the antigen-binding site, for example, the Fc region, the C_{H}1 domain, the C_{H}2 domain, the C_{H}3 domain, the C_{L} domain, etc. In some embodiments, each protein binding reagent can be conjugated with a single oligonucleotide molecule. In some embodiments, each protein binding reagent can be conjugated with, or with about, 2, 3, 4, 5, 10, 20, 30, 40, 50, 100, 1000, or a number or a range between any two of these values, oligonucleotide molecules, wherein each of the oligonucleotide molecule comprises the same unique identifier. In some embodiments, each protein binding reagent can be conjugated with more than one oligonucleotide molecule, for example, at least, or at most, 2, 3, 4, 5, 10, 20, 30, 40, 50, 100, or 1000, oligonucleotide molecules, wherein each of the oligonucleotide molecule comprises the same unique identifier.

In some embodiments, the plurality of cellular component binding reagents (e.g., protein binding reagents) are capable of specifically binding to a plurality of cellular component targets (e.g., protein targets) in a sample. The sample can be, or comprise, a single cell, a plurality of cells, a tissue sample, a tumor sample, a blood sample, or the like. In some embodiments, the plurality of cellular component targets comprises a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular proteins. In some embodiments, the plurality of cellular component targets can comprise intracellular proteins. In some embodiments, the plurality of cellular component targets can be, or be about, 1%, 2%, 3%, 4%, 5%, 6%, 7%, 8%, 9%, 10%, 20%, 30%, 40%, 50%, 60%, 70%, 80%, 90%, 95%, 98%, 99%, or a number or a range between any two of these values of all cellular component targets (e.g., proteins expressed or could be expressed) in an organism. In some embodiments, the plurality of cellular component targets can be at least, or be at most, 1%, 2%, 3%, 4%, 5%, 6%, 7%, 8%, 9%, 10%, 20%, 30%, 40%, 50%, 60%, 70%, 80%, 90%, 95%, 98%, or 99%, of all cellular component targets (e.g., proteins expressed or could be expressed) in an organism. In some embodiments, the plurality of cellular component targets can comprise, or comprise about, 2, 3, 4, 5, 10, 20, 30, 40, 50, 100, 1000, 10000, or a number or a range between any two of these values, different cellular component targets. In some embodiments, the plurality of cellular component targets can comprise at least, or comprise at most, 2, 3, 4, 5, 10, 20, 30, 40, 50, 100, 1000, or 10000, different cellular component targets.

### Sample Indexing Using Oligonucleotide-Conjugated Cellular Component Binding Reagent

Disclosed herein include methods for sample identification. In some embodiments, the method comprises: contacting one or more cells from each of a plurality of samples with a sample indexing composition of a plurality of sample indexing compositions, wherein each of the one or more cells comprises one or more cellular component targets, wherein each of the plurality of sample indexing compositions comprises a cellular component binding reagent associated with a sample indexing oligonucleotide, wherein the cellular component binding reagent is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; removing unbound sample indexing compositions of the plurality of sample indexing compositions; barcoding (e.g., stochastically barcoding) the sample indexing oligonucleotides using a plurality of barcodes (e.g., stochastic barcodes) to create a plurality of barcoded sample indexing oligonucleotides; obtaining sequencing data of the plurality of barcoded sample indexing oligonucleotides; and identifying sample origin of at least one cell of the one or more cells based on the sample indexing sequence of at least one barcoded sample indexing oligonucleotide of the plurality of barcoded sample indexing oligonucleotides.

In some embodiments, barcoding the sample indexing oligonucleotides using the plurality of barcodes comprises: contacting the plurality of barcodes with the sample indexing oligonucleotides to generate barcodes hybridized to the sample indexing oligonucleotides; and extending the barcodes hybridized to the sample indexing oligonucleotides to generate the plurality of barcoded sample indexing oligonucleotides. Extending the barcodes can comprise extending the barcodes using a DNA polymerase to generate the plurality of barcoded sample indexing oligonucleotides. Extending the barcodes can comprise extending the barcodes using a reverse transcriptase to generate the plurality of barcoded sample indexing oligonucleotides.

An oligonucleotide-conjugated with an antibody, an oligonucleotide for conjugation with an antibody, or an oligonucleotide previously conjugated with an antibody is referred to herein as an antibody oligonucleotide ("AbOligo"). Antibody oligonucleotides in the context of sample indexing are referred to herein as sample indexing oligonucleotides. An antibody conjugated with an antibody oligonucleotide is referred to herein as a hot antibody or an oligonucleotide antibody. An antibody not conjugated with an antibody oligonucleotide is referred to herein as a cold antibody or an oligonucleotide free antibody. An oligonucleotide-conjugated with a binding reagent (e.g., a protein binding reagent), an oligonucleotide for conjugation with a binding reagent, or an oligonucleotide previously conjugated with a binding reagent is referred to herein as a reagent oligonucleotide. Reagent oligonucleotides in the context of sample indexing are referred to herein as sample indexing oligonucleotides. A binding reagent conjugated with an antibody oligonucleotide is referred to herein as a hot binding reagent or an oligonucleotide binding reagent. A binding reagent not conjugated with an antibody oligonucleotide is referred to herein as a cold binding reagent or an oligonucleotide free binding reagent.

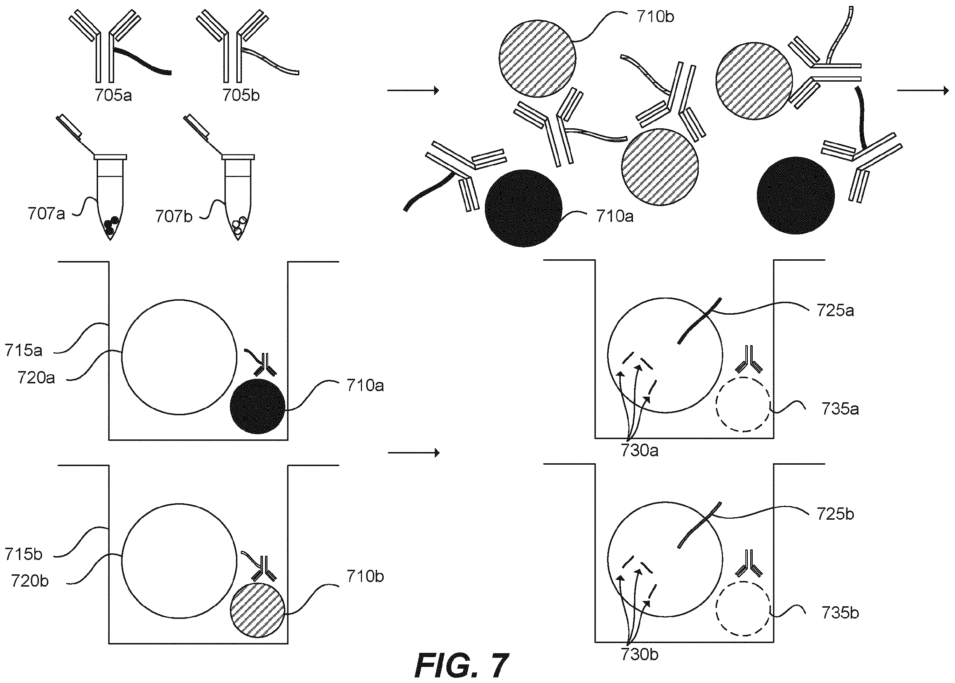

FIG. 7 shows a schematic illustration of an exemplary workflow using oligonucleotide-associated cellular component binding reagents for sample indexing. In some embodiments, a plurality of compositions 705a, 705b, etc., each comprising a binding reagent is provided. The binding reagent can be a protein binding reagent, such as an antibody. The cellular component binding reagent can comprise an antibody, a tetramer, an aptamers, a protein scaffold, or a combination thereof. The binding reagents of the plurality of compositions 705a, 705b can bind to an identical cellular component target. For example, the binding reagents of the plurality of compositions 705, 705b can be identical (except for the sample indexing oligonucleotides associated with the binding reagents).

Different compositions can include binding reagents conjugated with sample indexing oligonucleotides with different sample indexing sequences. The number of different compositions can be different in different implementations. In some embodiments, the number of different compositions can be, or be about, 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, or a number or a range between any two of these values. In some embodiments, the number of different compositions can be at least, or be at most, 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, or 10000.

In some embodiments, the sample indexing oligonucleotides of binding reagents in one composition can include an identical sample indexing sequence. The sample indexing oligonucleotides of binding reagents in one composition may not be identical. In some embodiments, the percentage of sample indexing oligonucleotides of binding reagents in one composition with an identical sample indexing sequence can be, or be about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, or a number or a range between any two of these values. In some embodiments, the percentage of sample indexing oligonucleotides of binding reagents in one composition with an identical sample indexing sequence can be at least, or be at most, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9%.

The compositions 705a and 705b can be used to label samples of different samples. For example, the sample indexing oligonucleotides of the cellular component binding reagent in the composition 705a can have one sample indexing sequence and can be used to label cells 710a, shown as black circles, in a sample 707a, such as a sample of a patient. The sample indexing oligonucleotides of the cellular component binding reagents in the composition 705b can have another sample indexing sequence and can be used to label cells 710b, shown as hatched circles, in a sample 707b, such as a sample of another patient or another sample of the same patient. The cellular component binding reagents can specifically bind to cellular component targets or proteins on the cell surface, such as a cell marker, a B-cell receptor, a T-cell receptor, an antibody, a major histocompatibility complex, a tumor antigen, a receptor, or any combination thereof. Unbound cellular component binding reagents can be removed, e.g., by washing the cells with a buffer.

The cells with the cellular component binding reagents can be then separated into a plurality of compartments, such as a microwell array, wherein a single compartment 715a, 715b is sized to fit a single cell 710a and a single bead 720a or a single cell 710b and a single bead 720b. Each bead 720a, 720b can comprise a plurality of oligonucleotide probes, which can comprise a cell label that is common to all oligonucleotide probes on a bead, and molecular label sequences. In some embodiments, each oligonucleotide probe can comprise a target binding region, for example, a poly(dT) sequence. The sample indexing oligonucleotides 725a conjugated to the cellular component binding reagent of the composition 705a can be configured to be (or can be) detachable or non-detachable from the cellular component binding reagent. The sample indexing oligonucleotides 725a conjugated to the cellular component binding reagent of the composition 705a can be detached from the cellular component binding reagent using chemical, optical or other means. The sample indexing oligonucleotides 725b conjugated to the cellular component binding reagent of the composition 705b can be configured to be (or can be) detachable or non-detachable from the cellular component binding reagent. The sample indexing oligonucleotides 725b conjugated to the cellular component binding reagent of the composition 705b can be detached from the cellular component binding reagent using chemical, optical or other means.

The cell 710a can be lysed to release nucleic acids within the cell 710a, such as genomic DNA or cellular mRNA 730a. The lysed cell 735a is shown as a dotted circle. Cellular mRNA 730a, sample indexing oligonucleotides 725a, or both can be captured by the oligonucleotide probes on bead 720a, for example, by hybridizing to the poly(dT) sequence. A reverse transcriptase can be used to extend the oligonucleotide probes hybridized to the cellular mRNA 730a and the oligonucleotides 725a using the cellular mRNA 730a and the oligonucleotides 725a as templates. The extension products produced by the reverse transcriptase can be subject to amplification and sequencing.

Similarly, the cell 710b can be lysed to release nucleic acids within the cell 710b, such as genomic DNA or cellular mRNA 730b. The lysed cell 735b is shown as a dotted circle. Cellular mRNA 730b, sample indexing oligonucleotides 725b, or both can be captured by the oligonucleotide probes on bead 720b, for example, by hybridizing to the poly(dT) sequence. A reverse transcriptase can be used to extend the oligonucleotide probes hybridized to the cellular mRNA 730b and the oligonucleotides 725b using the cellular mRNA 730b and the oligonucleotides 725b as templates. The extension products produced by the reverse transcriptase can be subject to amplification and sequencing.

Sequencing reads can be subject to demultiplexing of cell labels, molecular labels, gene identities, and sample identities (e.g., in terms of sample indexing sequences of sample indexing oligonucleotides 725a and 725b). Demultiplexing of cell labels, molecular labels, and gene identities can give rise to a digital representation of gene expression of each single cell in the sample. Demultiplexing of cell labels, molecular labels, and sample identities, using sample indexing sequences of sample indexing oligonucleotides, can be used to determine a sample origin.

In some embodiments, cellular component binding reagents against cellular component binding reagents on the cell surface can be conjugated to a library of unique sample indexing oligonucleotides to allow cells to retain sample identity. For example, antibodies against cell surface markers can be conjugated to a library of unique sample indexing oligonucleotides to allow cells to retain sample identity. This will enable multiple samples to be loaded onto the same Rhapsody^{™} cartridge as information pertaining sample source is retained throughout library preparation and sequencing. Sample indexing can allow multiple samples to be run together in a single experiment, simplifying and shortening experiment time, and eliminating batch effect.

Disclosed herein include methods for sample identification. In some embodiments, the method comprise: contacting one or more cells from each of a plurality of samples with a sample indexing composition of a plurality of sample indexing compositions, wherein each of the one or more cells comprises one or more cellular component targets, wherein each of the plurality of sample indexing compositions comprises a cellular component binding reagent associated with a sample indexing oligonucleotide, wherein the cellular component binding reagent is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; removing unbound sample indexing compositions of the plurality of sample indexing compositions. The method can include barcoding (e.g., stochastically barcoding) the sample indexing oligonucleotides using a plurality of barcodes (e.g., stochastic barcodes) to create a plurality of barcoded sample indexing oligonucleotides; obtaining sequencing data of the plurality of barcoded sample indexing oligonucleotides; and identifying sample origin of at least one cell of the one or more cells based on the sample indexing sequence of at least one barcoded sample indexing oligonucleotide of the plurality of barcoded sample indexing oligonucleotides.

In some embodiments, the method for sample identification comprises: contacting one or more cells from each of a plurality of samples with a sample indexing composition of a plurality of sample indexing compositions, wherein each of the one or more cells comprises one or more cellular component targets, wherein each of the plurality of sample indexing compositions comprises a cellular component binding reagent associated with a sample indexing oligonucleotide, wherein the cellular component binding reagent is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; removing unbound sample indexing compositions of the plurality of sample indexing compositions; and identifying sample origin of at least one cell of the one or more cells based on the sample indexing sequence of at least one sample indexing oligonucleotide of the plurality of sample indexing compositions.

In some embodiments, identifying the sample origin of the at least one cell comprises: barcoding (e.g., stochastically barcoding) sample indexing oligonucleotides of the plurality of sample indexing compositions using a plurality of barcodes (e.g., stochastic barcodes) to create a plurality of barcoded sample indexing oligonucleotides; obtaining sequencing data of the plurality of barcoded sample indexing oligonucleotides; and identifying the sample origin of the cell based on the sample indexing sequence of at least one barcoded sample indexing oligonucleotide of the plurality of barcoded sample indexing oligonucleotides. In some embodiments, barcoding the sample indexing oligonucleotides using the plurality of barcodes to create the plurality of barcoded sample indexing oligonucleotides comprises stochastically barcoding the sample indexing oligonucleotides using a plurality of stochastic barcodes to create a plurality of stochastically barcoded sample indexing oligonucleotides.

In some embodiments, identifying the sample origin of the at least one cell can comprise identifying the presence or absence of the sample indexing sequence of at least one sample indexing oligonucleotide of the plurality of sample indexing compositions. Identifying the presence or absence of the sample indexing sequence can comprise: replicating the at least one sample indexing oligonucleotide to generate a plurality of replicated sample indexing oligonucleotides; obtaining sequencing data of the plurality of replicated sample indexing oligonucleotides; and identifying the sample origin of the cell based on the sample indexing sequence of a replicated sample indexing oligonucleotide of the plurality of sample indexing oligonucleotides that correspond to the least one barcoded sample indexing oligonucleotide in the sequencing data.

In some embodiments, replicating the at least one sample indexing oligonucleotide to generate the plurality of replicated sample indexing oligonucleotides comprises: prior to replicating the at least one barcoded sample indexing oligonucleotide, ligating a replicating adaptor to the at least one barcoded sample indexing oligonucleotide. Replicating the at least one barcoded sample indexing oligonucleotide can comprise replicating the at least one barcoded sample indexing oligonucleotide using the replicating adaptor ligated to the at least one barcoded sample indexing oligonucleotide to generate the plurality of replicated sample indexing oligonucleotides.

In some embodiments, replicating the at least one sample indexing oligonucleotide to generate the plurality of replicated sample indexing oligonucleotides comprises: prior to replicating the at least one barcoded sample indexing oligonucleotide, contacting a capture probe with the at least one sample indexing oligonucleotide to generate a capture probe hybridized to the sample indexing oligonucleotide; and extending the capture probe hybridized to the sample indexing oligonucleotide to generate a sample indexing oligonucleotide associated with the capture probe. Replicating the at least one sample indexing oligonucleotide can comprise replicating the sample indexing oligonucleotide associated with the capture probe to generate the plurality of replicated sample indexing oligonucleotides.

### Cell Overloading and Multiplet Identification

Also disclosed herein include methods, kits and systems for identifying cell overloading and multiplet. Such methods, kits and systems can be used in, or in combination with, any suitable methods, kits and systems disclosed herein, for example the methods, kits and systems for measuring cellular component expression level (such as protein expression level) using cellular component binding reagents associated with oligonucleotides.

Using current cell-loading technology, when about 20000 cells are loaded into a microwell cartridge or array with ~60000 microwells, the number of microwells or droplets with two or more cells (referred to as doublets or multiplets) can be minimal. However, when the number of cells loaded increases, the number of microwells or droplets with multiple cells can increase significantly. For example, when about 50000 cells are loaded into about 60000 microwells of a microwell cartridge or array, the percentage of microwells with multiple cells can be quite high, such as 11-14%. Such loading of high number of cells into microwells can be referred to as cell overloading. However, if the cells are divided into a number of groups (e.g., 5), and cells in each group are labeled with sample indexing oligonucleotides with distinct sample indexing sequences, a cell label (e.g., a cell label of a barcode, such as a stochastic barcode) associated with two or more sample indexing sequences can be identified in sequencing data and removed from subsequent processing. In some embodiments, the cells are divided into a large number of groups (e.g., 10000), and cells in each group are labeled with sample indexing oligonucleotides with distinct sample indexing sequences, a sample label associated with two or more sample indexing sequences can be identified in sequencing data and removed from subsequent processing. In some embodiments, different cells are labeled with cell identification oligonucleotides with distinct cell identification sequences, a cell identification sequence associated with two or more cell identification oligonucleotides can be identified in sequencing data and removed from subsequent processing. Such higher number of cells can be loaded into microwells relative to the number of microwells in a microwell cartridge or array.

Disclosed herein include methods for sample identification. In some embodiments, the method comprises: contacting a first plurality of cells and a second plurality of cells with two sample indexing compositions respectively, wherein each of the first plurality of cells and each of the second plurality of cells comprise one or more cellular components, wherein each of the two sample indexing compositions comprises a cellular component binding reagent associated with a sample indexing oligonucleotide, wherein the cellular component binding reagent is capable of specifically binding to at least one of the one or more cellular components, wherein the sample indexing oligonucleotide comprises a sample indexing sequence, and wherein sample indexing sequences of the two sample indexing compositions comprise different sequences; barcoding the sample indexing oligonucleotides using a plurality of barcodes to create a plurality of barcoded sample indexing oligonucleotides, wherein each of the plurality of barcodes comprises a cell label sequence, a barcode sequence (e.g., a molecular label sequence), and a target-binding region, wherein the barcode sequences of at least two barcodes of the plurality of barcodes comprise different sequences, and wherein at least two barcodes of the plurality of barcodes comprise an identical cell label sequence; obtaining sequencing data of the plurality of barcoded sample indexing oligonucleotides; and identifying one or more cell label sequences that is each associated with two or more sample indexing sequences in the sequencing data obtained; and removing the sequencing data associated with the one or more cell label sequences that is each associated with two or more sample indexing sequences from the sequencing data obtained and/or excluding the sequencing data associated with the one or more cell label sequences that is each associated with two or more sample indexing sequences from subsequent analysis (e.g., single cell mRNA profiling, or whole transcriptome analysis). In some embodiments, the sample indexing oligonucleotide comprises a barcode sequence (e.g., a molecular label sequence), a binding site for a universal primer, or a combination thereof.

For example, the method can be used to load 50000 or more cells (compared to 10000-20000 cells) using sample indexing. Sample indexing can use oligonucleotide-conjugated cellular component binding reagents (e.g., antibodies) or cellular component binding reagents against a cellular component (e.g., a universal protein marker) to label cells from different samples with a unique sample index. When two or more cells from different samples, two or more cells from different populations of cells of a sample, or two or more cells of a sample, are captured in the same microwell or droplet, the combined "cell" (or contents of the two or more cells) can be associated with sample indexing oligonucleotides with different sample indexing sequences (or cell identification oligonucleotides with different cell identification sequences). The number of different populations of cells can be different in different implementations. In some embodiments, the number of different populations can be, or be about, 2, 3, 4, 5, 6, 7, 8, 9, 10, 20, 30, 40, 50, 60, 70, 80, 90, 100, or a number or a range between any two of these values. In some embodiments, the number of different populations can be at least, or be at most, 2, 3, 4, 5, 6, 7, 8, 9, 10, 20, 30, 40, 50, 60, 70, 80, 90, or 100. The number, or the average number, of cells in each population can be different in different implementations. In some embodiments, the number, or the average number, of cells in each population can be, or be about, 1, 2, 3, 4, 5, 6, 7, 8, 9, 10, 20, 30, 40, 50, 60, 70, 80, 90, 100, or a number or a range between any two of these values. In some embodiments, the number, or the average number, of cells in each population can be at least, or be at most, 1, 2, 3, 4, 5, 6, 7, 8, 9, 10, 20, 30, 40, 50, 60, 70, 80, 90, or 100. When the number, or the average number, of cells in each population is sufficiently small (e.g., equal to, or fewer than, 50, 25, 10, 5, 4, 3, 2, or 1 cells per population), the sample indexing composition for cell overloading and multiplet identification can be referred to as cell identification compositions.

Cells of a sample can be divided into multiple populations by aliquoting the cells of the sample into the multiple populations. A "cell" associated with more than one sample indexing sequence in the sequencing data can be identified as a "multiplet" based on two or more sample indexing sequences associated with one cell label sequence (e.g., a cell label sequence of a barcode, such as a stochastic barcode) in the sequencing data. The sequencing data of a combined "cell" is also referred to herein as a multiplet. A multiplet can be a doublet, a triplet, a quartet, a quintet, a sextet, a septet, an octet, a nonet, or any combination thereof. A multiplet can be any n-plet. In some embodiments, n is, or is about, 2, 3, 4, 5, 6, 7, 8, 9, 10, 11, 12, 13, 14, 15, 16, 17, 18, 19, 20, or a range between any two of these values. In some embodiments, n is at least, or is at most, 2, 3, 4, 5, 6, 7, 8, 9, 10, 11, 12, 13, 14, 15, 16, 17, 18, 19, or 20.

When determining expression profiles of single cells, two cells may be identified as one cell and the expression profiles of the two cells may be identified as the expression profile for one cell (referred to as a doublet expression profile). For example, when determining expression profiles of two cells using barcoding (e.g., stochastic barcoding), the mRNA molecules of the two cells may be associated with barcodes having the same cell label. As another example, two cells may be associated with one particle (e.g., a bead). The particle can include barcodes with the same cell label. After lysing the cells, the mRNA molecules in the two cells can be associated with the barcodes of the particle, thus the same cell label. Doublet expression profiles can skew the interpretation of the expression profiles.

A doublet can refer to a combined "cell" associated with two sample indexing oligonucleotides with different sample indexing sequences. A doublet can also refer to a combined "cell" associated with sample indexing oligonucleotides with two sample indexing sequences. A doublet can occur when two cells associated with two sample indexing oligonucleotides of different sequences (or two or more cells associated with sample indexing oligonucleotides with two different sample indexing sequences) are captured in the same microwell or droplet, the combined "cell" can be associated with two sample indexing oligonucleotides with different sample indexing sequences. A triplet can refer to a combined "cell" associated with three sample indexing oligonucleotides all with different sample indexing sequences, or a combined "cell" associated with sample indexing oligonucleotides with three different sample indexing sequences. A quartet can refer to a combined "cell" associated with four sample indexing oligonucleotides all with different sample indexing sequences, or a combined "cell" associated with sample indexing oligonucleotides with four different sample indexing sequences. A quintet can refer to a combined "cell" associated with five sample indexing oligonucleotides all with different sample indexing sequences, or a combined "cell" associated with sample indexing oligonucleotides with five different sample indexing sequences. A sextet can refer to a combined "cell" associated with six sample indexing oligonucleotides all with different sample indexing sequences, or a combined "cell" associated with sample indexing oligonucleotides with six different sample indexing sequences. A septet can refer to a combined "cell" associated with seven sample indexing oligonucleotides all with different sample indexing sequences, or a combined "cell" associated with sample indexing oligonucleotides with seven different sample indexing sequences. A octet can refer to a combined "cell" associated with eight sample indexing oligonucleotides all with different sample indexing sequences, or a combined "cell" associated with sample indexing oligonucleotides with eight different sample indexing sequences. A nonet can refer to a combined "cell" associated with nine sample indexing oligonucleotides all with different sample indexing sequences, or a combined "cell" associated with sample indexing oligonucleotides with nine different sample indexing sequences. A multiplet can occur when two or more cells associated with two or more sample indexing oligonucleotides of different sequences (or two or more cells associated with sample indexing oligonucleotides with two or more different sample indexing sequences) are captured in the same microwell or droplet, the combined "cell" can be associated with sample indexing oligonucleotides with two or more different sample indexing sequences.

As another example, the method can be used for multiplet identification, whether in the context of sample overloading or in the context of loading cells onto microwells of a microwell array or generating droplets containing cells. When two or more cells are loaded into one microwell, the resulting data from the combined "cell" (or contents of the two or more cells) is a multiplet with aberrant gene expression profile. By using sample indexing, one can recognize some of these multiplets by looking for cell labels that are each associated with or assigned to two or more sample indexing oligonucleotides with different sample indexing sequences (or sample indexing oligonucleotides with two or more sample indexing sequences). With sample indexing sequence, the methods disclosed herein can be used for multiplet identification (whether in the context of sample overloading or not, or in the context of loading cells onto microwells of a microwell array or generating droplets containing cells). In some embodiments, the method comprises: contacting a first plurality of cells and a second plurality of cells with two sample indexing compositions respectively, wherein each of the first plurality of cells and each of the second plurality of cells comprise one or more cellular components, wherein each of the two sample indexing compositions comprises a cellular component binding reagent associated with a sample indexing oligonucleotide, wherein the cellular component binding reagent is capable of specifically binding to at least one of the one or more cellular components, wherein the sample indexing oligonucleotide comprises a sample indexing sequence, and wherein sample indexing sequences of the two sample indexing compositions comprise different sequences; barcoding the sample indexing oligonucleotides using a plurality of barcodes to create a plurality of barcoded sample indexing oligonucleotides, wherein each of the plurality of barcodes comprises a cell label sequence, a barcode sequence (e.g., a molecular label sequence), and a target-binding region, wherein barcode sequences of at least two barcodes of the plurality of barcodes comprise different sequences, and wherein at least two barcodes of the plurality of barcodes comprise an identical cell label sequence; obtaining sequencing data of the plurality of barcoded sample indexing oligonucleotides; and identifying one or more multiplet cell label sequences that is each associated with two or more sample indexing sequences in the sequencing data obtained.

The number of cells that can be loaded onto microwells of a microwell cartridge or into droplets generated using a microfluidics device can be limited by the multiplet rate. Loading more cells can result in more multiplets, which can be hard to identify and create noise in the single cell data. With sample indexing, the method can be used to more accurately label or identify multiplets and remove the multiplets from the sequencing data or subsequent analysis. Being able to identify multiplets with higher confidence can increase user tolerance for the multiplet rate and load more cells onto each microwell cartridge or generating droplets with at least one cell each.

In some embodiments, contacting the first plurality of cells and the second plurality of cells with the two sample indexing compositions respectively comprises: contacting the first plurality of cells with a first sample indexing compositions of the two sample indexing compositions; and contacting the first plurality of cells with a second sample indexing compositions of the two sample indexing compositions. The number of pluralities of cells and the number of pluralities of sample indexing compositions can be different in different implementations. In some embodiments, the number of pluralities of cells and/or sample indexing compositions can be, or be about, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, 1000000, or a number or a range between any two of these values. In some embodiments, the number of pluralities of cells and/or sample indexing compositions can be at least, or be at most, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, or 1000000. The number of cells can be different in different implementations. In some embodiments, the number, or the average number, of cells can be, or be about, 1, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, 1000000, or a number or a range between any two of these values. In some embodiments, the number, or the average number, or cells can be at least, or be at most, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, or 1000000.

In some embodiments, the method comprises: removing unbound sample indexing compositions of the two sample indexing compositions. Removing the unbound sample indexing compositions can comprise washing cells of the first plurality of cells and the second plurality of cells with a washing buffer. Removing the unbound sample indexing compositions can comprise selecting cells bound to at least one cellular component binding reagent of the two sample indexing compositions using flow cytometry. In some embodiments, the method comprises: lysing the one or more cells from each of the plurality of samples.

In some embodiments, the sample indexing oligonucleotide is configured to be (or can be) detachable or non-detachable from the cellular component binding reagent. The method can comprise detaching the sample indexing oligonucleotide from the cellular component binding reagent. Detaching the sample indexing oligonucleotide can comprise detaching the sample indexing oligonucleotide from the cellular component binding reagent by UV photocleaving, chemical treatment (e.g., using reducing reagent, such as dithiothreitol), heating, enzyme treatment, or any combination thereof.

In some embodiments, barcoding the sample indexing oligonucleotides using the plurality of barcodes comprises: contacting the plurality of barcodes with the sample indexing oligonucleotides to generate barcodes hybridized to the sample indexing oligonucleotides; and extending the barcodes hybridized to the sample indexing oligonucleotides to generate the plurality of barcoded sample indexing oligonucleotides. Extending the barcodes can comprise extending the barcodes using a DNA polymerase to generate the plurality of barcoded sample indexing oligonucleotides. Extending the barcodes can comprise extending the barcodes using a reverse transcriptase to generate the plurality of barcoded sample indexing oligonucleotides.

In some embodiments, the method comprises: amplifying the plurality of barcoded sample indexing oligonucleotides to produce a plurality of amplicons. Amplifying the plurality of barcoded sample indexing oligonucleotides can comprise amplifying, using polymerase chain reaction (PCR), at least a portion of barcode sequence (e.g., the molecular label sequence) and at least a portion of the sample indexing oligonucleotide. In some embodiments, obtaining the sequencing data of the plurality of barcoded sample indexing oligonucleotides can comprise obtaining sequencing data of the plurality of amplicons. Obtaining the sequencing data comprises sequencing at least a portion of the barcode sequence and at least a portion of the sample indexing oligonucleotide. In some embodiments, identifying the sample origin of the at least one cell comprises identifying sample origin of the plurality of barcoded targets based on the sample indexing sequence of the at least one barcoded sample indexing oligonucleotide.

In some embodiments, barcoding the sample indexing oligonucleotides using the plurality of barcodes to create the plurality of barcoded sample indexing oligonucleotides comprises stochastically barcoding the sample indexing oligonucleotides using a plurality of stochastic barcodes to create a plurality of stochastically barcoded sample indexing oligonucleotides.

In some embodiments, the method includes: barcoding a plurality of targets of the cell using the plurality of barcodes to create a plurality of barcoded targets, wherein each of the plurality of barcodes comprises a cell label sequence, and wherein at least two barcodes of the plurality of barcodes comprise an identical cell label sequence; and obtaining sequencing data of the barcoded targets. Barcoding the plurality of targets using the plurality of barcodes to create the plurality of barcoded targets can include: contacting copies of the targets with target-binding regions of the barcodes; and reverse transcribing the plurality targets using the plurality of barcodes to create a plurality of reverse transcribed targets.

In some embodiments, the method comprises: prior to obtaining the sequencing data of the plurality of barcoded targets, amplifying the barcoded targets to create a plurality of amplified barcoded targets. Amplifying the barcoded targets to generate the plurality of amplified barcoded targets can comprise: amplifying the barcoded targets by polymerase chain reaction (PCR). Barcoding the plurality of targets of the cell using the plurality of barcodes to create the plurality of barcoded targets can comprise stochastically barcoding the plurality of targets of the cell using a plurality of stochastic barcodes to create a plurality of stochastically barcoded targets.

In some embodiments, the method for cell identification comprise: contacting a first plurality of one or more cells and a second plurality of one or more cells with two cell identification compositions respectively, wherein each of the first plurality of one or more cells and each of the second plurality of one or more cells comprise one or more cellular components, wherein each of the two cell identification compositions comprises a cellular component binding reagent associated with a cell identification oligonucleotide, wherein the cellular component binding reagent is capable of specifically binding to at least one of the one or more cellular components, wherein the cell identification oligonucleotide comprises a cell identification sequence, and wherein cell identification sequences of the two cell identification compositions comprise different sequences; barcoding the cell identification oligonucleotides using a plurality of barcodes to create a plurality of barcoded cell identification oligonucleotides, wherein each of the plurality of barcodes comprises a cell label sequence, a barcode sequence (e.g., a molecular label sequence), and a target-binding region, wherein the barcode sequences of at least two barcodes of the plurality of barcodes comprise different sequences, and wherein at least two barcodes of the plurality of barcodes comprise an identical cell label sequence; obtaining sequencing data of the plurality of barcoded cell identification oligonucleotides; and identifying one or more cell label sequences that is each associated with two or more cell identification sequences in the sequencing data obtained; and removing the sequencing data associated with the one or more cell label sequences that is each associated with two or more cell identification sequences from the sequencing data obtained and/or excluding the sequencing data associated with the one or more cell label sequences that is each associated with two or more cell identification sequences from subsequent analysis (e.g., single cell mRNA profiling, or whole transcriptome analysis). In some embodiments, the cell identification oligonucleotide comprises a barcode sequence (e.g., a molecular label sequence), a binding site for a universal primer, or a combination thereof.

A multiplet (e.g., a doublet, triplet, etc.) can occur when two or more cells associated with two or more cell identification oligonucleotides of different sequences (or two or more cells associated with cell identification oligonucleotides with two or more different cell identification sequences) are captured in the same microwell or droplet, the combined "cell" can be associated with cell identification oligonucleotides with two or more different cell identification sequences.

Cell identification compositions can be used for multiplet identification, whether in the context of cell overloading or in the context of loading cells onto microwells of a microwell array or generating droplets containing cells. When two or more cells are loaded into one microwell, the resulting data from the combined "cell" (or contents of the two or more cells) is a multiplet with aberrant gene expression profile. By using cell identification, one can recognize some of these multiplets by looking for cell labels (e.g., cell labels of barcodes, such as stochastic barcodes) that are each associated with or assigned to two or more cell identification oligonucleotides with different cell identification sequences (or cell identification oligonucleotides with two or more cell identification sequences). With cell identification sequence, the methods disclosed herein can be used for multiplet identification (whether in the context of sample overloading or not, or in the context of loading cells onto microwells of a microwell array or generating droplets containing cells). In some embodiments, the method comprises: contacting a first plurality of one or more cells and a second plurality of one or more cells with two cell identification compositions respectively, wherein each of the first plurality of one or more cells and each of the second plurality of one or more cells comprise one or more cellular components, wherein each of the two cell identification compositions comprises a cellular component binding reagent associated with a cell identification oligonucleotide, wherein the cellular component binding reagent is capable of specifically binding to at least one of the one or more cellular components, wherein the cell identification oligonucleotide comprises a cell identification sequence, and wherein cell identification sequences of the two cell identification compositions comprise different sequences; barcoding the cell identification oligonucleotides using a plurality of barcodes to create a plurality of barcoded cell identification oligonucleotides, wherein each of the plurality of barcodes comprises a cell label sequence, a barcode sequence (e.g., a molecular label sequence), and a target-binding region, wherein barcode sequences of at least two barcodes of the plurality of barcodes comprise different sequences, and wherein at least two barcodes of the plurality of barcodes comprise an identical cell label sequence; obtaining sequencing data of the plurality of barcoded cell identification oligonucleotides; and identifying one or more multiplet cell label sequences that is each associated with two or more cell identification sequences in the sequencing data obtained.

The number of cells that can be loaded onto microwells of a microwell cartridge or into droplets generated using a microfluidics device can be limited by the multiplet rate. Loading more cells can result in more multiplets, which can be hard to identify and create noise in the single cell data. With cell identification, the method can be used to more accurately label or identify multiplets and remove the multiplets from the sequencing data or subsequent analysis. Being able to identify multiplets with higher confidence can increase user tolerance for the multiplet rate and load more cells onto each microwell cartridge or generating droplets with at least one cell each.

In some embodiments, contacting the first plurality of one or more cells and the second plurality of one or more cells with the two cell identification compositions respectively comprises: contacting the first plurality of one or more cells with a first cell identification compositions of the two cell identification compositions; and contacting the first plurality of one or more cells with a second cell identification compositions of the two cell identification compositions. The number of pluralities of cell identification compositions can be different in different implementations. In some embodiments, the number of cell identification compositions can be, or be about, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, 1000000, or a number or a range between any two of these values. In some embodiments, the number of cell identification compositions can be at least, or be at most, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, or 1000000. The number, or average number, of cells in each plurality of one or more cells can be different in different implementations. In some embodiments, the number, or average number, of cells in each plurality of one or more cells can be, or be about, 1, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, 1000000, or a number or a range between any two of these values. In some embodiments, the number, or average number, of cells in each plurality of one or more cells can be at least, or be at most, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, or 1000000.

In some embodiments, the method comprises: removing unbound cell identification compositions of the two cell identification compositions. Removing the unbound cell identification compositions can comprise washing cells of the first plurality of one or more cells and the second plurality of one or more cells with a washing buffer. Removing the unbound cell identification compositions can comprise selecting cells bound to at least one cellular component binding reagent of the two cell identification compositions using flow cytometry. In some embodiments, the method comprises: lysing the one or more cells from each of the plurality of samples.

In some embodiments, the cell identification oligonucleotide is configured to be (or can be) detachable or non-detachable from the cellular component binding reagent. The method can comprise detaching the cell identification oligonucleotide from the cellular component binding reagent. Detaching the cell identification oligonucleotide can comprise detaching the cell identification oligonucleotide from the cellular component binding reagent by UV photocleaving, chemical treatment (e.g., using reducing reagent, such as dithiothreitol), heating, enzyme treatment, or any combination thereof.

In some embodiments, barcoding the cell identification oligonucleotides using the plurality of barcodes comprises: contacting the plurality of barcodes with the cell identification oligonucleotides to generate barcodes hybridized to the cell identification oligonucleotides; and extending the barcodes hybridized to the cell identification oligonucleotides to generate the plurality of barcoded cell identification oligonucleotides. Extending the barcodes can comprise extending the barcodes using a DNA polymerase to generate the plurality of barcoded cell identification oligonucleotides. Extending the barcodes can comprise extending the barcodes using a reverse transcriptase to generate the plurality of barcoded cell identification oligonucleotides.

In some embodiments, the method comprises: amplifying the plurality of barcoded cell identification oligonucleotides to produce a plurality of amplicons. Amplifying the plurality of barcoded cell identification oligonucleotides can comprise amplifying, using polymerase chain reaction (PCR), at least a portion of barcode sequence (e.g., the molecular label sequence) and at least a portion of the cell identification oligonucleotide. In some embodiments, obtaining the sequencing data of the plurality of barcoded cell identification oligonucleotides can comprise obtaining sequencing data of the plurality of amplicons. Obtaining the sequencing data comprises sequencing at least a portion of the barcode sequence and at least a portion of the cell identification oligonucleotide. In some embodiments, identifying the sample origin of the at least one cell comprises identifying sample origin of the plurality of barcoded targets based on the cell identification sequence of the at least one barcoded cell identification oligonucleotide.

In some embodiments, barcoding the cell identification oligonucleotides using the plurality of barcodes to create the plurality of barcoded cell identification oligonucleotides comprises stochastically barcoding the cell identification oligonucleotides using a plurality of stochastic barcodes to create a plurality of stochastically barcoded cell identification oligonucleotides.

### Aptamer Cellular Component Profiling Method

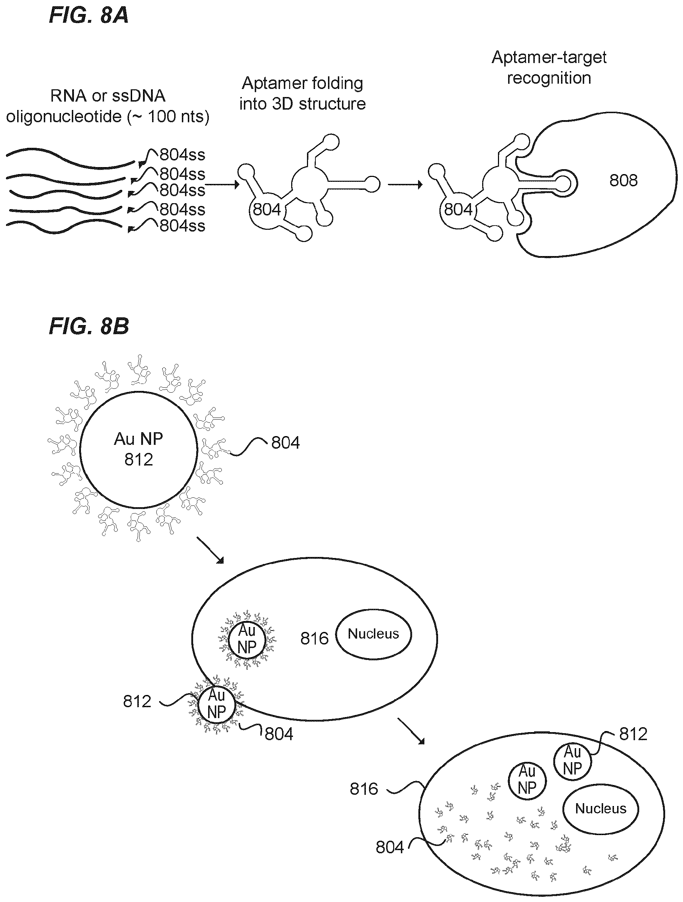

Disclosed herein include embodiments of a method for profiling cellular component expression (such as intracellular cellular component expression, including intracellular protein expression) using aptamers. FIGS. 8A-8E show a schematic illustration of an exemplary workflow of using oligonucleotide-associated (e.g., conjugated) aptamers to determine cellular component expression (e.g., protein expression). In some embodiments, the method can be used with any single-cell RNA sequencing method (including any single-cell 3' RNA sequencing method, such as BD Rhapsody^{™}, and single-cell 5' RNA sequencing method). In some embodiments, the method can be used with a single-cell RNA sequencing method without needing to fix and permeabilize the cells (such as partially or completely fixing and/or permeabilizing cells).

In some embodiments, an aptamer 804 can be a single stranded polynucleotide 804ss (e.g., ribonucleic acid (RNA) or deoxyribonucleic acid (DNA)). The single stranded polynucleotide can be for example, 100 nucleotides in length. The length of the single stranded polynucleotide can be different in different implementations. In some embodiments, the single stranded polynucleotide can be, or can be about, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the single stranded nucleotide can be at least, or can be at most, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000. An aptamer 804 can fold into a three dimensional structure 804 that is able to recognize a target 808, such as a cellular component target 808 (e.g., a protein target).

In some embodiments, aptamers 804 specific to the target intracellular components (e.g., intracellular proteins) can be associated with (e.g., bound to or conjugated to) gold nanoparticles 812 (See FIG. 8B). Such association can allow the uptake of aptamers 804 into cells 816. After uptake of aptamers 804 into cells 816, aptamers 804 can dissociate from gold nanoparticles 816 and bind to the targets of the aptamers 804.

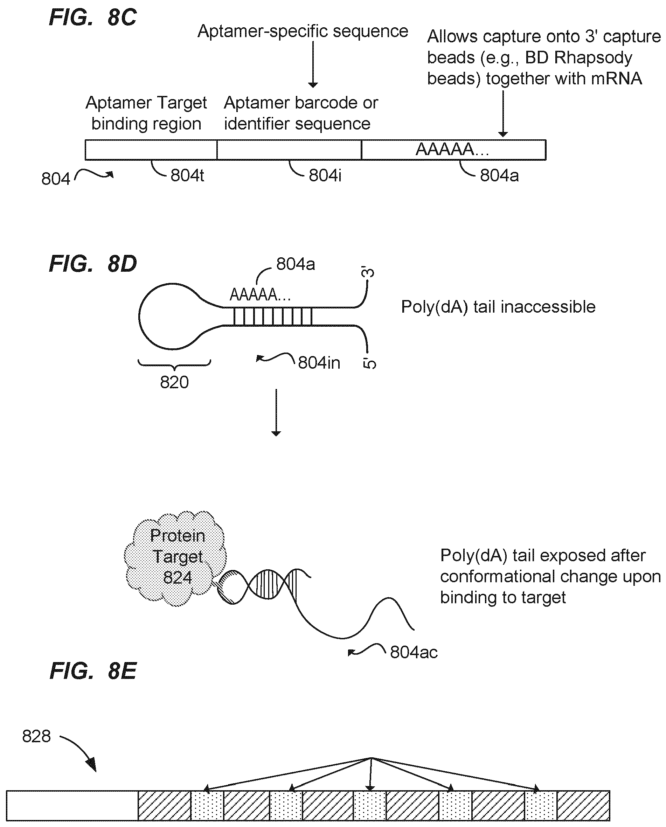

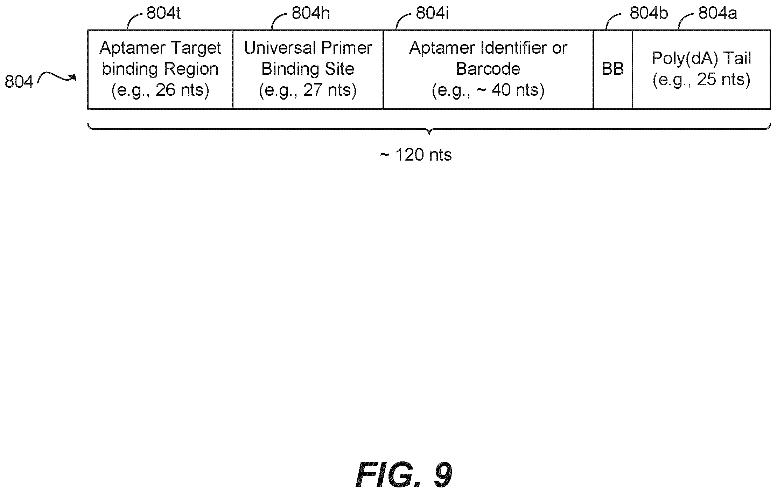

In some embodiments, the aptamer 804 is a single polynucleotide that contains an aptamer target binding region 804t (that is capable of binding to a cellular component target of the aptamer), a universal primer binding site 804h (e.g., a PCR handle, a complete or partial Illumina R2 and/or P7 sequence), an identifier sequence 804i (also referred to herein as an aptamer barcode), and a poly(dA) tail 804a (See FIGS. 8C and 9) to enable the capturing and amplification of the aptamer using a single-cell 3' RNA sequencing platform (e.g., BD Rhapsody^{™}). In some embodiments, there is a plurality of nucleotides 804b between the aptamer barcode and the poly(dA) for aligning the poly(dA) tail to the oligo(dT) sequence of a barcode (referred to herein as an alignment sequence). Each of the plurality of nucleotides can be a non-adenosine (e.g., a guanine, a cytosine, a thymine, and a uracil). In some embodiments, one polynucleotide includes the aptamer target binding region, and a second polynucleotide includes the universal primer binding site, the identifier sequence, the alignment sequence, and the poly(dA) tail.

In some embodiments, excess aptamers not bound to target cellular components (e.g., protein molecules) can be removed. For example, as illustrated in FIG. 8D, the aptamers 804 can be designed so each can form a hairpin structure 820, such that the poly(dA) tail 804a can only be accessible upon conformational changes induced by binding to the target cellular component (a protein target 824 illustrated here). Any unbound aptamers 820 cannot hybridize to oligo(dT) sequences on barcodes (e,g., oligo(dT) sequences on barcodes, such as stochastic barcodes, associated to a 3' RNA sequencing capture bead). As another example, anti-sense aptamers can be introduced (e.g., into cells) following sense aptamer delivery to soak up unbound aptamers and create double stranded nucleotides (e.g., double stranded DNA, double stranded RNA, DNA/RNA hybrid) that cannot be captured onto the a RNA sequencing capture bead.

As another example, as illustrated in FIG. 8E, an aptamer "sponge" 828 can be introduced into a cell using, for example, a vector with tandem sequences 828 complementary (e.g., partially or completely) to the sequences of aptamers 804 or subsequences thereof. The sequence identity between a tandem sequence 828 complementary to an aptamer sequence (or a subsequence thereof) and the aptamer sequence (or a subsequence thereof) can be different in different implementations. Unbound aptamers can bind to the tandem sequences 828 complementary to the aptamer sequences, aggregate, and be targeted for degradation in the cell. Aptamers thus can absorb aptamers not bound to cellular component targets. In some embodiments, the sequence identity between the complementary sequence 828 to the aptamer sequence (or a subsequence thereof) and the aptamer sequence (or a subsequence thereof) can be, or can be about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values. In some embodiments, the sequence identity between the complementary sequence 828 to the aptamer sequence (or a subsequence thereof) and the aptamer sequence (or a subsequence thereof) can be at least, or can be at most, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

Disclosed herein are embodiments of a method for measuring protein expression in cells. In some embodiments, the method comprises: contacting a plurality of protein-binding aptamers (e.g., the aptamer target binding region 804t described with reference to FIGS. 8C and 9) with a plurality of cells comprising a plurality of protein targets (such as the cell 816 described with reference to FIG. 8B), wherein each of the plurality of protein-binding aptamers comprises an aptamer specific oligonucleotide comprising a unique identifier sequence (e.g, the aptamer identifier or barcode 804i described with reference to FIGS. 8C and 9) for the cellular component-binding aptamer, and wherein the protein-binding aptamer is capable of specifically binding to at least one of the plurality of protein targets. In some embodiments, the method comprises partitioning the plurality of cells (such as the cell 816 described with reference to FIG. 8B) associated with the plurality of protein-binding aptamers to a plurality of partitions, wherein a partition of the plurality of partitions comprises a single cell from the plurality of cells associated with the protein-binding aptamers; in the partition comprising the single cell, contacting a barcoding particle with the aptamer specific oligonucleotides, wherein the barcoding particle comprises a plurality of oligonucleotide probes (e.g., the barcode 104 described with reference to FIG. 1) each comprising a target binding region and a barcode sequence selected from a diverse set of unique barcode sequences; extending oligonucleotide probes hybridized to the aptamer specific oligonucleotides to produce a plurality of labeled nucleic acids, wherein each of the labeled nucleic acid comprises a unique identifier sequence, or a complementary sequence thereof, and a barcode sequence; and obtaining sequence information of the plurality of labeled nucleic acids or a portion thereof to determine the quantity of one or more of the plurality of protein targets in one or more of the plurality of cells.

Disclosed herein include embodiments of a method of measuring cellular component expression in cells. In some embodiments, the method comprises: contacting a plurality of cellular component-binding aptamers (e.g., aptamers 804 described with reference to FIGS. 8A-8E and 9) with a plurality of cells (such as the cell 816 described with reference to FIG. 8B) comprising a plurality of cellular component targets (such as the cell 816 described with reference to FIG. 8B), wherein each of the plurality of cellular component-binding aptamers comprises an aptamer specific oligonucleotide comprising a unique identifier sequence (e.g., the aptamer identifier or barcode 804i described with reference to FIGS. 8C and 9) for the cellular component-binding aptamer, and wherein the cellular component-binding aptamer is capable of specifically binding to at least one of the plurality of cellular component targets; extending oligonucleotide probes (e.g., the barcode 104 described with reference to FIG. 1) hybridized to the aptamer specific oligonucleotides to produce a plurality of labeled nucleic acids, wherein each of the labeled nucleic acid comprises a unique identifier sequence, or a complementary sequence thereof, and a barcode sequence; and obtaining sequence information of the plurality of labeled nucleic acids or a portion thereof to determine the quantity of one or more of the plurality of cellular component targets in one or more of the plurality of cells.

The aptamer specific oligonucleotide can refer to a polynucleotide that does not include the aptamer target binding region 804t, such as a polynucleotide that includes a PCR handle 804h (e.g., a complete or partial Illumina R2 and/or P7 sequence), an identifier sequence 804i (also referred to herein as an aptamer barcode), an alignment sequence 804a, and/or a poly(dA) tail 804a. In some embodiments, the aptamer target binding region 804t and the aptamer specific oligonucleotide can form one single polynucleotide as illustrated with reference to FIG. 9.

The length of an aptamer (or an aptamer target binding region) can be different in different implementations. In some embodiments, the length of an aptamer can be, or can be about, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the length of an aptamer can be at least, or can be at most, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

The length of an aptamer specific oligonucleotide can be different in different implementations. In some embodiments, the length of an aptamer specific oligonucleotide can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the length of an aptamer specific oligonucleotide can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

The length of an identifier sequence of an aptamer can be different in different implementations. In some embodiments, the length of an identifier sequence of an aptamer can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the length of an identifier sequence of an aptamer can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

The method can comprise: prior to extending the oligonucleotide probes: partitioning the plurality of cells associated with the plurality of cellular component-binding aptamers to a plurality of partitions, wherein a partition of the plurality of partitions comprises a single cell from the plurality of cells associated with the cellular component-binding aptamers; in the partition comprising the single cell, contacting a barcoding particle (e.g., the bead 105 described with reference to FIG. 1) with the aptamer specific oligonucleotides, wherein the barcoding particle comprises a plurality of oligonucleotide probes each comprising a target binding region and a barcode sequence selected from a can diverse set of unique barcode sequences. The plurality of cellular component targets can comprise a plurality of protein targets, and wherein the cellular component-binding aptamer is capable of specifically binding to at least one of the plurality of protein targets.

In some embodiments, partitioning the plurality of cells comprises partitioning the plurality of cells associated with the plurality of aptamers and a plurality of barcoding particles comprising the barcoding particle to the plurality of partitions (See, for example, blocks 208 and 212 described with reference to FIG. 2), wherein the partition of the plurality of partitions comprises the single cell from the plurality of cells associated with the oligonucleotide-conjugated aptamer and the barcoding particle. In some embodiments, the partition is a well or a droplet.

In some embodiments, obtaining sequencing information of the plurality of labeled nucleic acids or a portion thereof comprises subjecting the labeled nucleic acids to one or more reactions to generate a set of nucleic acids for nucleic acid sequencing (See, for example, FIG. 3 and the accompanying description).

In some embodiments, each of the oligonucleotide probes comprises a cell label, a binding site for a universal primer, an amplification adaptor, a sequencing adaptor, or a combination thereof (e.g., the barcode 104 described with reference to FIG. 1). In some embodiments, the barcoding particle is a Sepharose bead, a streptavidin bead, an agarose bead, a magnetic bead, a silica bead, a silica-like bead, a hydrogel bead, an anti-biotin microbead, an anti-fluorochrome microbead, or a hydrogel bead.

### Aptamer Specific Oligonucleotides

The association between the aptamer specific oligonucleotide and the aptamer can be different in different implementations. The aptamer specific oligonucleotide and the aptamer can, for example, be present in (e.g., form) a single polynucleotide (e.g., the single polynucleotide 804 shown in FIGS. 8A and 9). The aptamer can be 5' to the aptamer specific oligonucleotide in the single polynucleotide. The aptamer can be 3' to the aptamer specific oligonucleotide in the single polynucleotide. The aptamer specific oligonucleotide can be associated with the aptamer. The aptamer specific oligonucleotide can be attached to the aptamer. The aptamer specific oligonucleotide can be covalently attached to the aptamer. The aptamer specific oligonucleotide can be non-covalently attached to the aptamer. The aptamer specific oligonucleotide can be conjugated to the aptamer. The aptamer specific oligonucleotide can be conjugated to the aptamer through a chemical group selected from the group consisting of a UV photocleavable group, a streptavidin, a biotin, an amine, and a combination thereof. The aptamer specific oligonucleotide can be covalently attached to the aptamer. In some embodiments, the method comprises detaching the aptamer specific oligonucleotides from the aptamer.

In some embodiments, the aptamer specific oligonucleotide comprises a sequence complementary to a capture sequence configured to capture (or capable of capturing, hybridizing to, or binding to) the sequence of the aptamer specific oligonucleotide. The barcode can comprise a target-binding region which comprises the capture sequence. The target-binding region can comprise a poly(dT) region. The sequence of the aptamer specific oligonucleotide complementary to the capture sequence can comprise a poly(dA) region.

In some embodiments, the aptamer specific oligonucleotide comprises a molecular label sequence, a binding site for a universal primer (e.g., the universal primer binding site 804h described with reference to FIG. 9), or both. The molecular label sequence can be 2-20 nucleotides in length. The universal primer can be 5-50 nucleotides in length. The universal primer can comprise an amplification primer (e.g., an Illumina P7 sequence or a subsequence thereof), a sequencing primer (e.g., an Illumina R2 sequence or a subsequence thereof), or a combination thereof. The length of a molecular label or a binding site for a universal primer of an aptamer specific oligonucleotide can be different in different implementations. In some embodiments, the length of a molecular label or a binding site for a universal primer of an aptamer specific oligonucleotide can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values. In some embodiments, the length of a molecular label or a binding site for a universal primer of an aptamer specific oligonucleotide can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100.

### Alignment Sequence

In some embodiments, the aptamer specific oligonucleotide comprises an alignment sequence (e.g., the alignment sequence 804b described with reference to FIG. 9( adjacent to the poly(dA) region. The alignment sequence can be 1 or more nucleotides in length. The alignment sequence can be 2 nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof.

The length of the alignment sequence can be different in different implementations. In some embodiments, the length of the alignment sequence can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values. In some embodiments, the length of the alignment sequence can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100. The number of guanine(s), cytosine(s), thymine(s), or uracil(s) in the alignment sequence can be different in different implementations. The number of guanine(s), cytosine(s), thymine(s), or uracil(s) can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values. The number of guanine(s), cytosine(s), thymine(s), or uracil(s) can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100.

### Aptamer

In some embodiments, the aptamer is or comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The aptamer can comprise a peptide aptamer.

### Carrier

In some embodiments, the plurality of protein-binding aptamers comprises a second protein-binding aptamer, or the plurality of cellular component-binding aptamers comprises a second cellular component-binding aptamer. The aptamer and the second aptamer can be associated with a first carrier. The aptamer can be associated with a first carrier, and the second aptamer can be associated with a second carrier. The aptamer and the second aptamer can be at least 60%, 70%, 80%, 90%, or 95% identical. The sequence identity between the aptamer and the second aptamer can be different in different implementations. In some embodiments, the aptamer and the second aptamer can have a sequence identity of, or of about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 30%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values. In some embodiments, The aptamer and the second aptamer can have a sequence identity of at least, or of at most, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 30%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. The aptamer and the second protein-binding aptamer can be identical. The aptamer and the second aptamer can be different.

The number of different carriers can be different in different implementations. In some embodiments, there is one type of carrier. In some embodiments, the number of different types of carriers can be, or can be about, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the number of different types of carriers can be at least, or can be at most, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000. The number of carrier molecules of each type of carrier can be different in different implementations. In some embodiments, the number of carrier molecules of each type of carrier can be, or can be about, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 2000, 3000, 4000, 5000, 6000, 7000, 8000, 9000, 10000, 100000, 1000000, 10000000, 100000000, or 1000000000, or a number or a range between any two of these values. In some embodiments, the number of carrier molecules of each type of carrier can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 2000, 3000, 4000, 5000, 6000, 7000, 8000, 9000, 10000, 100000, 1000000, 10000000, 100000000, or 1000000000.

In some embodiments, the protein targets, or the cellular component targets, of the aptamer and the second aptamer are identical. The aptamer and the second aptamer can be capable of binding to different regions of a protein target, or a cellular component target. The protein targets, or the cellular component targets, of the aptamer and the second aptamer can be different.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the aptamer is attached to the first carrier. The aptamer can be covalently attached to the first carrier. The aptamer can be conjugated to the first carrier. The aptamer can be conjugated to the first carrier through a chemical group selected from the group consisting of a UV photocleavable group, a streptavidin, a biotin, an amine, and a combination thereof. The aptamer can be non-covalently linked to the first carrier. The aptamer can be associated with the first carrier through a linker. The aptamer can be immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the method comprises dissociating the aptamer from the first carrier. The dissociating can occur after the contacting in the partition comprising the single cells. The dissociating can occur before the contacting in the partition comprising the single cells.

In some embodiments, contacting the plurality of aptamers with the plurality of cells comprises: contacting the first carrier comprising the aptamer with a cell of the plurality of cells comprising the plurality of protein targets. The first carrier can be internalized into the cell. The first carrier can be internalized into the cell via endocytosis, pinocytosis, nanopinocytosis, micropinocytosis, phagocytosis, membrane fusion, or a combination thereof. The first carrier can be internalized into the cell via clathrin-medicated internalization, caveolin-mediated internalization, receptor dependent internalization, receptor independent internalization or a combination thereof.

### Removal of Aptamers

In some embodiments, the method comprises after contacting the plurality of aptamers with the plurality of cells, removing the aptamers that are not contacted with the plurality of cells. Removing the aptamers not contacted with the plurality of cells can comprise: removing the aptamers not contacted with the respective at least one of the plurality of protein targets.

### Removal of Aptamers - Aptamers Sponge

Removing the aptamers not contacted with the respective at least one of the plurality of protein targets can comprise: removing the aptamers not contacted with the respective at least one of the plurality of protein targets comprises using an aptamer sponge (e.g., the aptamer sponge 828 described with reference to FIG. 8E). The aptamer sponge can, for example, comprise a plurality of sequences complementary to the sequences of the aptamers. The aptamers not contacted with the respective at least one of the plurality of protein targets can hybridize to the plurality of sequences of the aptamer sponge.

### Removal of Aptamers - Aptamer Specific Oligonucleotide Conformation

In some embodiments, the aptamer specific oligonucleotide changes from a first conformation in which the poly(dA) region is inaccessible to a second conformation in which the poly(dA) region is accessible when the aptamer contacts the at least one of the plurality of protein targets, or cellular component targets. For example, as illustrated in FIG. 8D, an aptamer 804in associated with an aptamer specific oligonucleotide with an inaccessible poly(dA) region can change into an aptamer 804ac associated with an aptamer specific oligonucleotide with an accessible poly(dA) region when the aptamer 804in binds to its cellular component target. The poly(dA) region of the aptamer specific oligonucleotide in the first conformation can comprise a hairpin structure (e.g., the hairpin structure 820 described with reference to FIG. 8D). The poly(dA) region with the hairpin structure can be accessible to the poly(dT) region of the target-binding region of the barcode. The oligonucleotide probes can be hybridized to the aptamer specific oligonucleotides via the hybridization between the poly(dA) regions of the aptamer specific oligonucleotides and the poly(dT) regions of the oligonucleotide probes. An aptamer specific oligonucleotide associated with an aptamer not in contact with the cellular compartment target can have an inaccessible poly(dA) region that cannot hybridize to the poly(dT) region of an oligonucleotide probe. Such an aptamer specific oligonucleotide and its associated aptamer can be removed.

In some embodiments, the aptamers comprise complementary sequences. The aptamers not contacted with the respective at least one of the plurality of protein targets can hybridize to one another to form an aggregate. The aggregate can be targeted for degradation in the cell.

### Removal of Aptamers - Antisense

In some embodiments, removing the aptamers not contacted with the respective at least one of the plurality of protein targets comprises: removing the aptamers not contacted with the respective at least one of the plurality of protein targets comprises using a plurality of antisense aptamers, wherein each anti-sense aptamer of the plurality of antisense aptamer comprises a second aptamer comprising an anti-sense aptamer specific oligonucleotide comprising the complementary sequence, or a portion thereof, of the aptamer specific oligonucleotide of one of the aptamers not contacted with the respective at least one of the plurality of protein targets, and wherein the aptamer specific oligonucleotide and the anti-sense aptamer specific oligonucleotide form a double stranded, or a partially double stranded, deoxyribonucleic acid (DNA) molecule.

### Targets and Samples

In some embodiments, the plurality of protein targets comprises a cell-surface protein, an intracellular protein, a cell marker, a B-cell receptor, a T-cell receptor, an antibody, a major histocompatibility complex, a tumor antigen, a receptor, or a combination thereof. In some embodiments, the plurality of cells comprises T cells, B cells, tumor cells, myeloid cells, blood cells, normal cells, fetal cells, maternal cells, or a mixture thereof.

### Aptamer Cellular Component Profiling Compositions

Disclosed herein include composition comprising: a plurality of cellular component-binding aptamers, wherein each of the plurality of cellular component-binding aptamers comprises an aptamer specific oligonucleotide comprising a unique identifier sequence for the cellular component-binding aptamer, and wherein the cellular component-binding aptamer is capable of specifically binding to at least one of a plurality of cellular component targets.

### Aptamer Specific Oligonucleotide

In some embodiments, the aptamer specific oligonucleotide and the aptamer form a single polynucleotide. The cellular component-binding aptamer can be 5' to the aptamer specific oligonucleotide in the single polynucleotide. The cellular component-binding aptamer can be 3' to the aptamer specific oligonucleotide in the single polynucleotide

In some embodiments, the aptamer specific oligonucleotide is associated with the cellular component-binding aptamer. The aptamer specific oligonucleotide can be attached to the cellular component-binding aptamer. The aptamer specific oligonucleotide can be covalently attached to the cellular component-binding aptamer. The aptamer specific oligonucleotide can be non-covalently attached to the cellular component-binding aptamer. The aptamer specific oligonucleotide can be conjugated to the cellular component-binding aptamer. The aptamer specific oligonucleotide can be conjugated to the cellular component-binding aptamer through a chemical group selected from the group consisting of a UV photocleavable group, a streptavidin, a biotin, an amine, and a combination thereof. The aptamer specific oligonucleotide can be covalently attached to the cellular component-binding aptamer.

In some embodiments, the aptamer specific oligonucleotide can comprise a molecular label sequence, a binding site for a universal primer, or both. The molecular label sequence can be 2-20 nucleotides in length. The universal primer can be 5-50 nucleotides in length. The universal primer can comprise an amplification primer, a sequencing primer, or a combination thereof.

### Alignment Sequence

In some embodiments, the aptamer specific oligonucleotide comprises an alignment sequence adjacent to the poly(dA) region. The alignment sequence can be 1 or more nucleotides in length. The alignment sequence can be 2 nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof.

### Aptamer

In some embodiments, the cellular component-binding aptamer comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The cellular component-binding aptamer can comprise a peptide aptamer.

### Carrier

In some embodiments, the plurality of cellular component-binding aptamers comprises a second cellular component-binding aptamer. The cellular component-binding aptamer and the second cellular component-binding aptamer can be associated with a first carrier. The cellular component-binding aptamer can be associated with a first carrier, and the second cellular component-binding aptamer can be associated with a second carrier.

In some embodiments, the cellular component-binding aptamer and the second cellular component-binding aptamer are at least 60%, 70%, 80%, 90%, or 95% identical. The cellular component-binding aptamer and the second protein-binding aptamer can be identical. The cellular component-binding aptamer and the second cellular component-binding aptamer can be different.

In some embodiments, the protein targets, or the cellular component targets, of the cellular component-binding aptamer and the second cellular component-binding aptamer are identical. The cellular component-binding aptamer and the second cellular component-binding aptamer can be capable of binding to different regions of a protein target, or a cellular component target. The protein targets, or the cellular component targets, of the cellular component-binding aptamer and the second cellular component-binding aptamer can be different.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the cellular component-binding aptamer is attached to the first carrier. The cellular component-binding aptamer can be covalently attached to the first carrier. The cellular component-binding aptamer can be conjugated to the first carrier. The cellular component-binding aptamer can be conjugated to the first carrier through a chemical group selected from the group consisting of a UV photocleavable group, a streptavidin, a biotin, an amine, and a combination thereof. The cellular component-binding aptamer can be non-covalently linked to the first carrier. The cellular component-binding aptamer can be associated with the first carrier through a linker. The cellular component-binding aptamer can be immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the first carrier is configured to be internalized (or capable of being internalized) into a cell. The first carrier can be configured to be internalized (or capable of being internalized) into the cell via endocytosis, pinocytosis, nanopinocytosis, micropinocytosis, phagocytosis, membrane fusion, or a combination thereof. The first carrier can be configured to be internalized (or capable of being internalized) into the cell via clathrin-medicated internalization, caveolin-mediated internalization, receptor dependent internalization, receptor independent internalization or a combination thereof.

In some embodiments, the aptamer specific oligonucleotide comprises a sequence complementary to a capture sequence configured to capture (or capable of capturing, binding to, or hybridizing to) the sequence of the aptamer specific oligonucleotide. The barcode can comprise a target-binding region which comprises the capture sequence. The target-binding region can comprise a poly(dT) region. The sequence of the aptamer specific oligonucleotide complementary to the capture sequence can comprise a poly(dA) region.

### Aptamers Removal - Aptamer Sponge

In some embodiments, the composition comprises an aptamer sponge. The aptamer sponge can comprise a plurality of sequences complementary to the sequences of the aptamers. The aptamer sponge can be used to remove aptamers not bound to the targets.

### Aptamers Removal - Aptamer Specific Oligonucleotide Conformation

In some embodiments, the aptamer specific oligonucleotide changes from a first conformation in which the poly(dA) region is inaccessible to a second conformation in which the poly(dA) region is accessible when the aptamer contacts the at least one of the plurality of protein targets, or cellular component targets. The poly(dA) region of the aptamer specific oligonucleotide in the first conformation can comprise a hairpin structure. The poly(dA) region with the hairpin structure can be accessible to the poly(dT) region of the target-binding region of the barcode. The oligonucleotide probes can be hybridized to the aptamer specific oligonucleotides via the hybridization between the poly(dA) regions of the aptamer specific oligonucleotides and the poly(dT) regions of the oligonucleotide probes.

In some embodiments, the aptamers comprise complementary sequences. The aptamers can be configured to hybridize to (or capable of hybridizing to) one another to form an aggregate. The aggregate can be targeted for degradation in the cell.

### Aptamers Removal - Antisense

In some embodiments, the composition comprises: a plurality of antisense aptamers, wherein each anti-sense aptamer of the plurality of antisense aptamer comprises a second aptamer comprising an anti-sense aptamer specific oligonucleotide comprising the complementary sequence, or a portion thereof, of the aptamer specific oligonucleotide of one of the aptamers, and wherein the aptamer specific oligonucleotide and the anti-sense aptamer specific oligonucleotide form a double stranded, or a partially double stranded, deoxyribonucleic acid (DNA) molecule.

### Aptamer Cellular Component Profiling Kit

Disclosed herein include a kit for profiling cellular components (e.g., for protein expression profiling). The kit can comprising a composition comprising: a plurality of cellular component-binding aptamers, wherein each of the plurality of cellular component-binding aptamers comprises an aptamer specific oligonucleotide comprising a unique identifier sequence for the cellular component-binding aptamer, and wherein the cellular component-binding aptamer is capable of specifically binding to at least one of a plurality of cellular component targets. The kit can include a barcoding particle for barcoding (e.g., stochastic barcoding) aptamer specific oligonucleotide. The kit can include an aptamer sponge. The aptamers can be configured to hybridize to (or capable of hybridizing to) one another to form an aggregate. The composition can include a plurality of antisense aptamers.

### Aptamer Sample Indexing Method

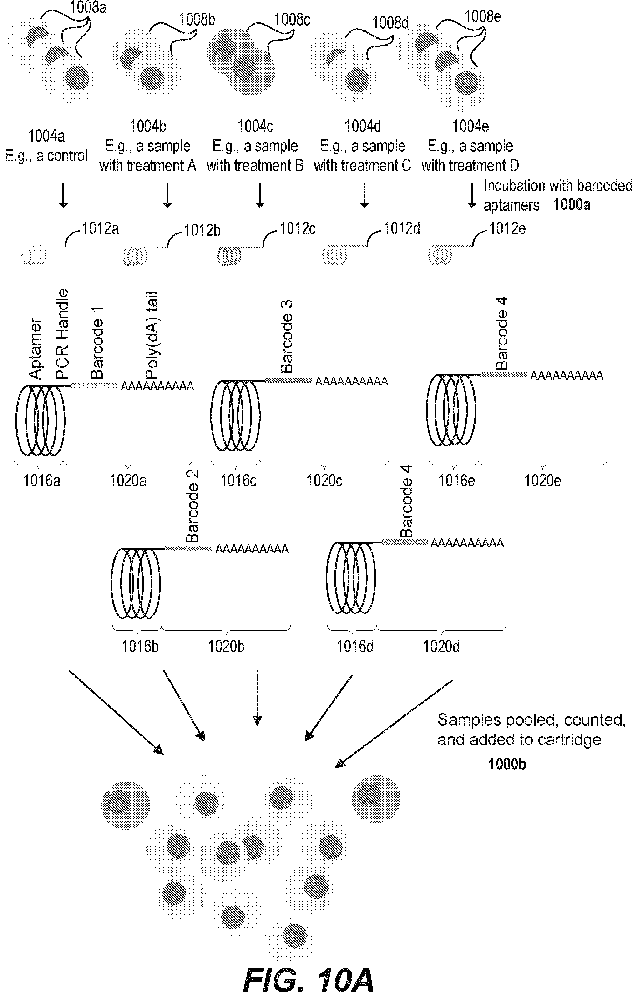

FIGS. 10A-10B show a schematic illustration of an exemplary workflow of using oligonucleotide-associated aptamer for sample indexing or sample identification. The method can include contacting cells of each sample with a sample indexing composition at step 1000a; pooling cells from different samples contacted with the sample indexing compositions at step 1000b; co-partitioning single cells of pooled cells with single beads into partitions at step 1000c; lysing cells and barcoding sample indexing oligonucleotides of the sample indexing compositions in the partitions at step 1000d; and obtaining sequencing data of the barcoded sample indexing oligonucleotides at step 1000e.

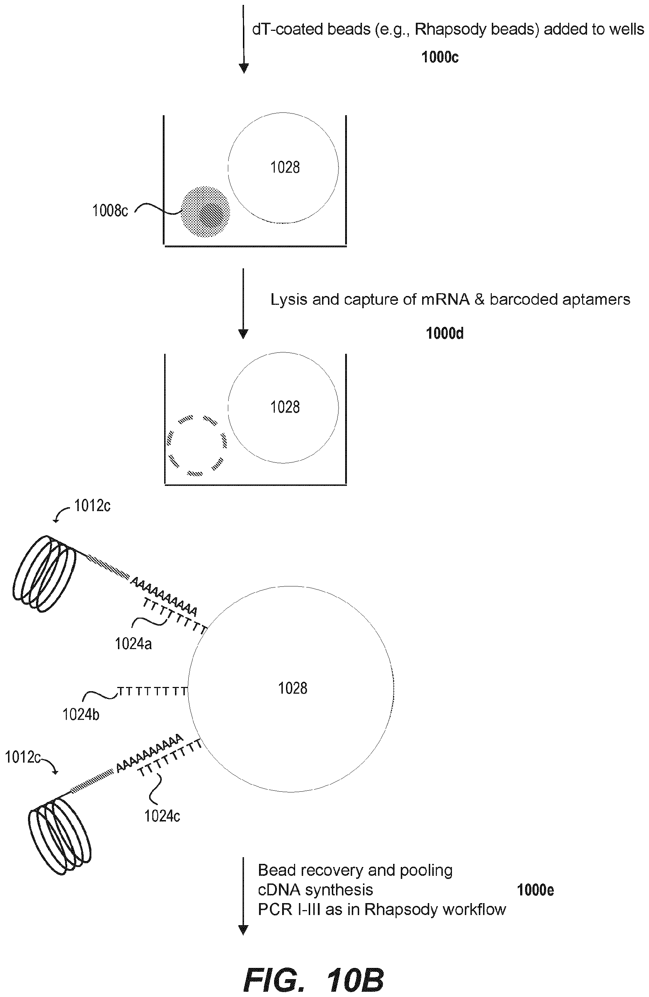

In some embodiments, the method comprises: contacting each of a plurality of samples (e.g., samples 1004a-1004e in FIG. 10A) with a sample indexing composition of a plurality of sample indexing compositions, respectively (e.g., at step 1000a in FIG. 10A), wherein each of the plurality of samples comprises one or more cells (e.g., the cells 1008a-1008e in FIG 10A) each comprising one or more protein targets, wherein the sample indexing composition comprises an aptamer composition (e.g., aptamer compositions 1012a-1012e described with reference to FIGS. 10A-10B) comprising an aptamer (e.g., aptamers 1016a-1016e) and a sample indexing oligonucleotide (e.g., sample indexing oligonucleotides 1020a-1020e), wherein the aptamer is capable of specifically binding to at least one of the one or more protein targets, wherein the sample indexing oligonucleotide comprises a sample indexing sequence, and wherein sample indexing sequences of at least two sample indexing compositions of the plurality of sample indexing compositions comprise different sequences For example, the sample indexing sequences (also referend to herein aptamer identifiers or barcodes) 1020a-1020e have different sequences as illustrated in FIG. 10A. The method can include barcoding (e.g., at step 1000d in FIG. 10) the sample indexing oligonucleotides using a plurality of barcodes (e.g., the barcodes 1024a-1024c associated with a bead 1028 in FIG. 10B) to generate a plurality of barcoded sample indexing oligonucleotides; obtaining sequencing data of the plurality of barcoded sample indexing oligonucleotides; and identifying sample origin of at least one cell of the one or more cells based on the sample indexing sequence of at least one barcoded sample indexing oligonucleotide of the plurality of barcoded sample indexing oligonucleotides.

In some embodiments, the method comprises: contacting each of a plurality of samples with a sample indexing composition of a plurality of sample indexing compositions, respectively, wherein each of the plurality of samples comprises one or more cells each comprising one or more cellular component targets, wherein the sample indexing composition comprises an aptamer composition comprising an aptamer and a sample indexing oligonucleotide, wherein the aptamer is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; and identifying sample origin of at least one cell of the one or more cells based on the sample indexing sequence of at least one sample indexing oligonucleotide of the plurality of sample indexing compositions.

In some embodiments, the cellular component target comprises a protein target. In some embodiments, identifying the sample origin of the at least one cell comprises: barcoding sample indexing oligonucleotides of the plurality of sample indexing compositions using a plurality of barcodes to generate a plurality of barcoded sample indexing oligonucleotides; obtaining sequencing data of the plurality of barcoded sample indexing oligonucleotides; and identifying the sample origin of the cell based on the sample indexing sequence of at least one barcoded sample indexing oligonucleotide of the plurality of barcoded sample indexing oligonucleotides in the sequencing data.

The length of the sample indexing oligonucleotide can be different in different implementations. In some embodiments, the length of the sample indexing oligonucleotide can be, or can be about, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the length of the sample indexing oligonucleotide can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

The length of the sample indexing sequence can be different in different implementations. In some embodiments, the length of the sample indexing sequence can be, or can be about, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the length of the sample indexing sequence can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

The number of sample indexing compositions of the plurality of sample indexing compositions comprising sample indexing sequences with different sequences can be different in different implementations. In some embodiments, the number of sample indexing compositions comprising sample indexing sequences with different sequences can be, or can be about, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the number of sample indexing compositions comprising sample indexing sequences with different sequences can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

### Sample Origin Identification

In some embodiments, identifying the sample origin of the at least one cell comprises identifying the presence or absence of the sample indexing sequence of at least one sample indexing oligonucleotide of the plurality of sample indexing compositions. Identifying the presence or absence of the sample indexing sequence can comprise: replicating the at least one sample indexing oligonucleotide to generate a plurality of replicated sample indexing oligonucleotides; obtaining sequencing data of the plurality of replicated sample indexing oligonucleotides; and identifying the sample origin of the cell based on the sample indexing sequence of a replicated sample indexing oligonucleotide of the plurality of sample indexing oligonucleotides that correspond to the least one barcoded sample indexing oligonucleotide in the sequencing data.

### Sample Indexing Compositions

In some embodiments, the sample indexing composition of the plurality of sample indexing compositions comprises a second aptamer not associated with the sample indexing oligonucleotide. The aptamer and the second aptamer can be identical. The number of aptamers in the sample indexing composition can be different in different implementations. In some embodiments, each of the plurality of sample indexing compositions comprises the aptamer.

In some embodiments, the number of aptamers in the sample indexing composition can be, or can be about, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the number of aptamers in the sample indexing composition can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

### Sample Indexing Oligonucleotides

In some embodiments, a single polynucleotide comprises the sample indexing oligonucleotide and the aptamer. The aptamer can be 5' to the sample indexing oligonucleotide in the single polynucleotide. The aptamer can 3' to the sample indexing oligonucleotide in the single polynucleotide. The sample indexing oligonucleotide can be associated with the aptamer. The sample indexing oligonucleotide can be attached to the aptamer. The sample indexing oligonucleotide can be covalently attached to the aptamer. The sample indexing oligonucleotide can be conjugated to the aptamer. The sample indexing oligonucleotide can be conjugated to the aptamer through a chemical group selected from the group consisting of a UV photocleavable group, a streptavidin, a biotin, an amine, and a combination thereof. In some embodiments, the sample indexing oligonucleotide can be non-covalently attached to the aptamer. The sample indexing oligonucleotide can be associated with the aptamer through a linker.

In some embodiments, the sample indexing oligonucleotide is configured to be (or can be) non-detachable from the aptamer. The sample indexing oligonucleotide can be configured to be (or can be) detachable from the aptamer. The method can comprise detaching the sample indexing oligonucleotide from the aptamer. Detaching the sample indexing oligonucleotide can comprise detaching the sample indexing oligonucleotide from the aptamer by UV photocleaving, chemical treatment, heating, enzyme treatment, or any combination thereof.

In some embodiments, the sample indexing sequence is 6-60 nucleotides in length. The sample indexing oligonucleotide can be 50-500 nucleotides in length. Sample indexing sequences of at least 10, 100, or 1000 sample indexing compositions of the plurality of sample indexing compositions can comprise different sequences.

The sample indexing oligonucleotide can comprise a molecular label sequence, a binding site for a universal primer, or both. The molecular label sequence can be 2-20 nucleotides in length. The universal primer can be 5-50 nucleotides in length. The universal primer can comprise an amplification primer (e.g., an Illumina P7 sequence or a subsequence thereof), a sequencing primer (e.g., an Illumina R2 sequence or a subsequence thereof), or a combination thereof. In some embodiments, the length of a molecular label or a binding site for a universal primer of a sample indexing oligonucleotide can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values. In some embodiments, the length of a molecular label or a binding site for a universal primer of a sample indexing oligonucleotide can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100.

In some embodiments, the sample indexing oligonucleotide is not homologous to genomic sequences of any of the one or more cells, is homologous to genomic sequences of a species, or a combination thereof. The species can be a non-mammalian species.

In some embodiments, the sample indexing oligonucleotide comprises a sequence complementary to a capture sequence configured to capture (or capable of capturing, hybridizing to, or binding to) the sequence of the sample indexing oligonucleotide. The barcode can comprise a target-binding region which comprises the capture sequence. The target-binding region can comprise a poly(dT) region. The sequence of the sample indexing oligonucleotide complementary to the capture sequence can comprise a poly(dA) region.

### Alignment Sequence

In some embodiments, the sample indexing oligonucleotide comprises an alignment sequence adjacent to the poly(dA) region. The alignment sequence can be 1 or more nucleotides in length. The alignment sequence can be 2 or more nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof.

### Aptamer

In some embodiments, the aptamer comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The aptamer can comprise a peptide aptamer.

In some embodiments, the aptamer is associated with two or more sample indexing oligonucleotides with an identical sequence. The aptamer can be associated with two or more sample indexing oligonucleotides with different sample indexing sequences.

### Carrier

In some embodiments, the sample indexing composition can be associated with a first carrier. Different sample indexing compositions of the plurality of sample indexing compositions can be associated with different first carriers. The number of carriers can be different in different implementations. In some embodiments, the number of carrier(s) can be, or can be about, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the number of carrier(s) can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the aptamer can be attached to the first carrier. The aptamer can be covalently attached to the first carrier. The aptamer can be conjugated to the first carrier. The aptamer can be conjugated to the first carrier through a chemical group selected from the group consisting of a UV photocleavable group, a streptavidin, a biotin, an amine, and a combination thereof. The aptamer can be non-covalently linked to the first carrier. The aptamer can be associated with the first carrier through a linker. The aptamer can be immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the method comprises dissociating the aptamer from the first carrier. The dissociating can occur after barcoding the sample indexing oligonucleotides. The dissociating can occur before barcoding the sample indexing oligonucleotides.

In some embodiments, contacting each of the plurality of samples with the sample indexing composition comprises contacting a cell of the one or more cells of the sample with the sample indexing composition. The first carrier can be internalized into the cell. The first carrier can be internalized into the cell via endocytosis, pinocytosis, nanopinocytosis, micropinocytosis, phagocytosis, membrane fusion, or a combination thereof. The first carrier can be internalized into the cell via clathrin-medicated internalization, caveolin-mediated internalization, receptor dependent internalization, receptor independent internalization or a combination thereof.

### Second Protein Binding-Binding Aptamer

In some embodiments, the sample indexing composition of the plurality of sample indexing compositions comprises a second aptamer composition comprising a second protein-binding aptamer, and the second aptamer is capable of specifically binding to at least one of the one or more protein targets, or to at least one of the one or more cellular component targets. The aptamer and the second aptamer can be capable of binding to the same protein target of the one or more protein targets, or the one or more cellular component targets, and wherein the second aptamer is not associated with the sample indexing oligonucleotide. The second aptamer can be associated with a second sample indexing oligonucleotide comprising a second sample indexing sequence. The aptamer and the second aptamer can be at least 60%, 70%, 80%, 90%, or 95% identical. The aptamer and the second aptamer can be identical. The aptamer and the second aptamer can be different.

The number of different aptamers can be different in different implementations. In some embodiments, the number of aptamer(s) can be, or can be about, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the number of different aptamer(s) can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

The sequence identity of the aptamer and the second aptamer can be different in different implementations. In some embodiments, the aptamer and the second aptamer can have a sequence identity of, or of about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 30%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values. In some embodiments, The aptamer and the second aptamer can have a sequence identity of at least, or of at most, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 30%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

The aptamer and the second aptamer can be capable of binding to different regions of the same protein target, or different regions of the same cellular component target. The aptamer and the second aptamer can be capable of binding to different protein targets of the one or more protein targets, or different cellular component targets of the one or more cellular component targets. The sample indexing sequence and the second sample indexing sequence can be identical. The sample indexing sequence and the second sample indexing sequence can be different.

In some embodiments, the aptamer composition can be associated with a first carrier. The aptamer composition can comprise: a second aptamer capable of specifically binding to at least one of the one or more protein targets or one or more cellular component targets, and a second sample indexing oligonucleotide comprising a second sample indexing sequence. The aptamer and the second aptamer can be associated with a first carrier. The aptamer can be associated with a first carrier and the second aptamer is associated with a second carrier. The aptamer and the second aptamer can be at least 60%, 70%, 80%, 90%, or 95% identical in sequence. The aptamer and the second aptamer can be identical, for example, in sequence. The aptamer and the second aptamer can be different. The protein targets, or the cellular component targets, of the aptamer and the second aptamer can be identical, for example, in sequence. The aptamer and the second aptamer can be capable of binding to different regions of a protein target, or a cellular component target. The protein targets, or the cellular component targets, of the aptamer and the second aptamer can be different.

### Targets and Samples

In some embodiments, a sample of the plurality of samples comprises a plurality of cells, a plurality of single cells, a tissue, a tumor sample, or any combination thereof. The plurality of samples can comprise a mammalian cell, a bacterial cell, a viral cell, a yeast cell, a fungal cell, or any combination thereof.

In some embodiments, the at least one of the one or more protein targets, or the one or more cellular component targets, is on a cell surface. In some embodiments, the protein target, or the cellular component target, comprises a carbohydrate, a lipid, a protein, an extracellular protein, a cell-surface protein, a cell marker, a B-cell receptor, a T-cell receptor, a major histocompatibility complex, a tumor antigen, a receptor, an intracellular protein, or any combination thereof. The protein target, or the cellular component target, can be selected from a group comprising 10-100 different protein targets, or cellular component targets.

The number of protein target(s), or the cellular component target(s) can be different in different implementations. In some embodiments, the number of target(s) can be, or can be about, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values. In some embodiments, the number of target(s) can be at least, or can be at most, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, 150, 160, 170, 180, 190, 200, 210, 220, 230, 240, 250, 260, 270, 280, 290, 300, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000.

### Sample Indexing Workflow

In some embodiments, the method comprises removing unbound sample indexing compositions of the plurality of sample indexing compositions. Removing the unbound sample indexing compositions can comprise washing the one or more cells from each of the plurality of samples with a washing buffer. Removing the unbound sample indexing compositions can comprise selecting cells bound to at least one aptamer using flow cytometry. In some embodiments, the method comprises lysing the one or more cells from each of the plurality of samples (e.g., at step 1000d in FIG. 10B).

In some embodiments, replicating the at least one sample indexing oligonucleotide to generate the plurality of replicated sample indexing oligonucleotides comprises: prior to replicating the at least one barcoded sample indexing oligonucleotide, ligating a replicating adaptor to the at least one barcoded sample indexing oligonucleotide, and wherein replicating the at least one barcoded sample indexing oligonucleotide comprises replicating the at least one barcoded sample indexing oligonucleotide using the replicating adaptor ligated to the at least one barcoded sample indexing oligonucleotide to generate the plurality of replicated sample indexing oligonucleotides. In some embodiments, replicating the at least one sample indexing oligonucleotide to generate the plurality of replicated sample indexing oligonucleotides comprises: prior to replicating the at least one barcoded sample indexing oligonucleotide, contacting a capture probe with the at least one sample indexing oligonucleotide to generate a capture probe hybridized to the sample indexing oligonucleotide; and extending the capture probe hybridized to the sample indexing oligonucleotide (e.g., at step 1000e in FIG. 10B) to generate a sample indexing oligonucleotide associated with the capture probe. Replicating the at least one sample indexing oligonucleotide can comprise replicating the sample indexing oligonucleotide associated with the capture probe to generate the plurality of replicated sample indexing oligonucleotides.

### Barcodes and Barcoding

In some embodiments, the method comprises: prior to barcoding the sample indexing oligonucleotides, pooling the plurality of samples contacted with the plurality of sample indexing compositions (e.g., at step 1000b in FIG. 10A).

In sine embodiments, a barcode of the plurality of barcodes comprises a target-binding region and a molecular label sequence, and molecular label sequences of at least two barcodes of the plurality of barcodes comprise different molecule label sequences. The barcode can comprise a cell label sequence, a binding site for a universal primer, or any combination thereof. The target-binding region can comprise a poly(dT) region.

In some embodiments, the plurality of barcodes is associated with a particle. At least one barcode of the plurality of barcodes can be immobilized on the particle, partially immobilized on the particle, enclosed in the particle, partially enclosed in the particle, or a combination thereof. The particle can be disruptable. The particle can comprise a bead. The particle can comprise a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, a hydrogel bead, an anti-biotin microbead, an anti-fluorochrome microbead, or any combination thereof, or wherein the particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof. The particle can comprise a disruptable hydrogel bead.

In some embodiments, the barcodes of the particle comprise molecular label sequences selected from at least 1000, 10000, or a combination thereof, different molecular label sequences. The molecular label sequences of the barcodes can comprise random sequences. The particle can comprise at least 10000 barcodes.

In some embodiments, barcoding the sample indexing oligonucleotides using the plurality of barcodes comprises: contacting the plurality of barcodes with the sample indexing oligonucleotides to generate barcodes hybridized to the sample indexing oligonucleotides; and extending the barcodes hybridized to the sample indexing oligonucleotides to generate the plurality of barcoded sample indexing oligonucleotides.

In some embodiments, the method comprises, prior to extending the barcodes hybridized to the sample indexing oligonucleotides, pooling the barcodes hybridized to the sample indexing oligonucleotides, and wherein extending the barcodes hybridized to the sample indexing oligonucleotides comprises extending the pooled barcodes hybridized to the sample indexing oligonucleotides to generated a plurality of pooled barcoded sample indexing oligonucleotides. Extending the barcodes can comprise extending the barcodes using a DNA polymerase to generate the plurality of barcoded sample indexing oligonucleotides. Extending the barcodes can comprise extending the barcodes using a reverse transcriptase to generate the plurality of barcoded sample indexing oligonucleotides.

In some embodiments, the method comprises: amplifying the plurality of barcoded sample indexing oligonucleotides to produce a plurality of amplicons. Amplifying the plurality of barcoded sample indexing oligonucleotides can comprise amplifying, using polymerase chain reaction (PCR), at least a portion of the molecular label sequence and at least a portion of the sample indexing oligonucleotide. Obtaining the sequencing data of the plurality of barcoded sample indexing oligonucleotides can comprise obtaining sequencing data of the plurality of amplicons. Obtaining the sequencing data can comprise sequencing at least a portion of the molecular label sequence and at least a portion of the sample indexing oligonucleotide.

In some embodiments, barcoding the sample indexing oligonucleotides using the plurality of barcodes to generate the plurality of barcoded sample indexing oligonucleotides comprises stochastically barcoding the sample indexing oligonucleotides using a plurality of stochastic barcodes to generate a plurality of stochastically barcoded sample indexing oligonucleotides.

In some embodiments, the method comprises: barcoding a plurality of targets of the cell using the plurality of barcodes to generate a plurality of barcoded targets, wherein each of the plurality of barcodes comprises a cell label sequence, and wherein at least two barcodes of the plurality of barcodes comprise an identical cell label sequence; and obtaining sequencing data of the barcoded targets. Barcoding the plurality of targets using the plurality of barcodes to generate the plurality of barcoded targets can comprise: contacting copies of the targets with target-binding regions of the barcodes; and reverse transcribing the plurality targets using the plurality of barcodes to generate a plurality of reverse transcribed targets. The method can comprise: prior to obtaining the sequencing data of the plurality of barcoded targets, amplifying the barcoded targets to generate a plurality of amplified barcoded targets. Amplifying the barcoded targets to generate the plurality of amplified barcoded targets can comprise: amplifying the barcoded targets by polymerase chain reaction (PCR). Barcoding the plurality of targets of the cell using the plurality of barcodes to generate the plurality of barcoded targets can comprise stochastically barcoding the plurality of targets of the cell using a plurality of stochastic barcodes to generate a plurality of stochastically barcoded targets.

### Aptamer Sample Indexing Compositions

Disclosed herein are embodiments of a plurality of sample indexing compositions. In some embodiments, each of the plurality of sample indexing compositions comprises an aptamer composition comprising a first cellular component-binding aptamer and a sample indexing oligonucleotide, the cellular component-binding aptamer is capable of specifically binding to at least one cellular component target, the sample indexing oligonucleotide comprises a sample indexing sequence for identifying sample origin of one or more cells of a sample, and sample indexing sequences of at least two sample indexing compositions of the plurality of sample indexing compositions comprise different sequences.

### Second Cellular Component-Binding Aptamer

In some embodiments, the sample indexing composition comprises a second cellular component-binding aptamer, and wherein the second cellular component-binding aptamer is capable of specifically binding to at least one of the one or more cellular component targets.

The cellular component-binding aptamer and the second cellular component-binding aptamer can be capable of binding to the same cellular component target of the one or more cellular component targets, and the second cellular component-binding aptamer can be not associated with the sample indexing oligonucleotide. The second cellular component-binding aptamer can be associated with a second sample indexing oligonucleotide comprising a second sample indexing sequence. The sample indexing sequence and the second sample indexing sequence can be identical. The sample indexing sequence and the second sample indexing sequence can be different.

In some embodiments, the cellular component-binding aptamer and the second cellular component-binding aptamer are at least 60%, 70%, 80%, 90%, or 95% identical in sequence. The cellular component-binding aptamer and the second cellular component-binding aptamer can be identical. The cellular component-binding aptamer and the second cellular component-binding aptamer can be capable of binding to different regions of the same protein target. The cellular component-binding aptamer and the second cellular component-binding aptamer can be capable of binding to different protein targets of the one or more protein targets.

### Sample Indexing Oligonucleotide

In some embodiments, a single polynucleotide comprises the sample indexing oligonucleotide and the cellular component-binding aptamer. The aptamer can be 5' to the sample indexing oligonucleotide in the single polynucleotide. The aptamer can be 3' to the sample indexing oligonucleotide in the single polynucleotide.

In some embodiment, the sample indexing oligonucleotide is associated with the aptamer. The sample indexing oligonucleotide can be attached to the cellular component-binding aptamer. The sample indexing oligonucleotide can be covalently attached to the cellular component-binding aptamer. The sample indexing oligonucleotide can be conjugated to the cellular component-binding aptamer. The sample indexing oligonucleotide can be conjugated to the cellular component-binding aptamer through a chemical group selected from the group consisting of a UV photocleavable group, a streptavidin, a biotin, an amine, and a combination thereof. The sample indexing oligonucleotide can be non-covalently attached the cellular component-binding aptamer. The sample indexing oligonucleotide can be associated with the protein-binding aptamer, or the cellular component-binding aptamer, through a linker.

In some embodiments, the sample indexing sequence is 6-60 nucleotides in length. The sample indexing oligonucleotide can be 50-500 nucleotides in length. Sample indexing sequences of at least 10, 100, or 1000 sample indexing compositions of the plurality of sample indexing compositions can comprise different sequences.

In some embodiments, the sample indexing oligonucleotide comprises a molecular label sequence, a poly(dA) region, or a combination thereof. The molecular label sequence can be 2-20 nucleotides in length. The universal primer can be 5-50 nucleotides in length. The universal primer can comprise an amplification primer (e.g., an Illumina P7 sequence or a subsequence thereof), a sequencing primer (e.g., an Illumina R2 sequence or a subsequence thereof), or a combination thereof.

In some embodiments, the sample indexing oligonucleotide is not homologous to genomic sequences of any of the one or more cells. At least one sample of the plurality of samples can comprise one or more single cells, a plurality of cells, a tissue, a tumor sample, or any combination thereof. The sample can comprise a mammalian sample, a bacterial sample, a viral sample, a yeast sample, a fungal sample, or any combination thereof.

In some embodiments, the sample indexing oligonucleotide comprises a sequence complementary to a capture sequence configured to capture (or capable of capturing, hybridizing to, or binding to) the sequence of the sample indexing oligonucleotide. The target-binding region can comprise a poly(dT) region. The sequence of the sample indexing oligonucleotide complementary to the capture sequence can comprise a poly(dA) region.

### Alignment Sequence

In some embodiments, the sample indexing oligonucleotide comprises an alignment sequence adjacent to the poly(dA) region. The alignment sequence can be or more nucleotides in length. The alignment sequence can be 2 or more nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof.

### Aptamer

In some embodiments, the protein-binding aptamer, or the cellular component-binding aptamer, comprises a nucleotide aptamer. The nucleotide aptamer can comprise deoxyribonucleic acid (DNA), ribonucleic acid (RNA), xeno nucleic acid (XNA), or a combination thereof. The nucleotide aptamer can comprise a base analogue. The base analogue can comprise a fluorescent base analogue. The nucleotide aptamer can comprise a fluorophore. The cellular component-binding aptamer can comprise a peptide aptamer.

In some embodiments, the cellular component-binding aptamer is associated with two or more sample indexing oligonucleotides with an identical sequence. The cellular component-binding aptamer can be associated with two or more sample indexing oligonucleotides with different sample indexing sequences.

### Carrier

In some embodiments, the sample indexing composition is associated with a first carrier. Different sample indexing compositions of the plurality of sample indexing compositions can be associated with different first carriers. In some embodiments, the aptamer composition is associated with a first carrier.

In some embodiments, the first carrier comprises metal nanomaterial. The first carrier can comprise a metal nanostructure, a metal nanoparticle, or a combination thereof. The first carrier can comprise gold nanomaterial. The first carrier can comprise a gold nanostructure, a gold nanoparticle, or a combination thereof. The first carrier can comprise a lysosome, a micelle, a vesicle, a lipid membrane, a lipid bilayer, a lipid monolayer, or a combination thereof.

In some embodiments, the cellular component-binding aptamer is attached to the first carrier. The cellular component-binding aptamer can be covalently attached to the first carrier. The cellular component-binding aptamer can be conjugated to the first carrier. The cellular component-binding aptamer can be conjugated to the first carrier through a chemical group selected from the group consisting of a UV photocleavable group, a streptavidin, a biotin, an amine, and a combination thereof. The cellular component-binding aptamer can be non-covalently linked to the first carrier. The cellular component-binding aptamer can be associated with the first carrier through a linker.

In some embodiments, the cellular component-binding aptamer is immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

In some embodiments, the first carrier is configured to be internalized (or capable of being internalized) into the cell. The first carrier can be internalized into the cell via endocytosis, pinocytosis, nanopinocytosis, micropinocytosis, phagocytosis, membrane fusion, or a combination thereof. The first carrier can be internalized into the cell via clathrin-medicated internalization, caveolin-mediated internalization, receptor dependent internalization, receptor independent internalization or a combination thereof.

### Second Cellular Component-Binding Aptamer-Binding Aptamer

In some embodiments, the aptamer composition comprises: a second cellular component-binding aptamer capable of specifically binding to at least one of the one or more protein targets or one or more cellular component targets, and a second sample indexing oligonucleotide comprising a second sample indexing sequence.

In some embodiments, the cellular component-binding aptamer and the second cellular component-binding aptamer can have at least 60%, 70%, 80%, 90%, or 95% sequence identity. The cellular component-binding aptamer and the second cellular component-binding aptamer can be identical. The cellular component-binding aptamer and the second cellular component-binding aptamer can be different. In some embodiments, the cellular component-binding aptamer and the second cellular component-binding aptamer can be associated with a first carrier. The cellular component-binding aptamer can be associated with a first carrier, and the second cellular component-binding aptamer can be associated with a second carrier.

In some embodiments, the protein targets, or the cellular component targets, of the cellular component-binding aptamer and the second cellular component-binding aptamer are identical. The cellular component-binding aptamer and the second cellular component-binding aptamer can be capable of binding to different regions of a protein target, or a cellular component target. The protein targets, or the cellular component targets, of the cellular component-binding aptamer and the second cellular component-binding aptamer can be different.

### Aptamer Sample Indexing Kit

Disclosed herein are embodiments of a kit for sample identification. In some embodiments, the kit comprises a plurality of sample indexing compositions. In some embodiments, the kit comprises a plurality of barcodes. A barcode of the plurality of barcodes comprises a target-binding region. The target-binding region can comprise a capture sequence configured to capture (or capable of capturing, hybridizing to or binding to) a sequence of a sample indexing oligonucleotide. A cellular component-binding aptamer can comprise the sample indexing oligonucleotide and a first cellular component-binding aptamer.

### EXAMPLES

Some aspects of the embodiments discussed above are disclosed in further detail in the following examples, which are not in any way intended to limit the scope of the present disclosure.

### Example 1

### Oligonucleotides for Associating with Protein Binding Reagents

This example demonstrates designing of oligonucleotides that can be conjugated with protein binding reagents. The oligonucleotides can be used to determine protein expression and gene expression simultaneously. The oligonucleotides can also be used for sample indexing to determine cells of the same or different samples.

### 95mer Oligonucleotide Design

The following method was used to generate candidate oligonucleotide sequences and corresponding primer sequences for simultaneous determination of protein expression and gene expression or sample indexing.

### 1. Sequence generation and elimination

The following process was used to generate candidate oligonucleotide sequences for simultaneous determination of protein expression and gene expression or sample indexing.

Step 1a. Randomly generate a number of candidate sequences (50000 sequences) with the desired length (45 bps).

Step 1b. Append the transcriptional regulator LSRR sequence to the 5' end of the sequences generated and a poly(dA) sequence (25 bps) to the 3' end of the sequences generated.

Step 1c. Remove sequences generated and appended that do not have GC contents in the range of 40% to 50%.

Step 1d. Remove remaining sequences with one or more hairpin structures each.

The number of remaining candidate oligonucleotide sequences was 423.

### 2. Primer design

The following method was used to design primers for the remaining 423 candidate oligonucleotide sequences.

2.1 N1 Primer: Use the universal N1 sequence: 5'-GTTGTCAAGATGCTACCGTTCAGAG-3' (LSRR sequence; SEQ ID NO. 5) as the N1 primer.

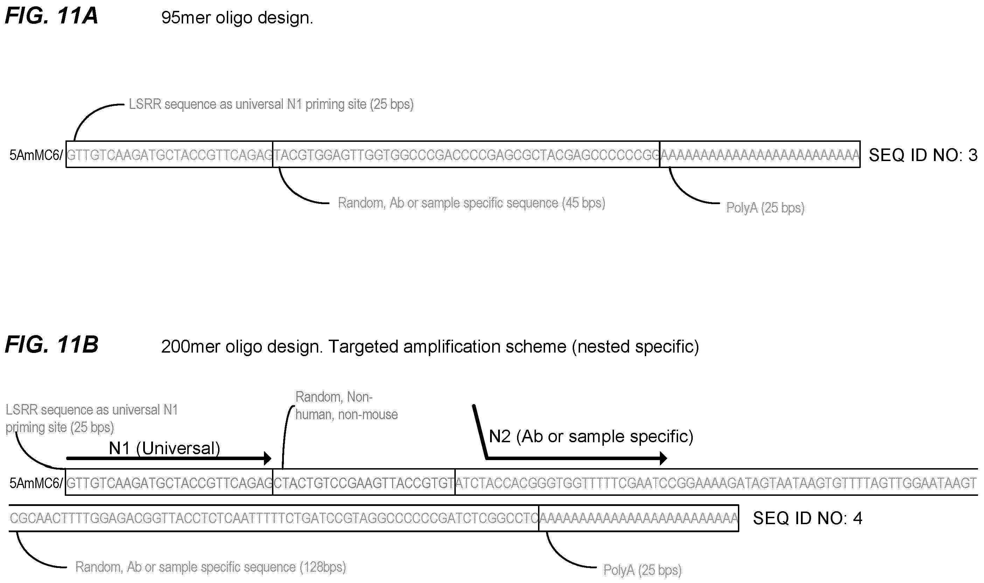

2.2 N2 Primer (for amplifying specific sample index oligonucleotides; e.g., N2 primer in FIGS. 11B-11D):
2.2a. Remove candidate N2 primers that do not start downstream of the N1 sequence.
2.2b. Remove candidate N2 primers that overlap in the last 35 bps of the candidate oligonucleotide sequence.
2.2c. Remove the primer candidates that are aligned to the transcriptome of the species of cells being studied using the oligonucleotides (e.g., the human transcriptome or the mouse transcriptome).
2.2d. Use the ILR2 sequence as the default control (ACACGACGCTCTTCCGATCT; SEQ ID NO. 6) to minimize or avoid primer-primer interactions.

Of the 423 candidate oligonucleotide sequences, N2 primers for 390 candidates were designed.

### 3. Filtering

The following process was used to filter the remaining 390 candidate primer sequences.

3a. Eliminate any candidate oligonucleotide sequence with a random sequence ending in As (i.e., the effective length of the poly(dA) sequence is greater than 25 bps) to keep poly(dA) tail the same length for all barcodes.

3b. Eliminate any candidate oligonucleotide sequences with 4 or more consecutive Gs (> 3Gs) because of extra cost and potentially lower yield in oligo synthesis of runs of Gs.

FIG. 11A shows a non-limiting exemplary candidate oligonucleotide sequence generated using the method above.

### 200mer Oligonucleotide Design

The following method was used to generate candidate oligonucleotide sequences and corresponding primer sequences for simultaneous determination of protein expression and gene expression and sample indexing.

### 1. Sequence generation and elimination

The following was used to generate candidate oligonucleotide sequences for simultaneous determination of protein expression and gene expression and sample indexing.

1a. Randomly generate a number of candidate sequences (100000 sequences) with the desired length (128 bps).

1b. Append the transcriptional regulator LSRR sequence and an additional anchor sequence that is non-human, non-mouse to the 5' end of the sequences generated and a poly(dA) sequence (25 bps) to the 3' end of the sequences generated.

1c. Remove sequences generated and appended that do not have GC contents in the range of 40% to 50%.

1d. Sort remaining candidate oligonucleotide sequences based on hairpin structure scores.

1e. Select 1000 remaining candidate oligonucleotide sequences with the lowest hairpin scores.

### 2. Primer design

The following method was used to design primers for 400 candidate oligonucleotide sequences with the lowest hairpin scores.

2.1 N1 Primer: Use the universal N1 sequence: 5'-GTTGTCAAGATGCTACCGTTCAGAG-3' (LSRR sequence; SEQ ID NO. 5) as the N1 primer.

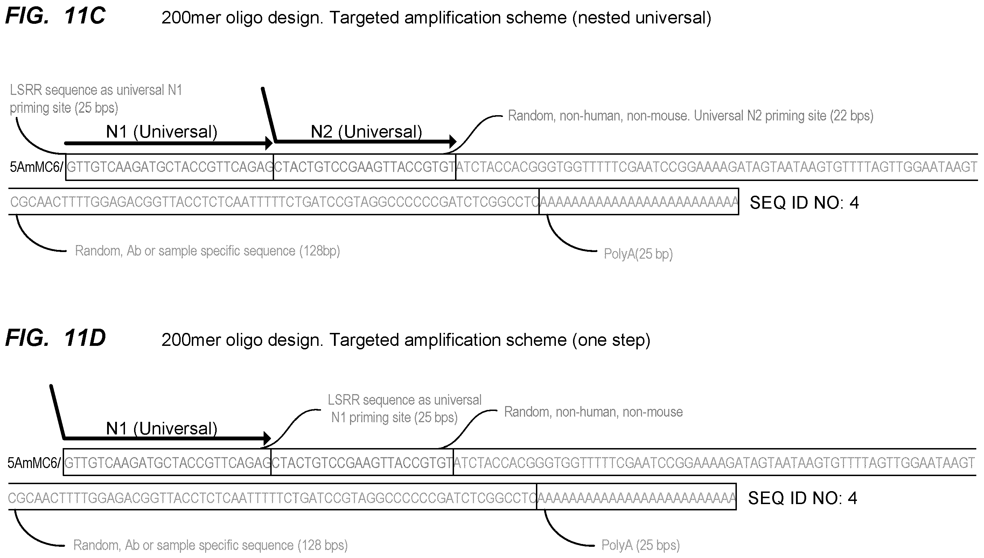

2.2 N2 Primer (for amplifying specific sample index oligonucleotides; e.g., N2 primer in FIGS. 11B and 11C):
2.2a. Remove candidate N2 primers that do not start 23 nts downstream of the N1 sequence (The anchor sequence was universal across all candidate oligonucleotide sequences).
2.2b. Remove candidate N2 primers that overlap in the last 100 bps of the target sequence. The resulting primer candidates can be between the 48th nucleotide and 100th nucleotide of the target sequence.
2.2c. Remove the primer candidates that are aligned to the transcriptome of the species of cells being studied using the oligonucleotides (e.g., the human transcriptome or the mouse transcriptome).
2.2d. Use the ILR2 sequence, 5'-ACACGACGCTCTTCCGATCT-3' (SEQ ID NO. 6) as the default control to minimize or avoid primer-primer interactions.
2.2e. Remove N2 primer candidates that overlap in the last 100 bps of the target sequence.

Of the 400 candidate oligonucleotide sequences, N2 primers for 392 candidates were designed.

### 3. Filtering

The following was used to filter the remaining 392 candidate primer sequences.

3a. Eliminate any candidate oligonucleotide sequence with a random sequence ending in As (i.e., the effective length of the poly(dA) sequence is greater than 25 bps) to keep poly(dA) tail the same length for all barcodes.

3b. Eliminate any candidate oligonucleotide sequences with 4 or more consecutive Gs (> 3Gs) because of extra cost and potentially lower yield in oligo synthesis of runs of Gs.

FIG. 11B shows a non-limiting exemplary candidate oligonucleotide sequence generated using the method above. The nested N2 primer shown in FIG. 11B can bind to the antibody or sample specific sequence for targeted amplification. FIG. 11C shows the same non-limiting exemplary candidate oligonucleotide sequence with a nested universal N2 primer that corresponds to the anchor sequence for targeted amplification. FIG. 11D shows the same non-limiting exemplary candidate oligonucleotide sequence with a N2 primer for one step targeted amplification.

Altogether, these data indicate that oligonucleotide sequences of different lengths can be designed for simultaneous determination of protein expression and gene expression or sample indexing. The oligonucleotide sequences can include a universal primer sequence, an antibody specific oligonucleotide sequence or a sample indexing sequence, and a poly(dA) sequence.

### Example 2

### Oligonucleotide-Associated Antibody Workflow

This example demonstrates a workflow of using an oligonucleotide-conjugated antibody for determining the expression profile of a protein target.

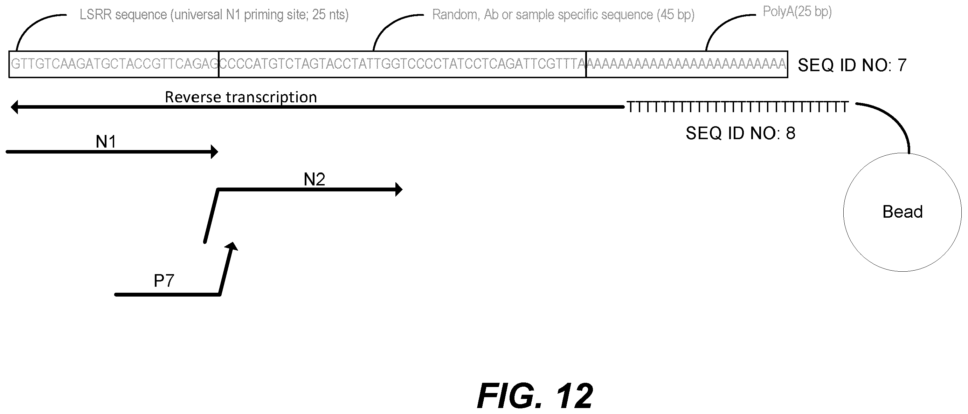

Frozen cells (e.g., frozen peripheral blood mononuclear cells (PBMCs)) of a subject are thawed. The thawed cells are stained with an oligonucleotide-conjugated antibody (e.g., an anti-CD4 antibody at 0.06 µg/100 µl (1:333 dilution of an oligonucleotide-conjugated antibody stock)) at a temperature for a duration (e.g., room temperature for 20 minutes). The oligonucleotide-conjugated antibody is conjugated with 1, 2, or 3 oligonucleotides ("antibody oligonucleotides"). The sequence of the antibody oligonucleotide is shown in FIG. 12. The cells are washed to remove unbound oligonucleotide-conjugated antibody. The cells are optionally stained with Calcein AM (BD (Franklin Lake, New Jersey)) and Draq7^{™} (Abcam (Cambridge, United Kingdom)) for sorting with flow cytometry to obtain cells of interest (e.g., live cells). The cells are optionally washed to remove excess Calcein AM and Draq7^{™}. Single cells stained with Calcein AM (live cells) and not Draq7^{™} (cells that are not dead or permeabilized) are sorted, using flow cytometry, into a BD Rhapsody^{™} cartridge.

Of the wells containing a single cell and a bead, the single cells in the wells (e.g., 3500 live cells) are lysed in a lysis buffer (e.g., a lysis buffer with 5 mM DTT). The mRNA expression profile of a target (e.g., CD4) is determined using BD Rhapsody^{™} beads. The protein expression profile of a target (e.g., CD4) is determined using BD Rhapsody^{™} beads and the antibody oligonucleotides. Briefly, the mRNA molecules are released after cell lysis. The Rhapsody^{™} beads are associated with barcodes (e.g., stochastic barcodes) each containing a molecular label, a cell label, and an oligo(dT) region. The poly(A) regions of the mRNA molecules released from the lysed cells hybridize to the poly(T) regions of the stochastic barcodes. The poly(dA) regions of the antibody oligonucleotides hybridize to the oligo(dT) regions of the barcodes. The mRNA molecules were reverse transcribed using the barcodes. The antibody oligonucleotides are replicated using the barcodes. The reverse transcription and replication optionally occur in one sample aliquot at the same time.

The reverse transcribed products and replicated products are PCR amplified using primers for determining mRNA expression profiles of genes of interest, using N1 primers, and the protein expression profile of a target, using the antibody oligonucleotide N1 primer. For example, the reverse transcribe products and replicated products can be PCR amplified for 15 cycles at 60 degrees annealing temperature using primers for determining the mRNA expression profiles of 488 blood panel genes, using blood panel N1 primers, and the expression profile of CD4 protein, using the antibody oligonucleotide N1 primer ("PCR 1"). Excess barcodes are optionally removed with Ampure cleanup. The products from PCR 1 are optionally divided into two aliquots, one aliquot for determining the mRNA expression profiles of the genes of interest, using the N2 primers for the genes of interest, and one aliquot for determining the protein expression profile of the target of interest, using the antibody oligonucleotide N2 primer ("PCR 2"). Both aliquots are PCR amplified (e.g., for 15 cycles at 60 degrees annealing temperature). The protein expression of the target in the cells are determined based on the antibody oligonucleotides as illustrated in FIG. 12 ("PCR 2"). Sequencing data is obtained and analyzed after sequencing adaptor addition ("PCR 3"), such as sequencing adaptor ligation. Cell types are determined based on the mRNA expression profiles of the genes of interest.

Altogether, this example describes using an oligonucleotide-Conjugated antibody for determining the protein expression profile of a target of interest. This example further describes that the protein expression profile of the target of interest and the mRNA expression profiles of genes of interest can be determine simultaneously.

### Terminology

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g.,* bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.,* the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.,* " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.,* " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

## Claims

1. A method for sample identification, comprising:
contacting each of a plurality of samples with a sample indexing composition of a plurality of sample indexing compositions, respectively,
wherein each of the plurality of samples comprises one or more cells each comprising one or more cellular component targets, wherein the sample indexing composition comprises an aptamer composition comprising an aptamer and a sample indexing oligonucleotide, wherein a single polynucleotide comprises the sample indexing oligonucleotide and the aptamer, wherein the aptamer is capable of specifically binding to at least one of the one or more cellular component targets, wherein the sample indexing composition of the plurality of sample indexing compositions comprises a second aptamer composition comprising a second aptamer, wherein the second aptamer is capable of specifically binding to at least one of the one or more cellular component targets, wherein the second aptamer is associated with a second sample indexing oligonucleotide comprising a second sample indexing sequence, and wherein the aptamer and the second aptamer are capable of binding to different cellular component targets of the one or more cellular component targets,
wherein the sample indexing oligonucleotide comprises a sample indexing sequence, wherein sample indexing sequences of at least two sample indexing compositions of the plurality of sample indexing compositions comprise different sequences, and wherein the sample indexing sequence and the second sample indexing sequence are identical; and
identifying sample origin of at least one cell of the one or more cells based on the sample indexing sequence of at least one sample indexing oligonucleotide of the plurality of sample indexing compositions.

2. The method of claim 1, wherein:
(a) identifying the sample origin of the at least one cell comprises:
barcoding sample indexing oligonucleotides of the plurality of sample indexing compositions using a plurality of barcodes to generate a plurality of barcoded sample indexing oligonucleotides;
obtaining sequencing data of the plurality of barcoded sample indexing oligonucleotides; and
identifying the sample origin of the cell based on the sample indexing sequence of at least one barcoded sample indexing oligonucleotide of the plurality of barcoded sample indexing oligonucleotides in the sequencing data ;
(b) identifying the sample origin of the at least one cell comprises identifying the presence or absence of the sample indexing sequence of at least one sample indexing oligonucleotide of the plurality of sample indexing compositions, optionally wherein identifying the presence or absence of the sample indexing sequence comprises:
replicating the at least one sample indexing oligonucleotide to generate a plurality of replicated sample indexing oligonucleotides;
obtaining sequencing data of the plurality of replicated sample indexing oligonucleotides; and
identifying the sample origin of the cell based on the sample indexing sequence of a replicated sample indexing oligonucleotide of the plurality of sample indexing oligonucleotides that correspond to the least one barcoded sample indexing oligonucleotide in the sequencing data; and optionally wherein replicating the at least one sample indexing oligonucleotide to generate the plurality of replicated sample indexing oligonucleotides comprises:
(i) prior to replicating the at least one barcoded sample indexing oligonucleotide, ligating a replicating adaptor to the at least one barcoded sample indexing oligonucleotide, and wherein replicating the at least one barcoded sample indexing oligonucleotide comprises replicating the at least one barcoded sample indexing oligonucleotide using the replicating adaptor ligated to the at least one barcoded sample indexing oligonucleotide to generate the plurality of replicated sample indexing oligonucleotides; or
(ii) prior to replicating the at least one barcoded sample indexing oligonucleotide, contacting a capture probe with the at least one sample indexing oligonucleotide to generate a capture probe hybridized to the sample indexing oligonucleotide; and extending the capture probe hybridized to the sample indexing oligonucleotide to generate a sample indexing oligonucleotide associated with the capture probe, and wherein replicating the at least one sample indexing oligonucleotide comprises replicating the sample indexing oligonucleotide associated with the capture probe to generate the plurality of replicated sample indexing oligonucleotides.

3. A plurality of sample indexing compositions,
wherein each of the plurality of sample indexing compositions comprises an aptamer composition comprising a first cellular component-binding aptamer and a sample indexing oligonucleotide,
wherein a single polynucleotide comprises the sample indexing oligonucleotide and the aptamer,
wherein the cellular component-binding aptamer is capable of specifically binding to at least one cellular component target,
wherein the sample indexing oligonucleotide comprises a sample indexing sequence for identifying sample origin of one or more cells of a sample,
wherein sample indexing sequences of at least two sample indexing compositions of the plurality of sample indexing compositions comprise different sequences, and
wherein the aptamer composition comprises:
a second cellular component-binding aptamer capable of specifically binding to at least one cellular component target, and
a second sample indexing oligonucleotide comprising a second sample indexing sequence,
wherein the cellular component targets of the cellular component-binding aptamer and the second cellular component-binding aptamer are different, and
wherein the sample indexing sequence and the second sample indexing sequence are identical.

4. The method of any one of claims 1-2, or the plurality of sample indexing compositions of claim 3, wherein:
(a) the cellular component target comprises a protein target;
(b) the sample indexing sequence is 6-60 nucleotides in length or 50-500 nucleotides in length;
(c) sample indexing sequences of at least 10, 100, or 1000 sample indexing compositions of the plurality of sample indexing compositions comprise different sequences;
(d) the aptamer is:
(i) 5' to the sample indexing oligonucleotide in the single polynucleotide; or
(ii) 3' to the sample indexing oligonucleotide in the single polynucleotide; and/or
(e) the aptamer comprises a nucleotide aptamer, optionally the nucleotide aptamer comprises deoxyribonucleic acid (DNA), ribonucleic acid (RNA), xeno nucleic acid (XNA), a fluorophore, or a combination thereof, optionally wherein the nucleotide aptamer comprises a base analogue, and optionally the base analogue comprises a fluorescent base analogue.

5. The method of any one of claims 1-2 or 4, or the plurality of sample indexing compositions of claim 3 or 4, wherein:
(a) the sample indexing composition is associated with a first carrier;
(b) the aptamer composition is associated with a first carrier; and/or
(c) wherein:
(i) the aptamer and the second aptamer are associated with a first carrier; or
(ii) the aptamer is associated with a first carrier and the second aptamer is associated with a second carrier.

6. The method or the plurality of sample indexing compositions of claim 4 or 5, wherein:
(a) the first carrier comprises:
(i) metal nanomaterial, and optionally the mental nanomaterial comprises a metal nanostructure, a metal nanoparticle, or a combination thereof; and/or
(ii) gold nanomaterial, and optionally the gold nanomaterial is a gold nanostructure, a gold nanoparticle, or a combination thereof; and/or
(iii) a lysosome, a micelle, a vesicle, a lipid membrane, a lipid bilayer, a lipid monolayer, or a combination thereof;
(b) the aptamer is attached to the first carrier, and optionally the aptamer is covalently attached to the first carrier;
(c) the aptamer is conjugated to the first carrier, and optionally the aptamer is conjugated to the first carrier through a chemical group selected from the group consisting of a UV photocleavable group, a streptavidin, a biotin, an amine, and a combination thereof;
(d) the aptamer is non-covalently linked to the first carrier;
(e) the aptamer is associated with the first carrier through a linker; and/or
(f) the aptamer is immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

7. The method of any one of claims 5(c) or 6:
(a) comprising dissociating the aptamer from the first carrier, optionally wherein the dissociating occurs:
(i) after barcoding the sample indexing oligonucleotides; or
(ii) before barcoding the sample indexing oligonucleotides; and/or
(b) wherein contacting each of the plurality of samples with the sample indexing composition comprises contacting a cell of the one or more cells of the sample with the sample indexing composition, optionally wherein the first carrier is internalized into the cell, optionally the internalization is via endocytosis, pinocytosis, nanopinocytosis, micropinocytosis, phagocytosis, membrane fusion, clathrin-medicated internalization, caveolin-mediated internalization, receptor dependent internalization, receptor independent internalization, or a combination thereof.

8. The method of any one of claims 1-2 or 4-7, wherein:
(a) the at least one of the one or more protein targets, or the one or more cellular component targets, is on a cell surface;
(b) the method comprises removing unbound sample indexing compositions of the plurality of sample indexing compositions;
(c) removing the unbound sample indexing compositions comprises (a) washing the one or more cells from each of the plurality of samples with a washing buffer, (b) selecting cells bound to at least one aptamer using flow cytometry, or both; and/or
(d) the method comprises lysing the one or more cells from each of the plurality of samples.

9. The method of any one of claims 1-2 or 4-8, or the plurality of sample indexing compositions of any one of claims 3-6, wherein:
(a) the sample indexing oligonucleotide is not homologous to genomic sequences of any of the one or more cells, is homologous to genomic sequences of a species, or a combination thereof, and optionally the species is a non-mammalian species;
(b) a sample of the plurality of samples comprises a plurality of cells, a plurality of single cells, a tissue, a tumor sample, or any combination thereof;
(c) the plurality of samples comprises a mammalian cell, a bacterial cell, a viral cell, a yeast cell, a fungal cell, or any combination thereof;
(d) the sample indexing oligonucleotide comprises a sequence complementary to a capture sequence configured to capture the sequence of the sample indexing oligonucleotide, optionally wherein:
(i) the barcode comprises a target-binding region which comprises the capture sequence, and optionally the target-binding region comprises a poly(dT) region; or
(ii) the sequence of the sample indexing oligonucleotide complementary to the capture sequence comprises a poly(dA) region, optionally wherein the sample indexing oligonucleotide comprises an alignment sequence adjacent to the poly(dA) region, and optionally (a) the alignment sequence is one or more nucleotides, or two or more nucleotides in length, (b) the alignment sequence comprises a guanine, a cytosine, a thymine, a uracil, or a combination thereof, and/or (c) the alignment sequence comprises a poly(dT) region, a poly(dG) region, a poly(dC) region, a poly(dU) region, or a combination thereof; and/or
(e) the sample indexing oligonucleotide comprises a molecular label sequence, a binding site for a universal primer, or both, and optionally the molecular label sequence is 2-20 nucleotides in length or 5-50 nucleotides in length, and optionally the universal primer comprises an amplification primer, a sequencing primer, or a combination thereof;
(f) the protein target, or the cellular component target, comprises a carbohydrate, a lipid, a protein, an extracellular protein, a cell-surface protein, a cell marker, a B-cell receptor, a T-cell receptor, a major histocompatibility complex, a tumor antigen, a receptor, an intracellular protein, or any combination thereof;
(g) the protein target, or the cellular component target, is selected from a group comprising 10-100 different protein targets, or cellular component targets; and/or
(h) the aptamer is associated with two or more sample indexing oligonucleotides with:
(A) an identical sequence; or
(B) different sample indexing sequences and/or
(i) the sample indexing composition of the plurality of sample indexing compositions comprises an aptamer not associated with the sample indexing oligonucleotide; and/or
(j) each of the plurality of sample indexing compositions comprises the aptamer; and/or
(k) the aptamer, or the cellular component-binding aptamer, is capable of binding to ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2, or any combination thereof; and/or
(l) the second aptamer, or the second cellular component-binding aptamer, is capable of binding to ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2, or any combination thereof.

10. A method of measuring cellular component expression in cells, comprising:
contacting a plurality of cellular component-binding aptamers with a plurality of cells comprising a plurality of cellular component targets, wherein each of the plurality of cellular component-binding aptamers comprises an aptamer specific oligonucleotide comprising a unique identifier sequence for the cellular component-binding aptamer, wherein the aptamer specific oligonucleotide and the aptamer form a single polynucleotide, wherein the cellular component-binding aptamer is capable of specifically binding to at least one of the plurality of cellular component targets, and wherein the plurality of cellular component-binding aptamers comprises a second cellular component-binding aptamer not conjugated to an oligonucleotide;
extending oligonucleotide probes hybridized to the aptamer specific oligonucleotides to produce a plurality of labeled nucleic acids, wherein each of the labeled nucleic acid comprises a unique identifier sequence, or a complementary sequence thereof, and a barcode sequence; and
obtaining sequence information of the plurality of labeled nucleic acids or a portion thereof to determine the quantity of one or more of the plurality of cellular component targets in one or more of the plurality of cells.

11. The method of claim 10, comprising prior to extending the oligonucleotide probes:
partitioning the plurality of cells associated with the plurality of cellular component-binding aptamers to a plurality of partitions, wherein a partition of the plurality of partitions comprises a single cell from the plurality of cells associated with the cellular component-binding aptamers;
in the partition comprising the single cell, contacting a barcoding particle with the aptamer specific oligonucleotides, wherein the barcoding particle comprises a plurality of oligonucleotide probes each comprising a target binding region and a barcode sequence selected from a diverse set of unique barcode sequences.

12. A composition comprising:
a plurality of cellular component-binding aptamers, wherein each of the plurality of cellular component-binding aptamers comprises an aptamer specific oligonucleotide comprising a unique identifier sequence for the cellular component-binding aptamer, wherein the aptamer specific oligonucleotide and the aptamer form a single polynucleotide, wherein the cellular component-binding aptamer is capable of specifically binding to at least one of a plurality of cellular component targets, and wherein the plurality of cellular component-binding aptamers comprises a second cellular component-binding aptamer not conjugated to an oligonucleotide.

13. The method of any one of claims 10-11, or the composition of claim 12, wherein:
(a) the plurality of cellular component targets comprises a plurality of protein targets, and wherein the cellular component-binding aptamer is capable of specifically binding to at least one of the plurality of protein targets; and/or
(b) the aptamer is:
(i) 5' to the aptamer specific oligonucleotide in the single polynucleotide; or
(ii) 3' to the aptamer specific oligonucleotide in the single polynucleotide; and/or
(c) the aptamer comprises a nucleotide aptamer, and optionally the nucleotide aptamer comprises deoxyribonucleic acid (DNA), ribonucleic acid (RNA), xeno nucleic acid (XNA), a fluorophore, or a combination thereof, optionally wherein the nucleotide aptamer comprises a base analogue, optionally the base analogue comprises a fluorescent base analogue.

14. The method of any one of claims 10-11 or 13, or the composition of claim 12 or 13, wherein:
(a) the aptamer:
(i) and the second aptamer are associated with a first carrier; or
(ii) is associated with a first carrier, and wherein the second aptamer is associated with a second carrier; and/or
(b) the aptamer and the second aptamer are at least 60%, 70%, 80%, 90%, or 95% identical in sequence; and/or
(c) the aptamer and the second protein-binding aptamer are:
(i) identical; or
(ii) different, optionally wherein:
the protein targets, or the cellular component targets, of the aptamer and the second aptamer are identical; and/or
(d) the first carrier comprises
(i) metal nanomaterial, and optionally the metal nanomaterial is a metal nanostructure, a metal nanoparticle, or a combination thereof; and/or
(ii) gold nanomaterial, and optionally the gold nanomaterial is a gold nanostructure, a gold nanoparticle, or a combination thereof; and/or
(iii) a lysosome, a micelle, a vesicle, a lipid membrane, a lipid bilayer, a lipid monolayer, or a combination thereof; and/or
(e) the aptamer is attached to the first carrier, and optionally the aptamer is covalently attached to the first carrier;
(f) the aptamer is conjugated to the first carrier, and optionally the aptamer is conjugated to the first carrier through a chemical group selected from the group consisting of a UV photocleavable group, a streptavidin, a biotin, an amine, and a combination thereof;
(g) the aptamer is non-covalently linked to the first carrier;
(h) the aptamer is associated with the first carrier through a linker; and/or
(i) the aptamer is immobilized on the first carrier, partially immobilized on the first carrier, immobilized in the first carrier, partially immobilized in the first carrier, enclosed in the first carrier, partially enclosed in the first carrier, embedded in the first carrier, partially embedded in the first carrier, or a combination thereof.

15. The method or composition of claim 14, wherein the aptamer specific oligonucleotide comprises a sequence complementary to a capture sequence configured to capture the sequence of the aptamer specific oligonucleotide, optionally wherein:
(a) the barcode comprises a target-binding region which comprises the capture sequence, and optionally target-binding region comprises a poly(dT) region; and/or
(b) the sequence of the aptamer specific oligonucleotide complementary to the capture sequence comprises a poly(dA) region, optionally wherein:
(i) the aptamer specific oligonucleotide changes from a first conformation in which the poly(dA) region is inaccessible to a second conformation in which the poly(dA) region is accessible when the aptamer contacts the at least one of the plurality of protein targets, or cellular component targets, optionally wherein the poly(dA) region of the aptamer specific oligonucleotide in the first conformation comprises a hairpin structure, and optionally the poly(dA) region with the hairpin structure is accessible to the poly(dT) region of the target-binding region of the barcode;
(ii) the oligonucleotide probes are hybridized to the aptamer specific oligonucleotides via the hybridization between the poly(dA) regions of the aptamer specific oligonucleotides and the poly(dT) regions of the oligonucleotide probes; and/or
(iii) the aptamer specific oligonucleotide comprises an alignment sequence adjacent to the poly(dA) region, and optionally (a) the alignment sequence is one or more nucleotides in length, or two or more nucleotides in length, (b) the alignment sequence comprises a guanine, a cytosine, a thymine, a uracil, or a combination thereof, and/or (c) the alignment sequence comprises a poly(dT) region, a poly(dG) region, a poly(dC) region, a poly(dU) region, or a combination thereof; and/or
(c) the aptamer specific oligonucleotide comprises a molecular label sequence, a binding site for a universal primer, or both, and optionally the molecular label sequence is 2-20 nucleotides in length, or the universal primer is 5-50 nucleotides in length, or both, optionally wherein the universal primer comprises an amplification primer, a sequencing primer, or a combination thereof.

## Patentansprüche

1. Verfahren zur Probenidentifizierung, umfassend:
In-Kontakt-Bringen einer von mehreren Proben mit jeweils einer Probenindizierungszusammensetzung von mehreren Probenindizierungszusammensetzungen,
wobei die mehreren Proben jeweils eine oder mehrere Zellen umfassen, die jeweils ein oder mehrere Zellkomponentenziele umfassen, wobei die Probenindizierungszusammensetzung eine Aptamerzusammensetzung umfasst, die ein Aptamer und ein Probenindizierungsoligonukleotid umfasst, wobei ein einzelnes Polynukleotid das Probenindizierungsoligonukleotid und das Aptamer umfasst, wobei das Aptamer in der Lage ist, an mindestens eines der ein oder mehreren Zellkomponentenziele spezifisch zu binden, wobei die Probenindizierungszusammensetzung der mehreren Probenindizierungszusammensetzungen eine zweite Aptamerzusammensetzung umfasst, die ein zweites Aptamer umfasst, wobei das zweite Aptamer in der Lage ist, an mindestens eines der ein oder mehreren Zellkomponentenziele spezifisch zu binden, wobei das zweite Aptamer mit einem zweiten Probenindizierungsoligonukleotid, das eine zweite Probenindizierungssequenz umfasst, assoziiert ist und wobei das Aptamer und das zweite Aptamer in der Lage sind, an unterschiedliche Zellkomponentenziele der ein oder mehreren Zellkomponentenziele zu binden,
wobei das Probenindizierungsoligonukleotid eine Probenindizierungssequenz umfasst, wobei Probenindizierungssequenzen von mindestens zwei Probenindizierungszusammensetzungen der mehreren Probenindizierungszusammensetzungen unterschiedliche Sequenzen umfassen und wobei die Probenindizierungssequenz und die zweite Probenindizierungssequenz identisch sind; und
Identifizieren von Probenursprung für mindestens eine Zelle der einen oder mehreren Zellen anhand der Probenindizierungssequenz mindestens eines Probenindizierungsoligonukleotids der mehreren Probenindizierungszusammensetzungen.

2. Verfahren nach Anspruch 1, wobei:
(a) Identifizieren des Probenursprungs der mindestens einen Zelle Folgendes umfasst:
Strichcodieren von Probenindizierungsoligonukleotiden der mehreren Probenindizierungszusammensetzungen unter Verwendung mehrerer Strichcodes, so dass mehrere strichcodierte Probenindizierungsoligonukleotide erzeugt werden;
Gewinnen von Sequenzierdaten der mehreren strichcodierten Probenindizierungsoligonukleotide; und
Identifizieren des Probenursprungs der Zelle anhand der Probenindizierungssequenz mindestens eines strichcodierten Probenindizierungsoligonukleotids der mehreren strichcodierten Probenindizierungsoligonukleotide in den Sequenzierdaten;
(b) Identifizieren des Probenursprungs der mindestens einen Zelle Identifizieren des Vorliegens oder Fehlens der Probenindizierungssequenz mindestens eines Probenindizierungsoligonukleotids der mehreren Probenindizierungszusammensetzungen umfasst, gegebenenfalls wobei Identifizieren des Vorliegens oder Fehlens der Probenindizierungssequenz Folgendes umfasst:
Replizieren des mindestens einen Probenindizierungsoligonukleotids zur Erzeugung mehrerer replizierter Probenindizierungsoligonukleotide;
Gewinnen von Sequenzierdaten der mehreren replizierten Probenindizierungsoligonukleotide; und
Identifizieren des Probenursprungs der Zelle anhand der Probenindizierungssequenz eines replizierten Probenindizierungsoligonukleotids der mehreren Probenindizierungsoligonukleotide, die dem mindestens einen strichcodierten Probenindizierungsoligonukleotid in den Sequenzierdaten entsprechen; und gegebenenfalls wobei Replizieren des wenigstens einen Probenindizierungsoligonukleotids zur Erzeugung der mehreren replizierten Probenindizierungsoligonukleotide Folgendes umfasst:
(i) vor dem Replizieren des mindestens einen strichcodierten Probenindizierungsoligonukleotids Ligieren eines Replizieradapters an das mindestens eine strichcodierte Probenindizierungsoligonukleotid, und wobei Replizieren des mindestens einen strichcodierten Probenindizierungsoligonukleotids Replizieren des mindestens einen strichcodierten Probenindizierungsoligonukleotids unter Verwendung des an das mindestens eine strichcodierte Probenindizierungsoligonukleotid ligierten Replizieradapters umfasst, so dass die mehreren replizierten Probenindizierungsoligonukleotide erzeugt werden; oder
(ii) vor dem Replizieren des mindestens einen strichcodierten Probenindizierungsoligonukleotids In-Kontakt-Bringen einer Einfangsonde mit dem wenigstens einen Probenindizierungsoligonukleotid, so dass eine an das Probenindizierungsoligonukleotid hybridisierte Einfangsonde erzeugt wird; und Verlängern der an das Probenindizierungsoligonukleotid hybridisierten Einfangsonde zur Erzeugung eines mit der Einfangsonde assoziierten Probenindizierungsoligonukleotids, und wobei Replizieren des mindestens einen Probenindizierungsoligonukleotids Replizieren des mit der Einfangsonde assoziierten Probenindizierungsoligonukleotids umfasst, so dass die mehreren replizierten Probenindizierungsoligonukleotide erzeugt werden.

3. Mehrere Probenindizierungszusammensetzungen,
wobei die mehreren Probenindizierungszusammensetzungen jeweils eine Aptamerzusammensetzung, die ein erstes zellkomponentenbindendes Aptamer und ein Probenindizierungsoligonukleotid umfasst, umfassen,
wobei ein einzelnes Polynukleotid das Probenindizierungsoligonukleotid und das Aptamer umfasst,
wobei das zellkomponentenbindende Aptamer in der Lage ist, an mindestens ein Zellkomponentenziel spezifisch zu binden,
wobei das Probenindizierungsoligonukleotid eine Probenindizierungssequenz zum Identifizieren von Probenursprung für eine oder mehrere Zellen einer Probe umfasst,
wobei Probenindizierungssequenzen von mindestens zwei Probenindizierungszusammensetzungen der mehreren Probenindizierungszusammensetzungen unterschiedliche Sequenzen umfassen und
wobei die Aptamerzusammensetzung Folgendes umfasst:
ein zweites zellkomponentenbindendes Aptamer, das in der Lage ist, an mindestens ein Zellkomponentenziel zu binden, und
ein zweites Probenindizierungsoligonukleotid, das eine zweite Probenindizierungssequenz umfasst,
wobei die Zellkomponentenziele des zellkomponentenbindenden Aptamers und des zweiten zellkomponentenbindenden Aptamers unterschiedlich sind und
wobei die Probenindizierungssequenz und die zweite Probenindizierungssequenz identisch sind.

4. Verfahren nach einem der Ansprüche 1-2 oder mehrere Probenindizierungszusammensetzungen nach Anspruch 3, wobei:
(a) das Zellkomponentenziel ein Proteinziel umfasst;
(b) die Probenindizierungssequenz eine Länge von 6-60 Nukleotiden oder 50-500 Nukleotiden aufweist;
(c) Probenindizierungssequenzen von mindestens 10, 100 oder 1000 Probenindizierungszusammensetzungen der mehreren Probenindizierungszusammensetzungen unterschiedliche Sequenzen umfassen;
(d) das Aptamer sich:
(i) 5' zum Probenindizierungsoligonukleotid in dem einzelnen Polynukleotid befindet; oder
(ii) 3' zum Probenindizierungsoligonukleotid in dem einzelnen Polynukleotid befindet; und/oder
(e) das Aptamer ein Nukleotid-Aptamer umfasst, gegebenenfalls das Nukleotid-Aptamer Desoxyribonukleinsäure (DNA), Ribonukleinsäure (RNA), Xeno-Nukleinsäure (XNA), ein Fluorophor oder eine Kombination davon umfasst, gegebenenfalls wobei das Nukleotid-Aptamer ein Basenanalog umfasst und gegebenenfalls das Basenanalog ein Fluoreszenzbasenanalog umfasst.

5. Verfahren nach einem der Ansprüche 1-2 oder 4 oder mehrere Probenindizierungszusammensetzungen nach Anspruch 3 oder 4, wobei:
(a) die Probenindizierungszusammensetzung mit einem ersten Träger assoziiert ist;
(b) die Aptamerzusammensetzung mit einem ersten Träger assoziiert ist; und/oder
(c) wobei:
(i) das Aptamer und das zweite Aptamer mit einem ersten Träger assoziiert sind; oder
(ii) das Aptamer mit einem ersten Träger und das zweite Aptamer mit einem zweiten Träger assoziiert ist.

6. Verfahren oder mehrere Probenindizierungszusammensetzungen nach Anspruch 4 oder 5, wobei:
(a) der erste Träger Folgendes umfasst:
(i) Metall-Nanomaterial, und gegebenenfalls das Metall-Nanomaterial eine Metall-Nanostruktur, ein Metall-Nanopartikel oder eine Kombination davon umfasst; und/oder
(ii) Gold-Nanomaterial, und gegebenenfalls es sich bei dem Gold-Nanomaterial um eine Gold-Nanostruktur, ein Gold-Nanopartikel oder eine Kombination davon handelt; und/oder
(iii) ein Lysosom, eine Micelle, ein Vesikel, eine Lipidmembran, eine Lipiddoppelschicht, eine Lipideinzelschicht oder eine Kombination davon;
(b) das Aptamer an den ersten Träger gebunden ist und gegebenenfalls das Aptamer kovalent an den ersten Träger gebunden ist;
(c) das Aptamer an den ersten Träger konjugiert ist und gegebenenfalls das Aptamer an den ersten Träger über eine chemische Gruppe, ausgewählt aus der Gruppe bestehend aus einer UV-photospaltbaren Gruppe, einem Streptavidin, einem Biotin, einem Amin und einer Kombination davon, konjugiert ist;
(d) das Aptamer mit dem ersten Träger nichtkovalent verknüpft ist;
(e) das Aptamer über einen Linker mit dem ersten Träger assoziiert ist; und/oder
(f) das Aptamer am ersten Träger immobilisiert, am ersten Träger teilimmobilisiert, im ersten Träger immobilisiert, im ersten Träger teilimmobilisiert, im ersten Träger eingeschlossen, im ersten Träger teileingeschlossen, im ersten Träger eingebettet, im ersten Träger teileingebettet ist oder es sich um eine Kombination davon handelt.

7. Verfahren nach einem der Ansprüche 5(c) oder 6,
(a) umfassend Dissoziieren des Aptamers vom ersten Träger, gegebenenfalls wobei das Dissoziieren erfolgt:
(i) nachdem die Probenindizierungsoligonukleotide strichcodiert wurden; oder
(ii) bevor die Probenindizierungsoligonukleotide strichcodiert werden; und/oder
(b) wobei In-Kontakt-Bringen jeder der mehreren Proben mit der Probenindizierungszusammensetzung In-Kontakt-Bringen einer Zelle der einen oder mehreren Zellen der Probe mit der Probenindizierungszusammensetzung umfasst, gegebenenfalls wobei der erste Träger in die Zelle internalisiert wird, gegebenenfalls die Internalisierung über Endozytose, Pinozytose, Nanopinozytose, Mikropinozytose, Phagozytose, Membranfusion, clathrinvermittelte Internalisierung, caveolinvermittelte Internalisierung, rezeptorabhängige Internalisierung, rezeptorunabhängige Internalisierung oder eine Kombination davon erfolgt.

8. Verfahren nach einem der Ansprüche 1-2 oder 4-7, wobei:
(a) sich das wenigstens eine der ein oder mehreren Proteinziele oder der ein oder mehreren Zellkomponentenziele auf einer Zelloberfläche befindet;
(b) das Verfahren Entfernen nicht gebundener Probenindizierungszusammensetzungen der mehreren Probenindizierungszusammensetzungen umfasst;
(c) Entfernen der nicht gebundenen Probenindizierungszusammensetzungen (a) Waschen der einen oder mehreren Zellen aus jeder der mehreren Proben mit einem Waschpuffer, (b) Selektieren von an wenigstens ein Aptamer gebundenen Zellen unter Verwendung von Durchflusszytometrie oder beides umfasst; und/oder
(d) das Verfahren Lysieren der einen oder mehreren Zellen aus jeder der mehreren Proben umfasst.

9. Verfahren nach einem der Ansprüche 1-2 oder 4-8 oder mehrere Probenindizierungszusammensetzungen nach einem der Ansprüche 3-6, wobei:
(a) das Probenindizierungsoligonukleotid nicht homolog zu genomischen Sequenzen irgendeiner der einen oder mehreren Zellen ist, homolog zu genomischen Sequenzen einer Spezies ist, oder eine Kombination davon, und es sich gegebenenfalls bei der Spezies um eine Nichtsäuger-Spezies handelt;
(b) eine Probe der mehreren Proben mehrere Zellen, mehrere Einzelzellen, ein Gewebe, eine Tumorprobe oder eine beliebige Kombination davon umfasst;
(c) die mehreren Proben eine Säugerzelle, eine Bakterienzelle, eine Viruszelle, eine Hefezelle, eine Pilzzelle oder eine beliebige Kombination davon umfassen;
(d) das Probenindizierungsoligonukleotid eine Sequenz umfasst, die komplementär zu einer Einfangsequenz ist, die so konfiguriert ist, dass die Sequenz des Probenindizierungsoligonukleotids eingefangen wird, gegebenenfalls wobei:
(i) der Strichcode eine zielbindende Region umfasst, die die Einfangsequenz umfasst, und gegebenenfalls die zielbindende Region eine Poly(dT)-Region umfasst; oder
(ii) die Sequenz des Probenindizierungsoligonukleotids, das zu der Einfangsequenz komplementär ist, eine Poly(dA)-Region umfasst, gegebenenfalls wobei das Probenindizierungsoligonukleotid eine der Poly(dA)-Region benachbarte Ausrichtungssequenz umfasst und gegebenenfalls (a) die Ausrichtungssequenz eine Länge von einem oder mehr Nukleotiden oder zwei oder mehr Nukleotiden aufweist, (b) die Ausrichtungssequenz ein Guanin, ein Cytosin, ein Thymin, ein Uracil oder eine Kombination davon umfasst und/oder (c) die Ausrichtungssequenz eine Poly(dT)-Region, eine Poly(dG)-Region, eine Poly(dC)-Region, eine Poly(dU)-Region oder eine Kombination davon umfasst; und/oder
(e) das Probenindizierungsoligonukleotid eine Molekülmarkierungssequenz, eine Bindungsstelle für einen Universalprimer oder beide umfasst und gegebenenfalls die Molekülmarkierungssequenz eine Länge von 2-20 Nukleotiden oder 5-50 Nukleotiden aufweist und gegebenenfalls der Universalprimer einen Amplifikationsprimer, einen Sequenzierprimer oder eine Kombination davon umfasst;
(f) das Proteinziel oder das Zellkomponentenziel ein Kohlenhydrat, ein Lipid, ein Protein, ein extrazelluläres Protein, ein Zelloberflächenprotein, einen Zellmarker, einen B-Zell-Rezeptor, einen T-Zell-Rezeptor, einen Haupthistokompatibilitätskomplex, ein Tumorantigen, einen Rezeptor, ein intrazelluläres Protein oder eine beliebige Kombination davon umfasst;
(g) das Proteinziel oder das Zellkomponentenziel aus einer Gruppe ausgewählt ist, die 10-100 unterschiedliche Proteinziele bzw. Zellkomponentenziele umfasst; und/oder
(h) das Aptamer mit zwei oder mehr Probenindizierungsoligonukleotiden mit:
(A) einer identischen Sequenz; oder
(B) unterschiedlichen Probenindizierungssequenzen assoziiert ist und/oder
(i) die Probenindizierungszusammensetzung der mehreren Probenindizierungszusammensetzungen ein Aptamer umfasst, das nicht mit dem Probenindizierungsoligonukleotid assoziiert ist; und/oder
(j) jede der mehreren Probenindizierungszusammensetzungen das Aptamer umfasst; und/oder
(k) das Aptamer oder das zellkomponentenbindende Aptamer in der Lage ist, an ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2 oder eine beliebige Kombination davon zu binden; und/oder
(l) das zweite Aptamer oder das zweite zellkomponentenbindende Aptamer in der Lage ist, an ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2 oder eine beliebige Kombination davon zu binden.

10. Verfahren zum Messen der Expression von Zellkomponenten in Zellen, umfassend:
In-Kontakt-Bringen mehrerer zellkomponentenbindender Aptamere mit mehreren Zellen, die mehrere Zellkomponentenziele umfassen, wobei die mehreren zellkomponentenbindenden Aptamere jeweils ein aptamerspezifisches Oligonukleotid umfassen, das eine einmalige Identifikatorsequenz für das zellkomponentenbindende Aptamer umfasst, wobei das aptamerspezifische Oligonukleotid und das Aptamer ein einziges Polynukleotid bilden und wobei das zellkomponentenbindende Aptamer in der Lage ist, an mindestens eines der mehreren Zellkomponentenziele spezifisch zu binden, und wobei die mehreren zellkomponentenbindenden Aptamere ein zweites zellkomponentenbindendes Aptamer umfassen, das nicht an ein Oligonukleotid konjugiert ist;
Verlängern von an die aptamerspezifischen Oligonukleotide hybridisierten Oligonukleotidsonden, so dass mehrere markierte Nukleinsäuren erzeugt werden, wobei die markierten Nukleinsäuren jeweils eine einmalige Identifikatorsequenz oder eine Komplementärsequenz davon sowie eine Strichcodesequenz umfassen; und
Gewinnen von Sequenzinformationen der mehreren markierten Nukleinsäuren oder eines Teils davon zur Bestimmung der Menge eines oder mehrerer der mehreren Zellkomponentenziele in einer oder mehreren der mehreren Zellen.

11. Verfahren nach Anspruch 10, umfassend vor dem Verlängern der Oligonukleotidsonden:
Unterteilen der mehreren mit den mehreren zellkomponentenbindenden Aptameren assoziierten Zellen in mehrere Unterteilungen, wobei eine Unterteilung der mehreren Unterteilungen eine einzelne Zelle aus den mehreren mit den mehreren zellkomponentenbindenden Aptameren assoziierten Zellen umfasst; und
in der die einzelne Zelle umfassenden Unterteilung In-Kontakt-Bringen eines Strichcodierungspartikels mit den aptamerspezifischen Oligonukleotiden, wobei das Strichcodierungspartikel mehrere Oligonukleotidsonden umfasst, die jeweils eine Zielbindungsregion und eine Strichcodesequenz, die aus einem vielfältigen Satz einmaliger Strichcodesequenzen ausgewählt ist, umfassen.

12. Zusammensetzung, umfassend:
mehrere zellkomponentenbindende Aptamere, wobei die mehreren zellkomponentenbindenden Aptamere jeweils ein aptamerspezifisches Oligonukleotid umfassen, das eine einmalige Identifikatorsequenz für das zellkomponentenbindende Aptamer umfasst, wobei das aptamerspezifische Oligonukleotid und das Aptamer ein einziges Polynukleotid bilden, wobei das zellkomponentenbindende Aptamer in der Lage ist, an mindestens eines von mehreren Zellkomponentenzielen spezifisch zu binden, und wobei die mehreren zellkomponentenbindenden Aptamere ein zweites zellkomponentenbindendes Aptamer umfassen, das nicht an ein Oligonukleotid konjugiert ist.

13. Verfahren nach einem der Ansprüche 10-11 oder Zusammensetzung nach Anspruch 12, wobei:
(a) die mehreren Zellkomponentenziele mehrere Proteinziele umfassen und wobei das zellkomponentenbindende Aptamer in der Lage ist, an mindestens eines der mehreren Proteinziele spezifisch zu binden; und/oder
b) das Aptamer sich:
(i) 5' zum aptamerspezifischen Oligonukleotid in dem einzelnen Polynukleotid befindet; oder
(ii) 3' zum aptamerspezifischen Oligonukleotid in dem einzelnen Polynukleotid befindet; und/oder
(c) das Aptamer ein Nukleotid-Aptamer umfasst und gegebenenfalls das Nukleotid-Aptamer Desoxyribonukleinsäure (DNA), Ribonukleinsäure (RNA), Xenonukleinsäure (XNA), ein Fluorophor oder eine Kombination davon umfasst, gegebenenfalls wobei das Nukleotid-Aptamer ein Basenanalog umfasst, gegebenenfalls das Basenanalog ein Fluoreszenzbasenanalog umfasst.

14. Verfahren nach einem der Ansprüche 10-11 oder 13 oder Zusammensetzung nach Anspruch 12 oder 13, wobei:
(a) das Aptamer:
(i) und das zweite Aptamer mit einem ersten Träger assoziiert sind; oder
(ii) mit einem ersten Träger assoziiert ist und wobei das zweite Aptamer mit einem zweiten Träger assoziiert ist; und/oder
(b) das Aptamer und das zweite Aptamer eine Sequenzidentität von mindestens 60 %, 70 %, 80 %, 90 % oder 95 % aufweisen; und/oder
(c) das Aptamer und das zweite proteinbindende Aptamer:
(i) identisch sind; oder
(ii) verschieden sind, gegebenenfalls wobei:
die Proteinziele oder die Zellkomponentenziele des Aptamers und des zweiten Aptamers identisch sind; und/oder
(d) der erste Träger
(i) Metall-Nanomaterial umfasst und gegebenenfalls es sich bei dem Metall-Nanomaterial um eine Metall-Nanostruktur, ein Metall-Nanopartikel oder eine Kombination davon handelt; und/oder
(ii) Gold-Nanomaterial umfasst und gegebenenfalls es sich bei dem Gold-Nanomaterial um eine Gold-Nanostruktur, ein Gold-Nanopartikel oder eine Kombination davon handelt; und/oder
(iii) ein Lysosom, eine Micelle, ein Vesikel, eine Lipidmembran, eine Lipiddoppelschicht, eine Lipideinzelschicht oder eine Kombination davon umfasst; und/oder
(e) das Aptamer an den ersten Träger gebunden ist und gegebenenfalls das Aptamer kovalent an den ersten Träger gebunden ist;
(f) das Aptamer an den ersten Träger konjugiert ist und gegebenenfalls das Aptamer an den ersten Träger über eine chemische Gruppe, ausgewählt aus der Gruppe bestehend aus einer UV-photospaltbaren Gruppe, einem Streptavidin, einem Biotin, einem Amin und einer Kombination davon, konjugiert ist;
(g) das Aptamer mit dem ersten Träger nichtkovalent verknüpft ist;
(h) das Aptamer über einen Linker mit dem ersten Träger assoziiert ist; und/oder
(i) das Aptamer am ersten Träger immobilisiert, am ersten Träger teilimmobilisiert, im ersten Träger immobilisiert, im ersten Träger teilimmobilisiert, im ersten Träger eingeschlossen, im ersten Träger teileingeschlossen, im ersten Träger eingebettet, im ersten Träger teileingebettet ist oder es sich um eine Kombination davon handelt.

15. Verfahren oder Zusammensetzung nach Anspruch 14, wobei das aptamerspezifische Oligonukleotid eine Sequenz umfasst, die komplementär zu einer zum Einfangen der Sequenz des aptamerspezifischen Oligonukleotids konfigurierten Einfangsequenz ist, gegebenenfalls wobei:
(a) der Strichcode eine zielbindende Region umfasst, die die Einfangsequenz umfasst, und gegebenenfalls wobei die zielbindende Region eine Poly(dT)-Region umfasst; und/oder
(b) die Sequenz des aptamerspezifischen Oligonukleotids, die zur Einfangsequenz komplementär ist, eine Poly(dA)-Region umfasst, gegebenenfalls wobei:
(i) das aptamerspezifische Oligonukleotid von einer ersten Konformation, in der die Poly(dA)-Region unzugänglich ist, zu einer zweiten Konformation, in der die Poly(dA)-Region zugänglich ist, wechselt, wenn das Aptamer mit dem mindestens einen der mehreren Proteinziele oder Zellkomponentenziele in Kontakt kommt, gegebenenfalls wobei die Poly(dA)-Region des aptamerspezifischen Oligonukleotids in der ersten Konformation eine Haarnadelstruktur umfasst und gegebenenfalls die Poly(dA)-Region mit der Haarnadelstruktur für die Poly(dT)-Region der zielbindenden Region des Strichcodes zugänglich ist;
(ii) die Oligonukleotidsonden an die aptamerspezifischen Oligonukleotide über die Hybridisierung zwischen den Poly(dA)-Regionen der aptamerspezifischen Oligonukleotide und den Poly(dT)-Regionen der Oligonukleotidsonden hybridisiert sind; und/oder
(iii) das aptamerspezifische Oligonukleotid eine der Poly(dA)-Region benachbarte Ausrichtungssequenz umfasst und gegebenenfalls (a) die Ausrichtungssequenz eine Länge von einem oder mehr Nukleotiden oder zwei oder mehr Nukleotiden aufweist, (b) die Ausrichtungssequenz ein Guanin, ein Cytosin, ein Thymin, ein Uracil oder eine Kombination davon umfasst und/oder (c) die Ausrichtungssequenz eine Poly(dT)-Region, eine Poly(dG)-Region, eine Poly(dC)-Region, eine Poly(dU)-Region oder eine Kombination davon umfasst; und/oder
(c) das aptamerspezifische Oligonukleotid eine Molekülmarkierungssequenz, eine Bindungsstelle für einen Universalprimer oder beide umfasst und gegebenenfalls die Molekülmarkierungssequenz eine Länge von 2-20 Nukleotiden oder/und der Universalprimer eine Länge von 5-50 Nukleotiden aufweist, gegebenenfalls wobei der Universalprimer einen Amplifikationsprimer, einen Sequenzierprimer oder eine Kombination davon umfasst.

## Revendications

1. Procédé pour une identification d'échantillon, comprenant :
la mise en contact de chacun d'une pluralité d'échantillons avec une composition d'indexation d'échantillon parmi une pluralité de compositions d'indexation d'échantillon, respectivement,
dans lequel chacun de la pluralité d'échantillons comprend une ou plusieurs cellules comprenant chacune une ou plusieurs cibles de composant cellulaire, la composition d'indexation d'échantillon comprenant une composition d'aptamère comprenant un aptamère et un oligonucléotide d'indexation d'échantillon, un polynucléotide unique comprenant l'oligonucléotide d'indexation d'échantillon et l'aptamère, l'aptamère étant capable de se lier spécifiquement à au moins une des une ou plusieurs cibles de composant cellulaire, dans lequel la composition d'indexation d'échantillon de la pluralité de compositions d'indexation d'échantillon comprend une seconde composition d'aptamère comprenant un second aptamère, le second aptamère étant capable de se lier spécifiquement à au moins une des une ou plusieurs cibles de composant cellulaire, le second aptamère étant associé à un second oligonucléotide d'indexation d'échantillon comprenant une seconde séquence d'indexation d'échantillon, et dans lequel l'aptamère et le second aptamère sont capables de se lier à différentes cibles de composant cellulaire de la ou des cibles de composants cellulaires,
dans lequel l'oligonucléotide d'indexation d'échantillon comprend une séquence d'indexation d'échantillon, les séquences d'indexation d'échantillon d'au moins deux compositions d'indexation d'échantillon de la pluralité de compositions d'indexation d'échantillon comprenant des séquences différentes, et dans lequel la séquence d'indexation d'échantillon et la seconde séquence d'indexation d'échantillon sont identiques ; et
l'identification de l'origine de l'échantillon d'au moins une cellule parmi la ou les cellules sur la base de la séquence d'indexation d'échantillon d'au moins un oligonucléotide d'indexation d'échantillon parmi la pluralité de compositions d'indexation d'échantillon.

2. Procédé selon la revendication 1, dans lequel :
(a) l'identification de l'origine de l'échantillon de l'au moins une cellule comprend :
le codage à barres d'oligonucléotides d'indexation d'échantillon de la pluralité de compositions d'indexation d'échantillon en utilisant une pluralité de codes-barres pour générer une pluralité d'oligonucléotides d'indexation d'échantillon à code-barres ;
l'obtention de données de séquençage de la pluralité d'oligonucléotides d'indexation d'échantillon à code-barres ; et
l'identification de l'origine de l'échantillon de la cellule sur la base de la séquence d'indexation d'échantillon d'au moins un oligonucléotide d'indexation d'échantillon à code-barres de la pluralité d'oligonucléotides d'indexation d'échantillon à code-barres dans les données de séquençage ;
(b) l'identification de l'origine de l'échantillon de l'au moins une cellule comprend l'identification de la présence ou de l'absence de la séquence d'indexation d'échantillon d'au moins un oligonucléotide d'indexation d'échantillon de la pluralité de compositions d'indexation d'échantillon, éventuellement l'identification de la présence ou de l'absence de la séquence d'indexation d'échantillon comprenant :
la réplication de l'au moins un oligonucléotide d'indexation d'échantillon pour générer une pluralité d'oligonucléotides d'indexation d'échantillon répliqués ;
l'obtention de données de séquençage de la pluralité d'oligonucléotides d'indexation d'échantillon répliqués ; et
l'identification de l'origine de l'échantillon de la cellule sur la base de la séquence d'indexation d'échantillon d'un oligonucléotide d'indexation d'échantillon répliqué de la pluralité d'oligonucléotides d'indexation d'échantillon correspondant à l'au moins un oligonucléotide d'indexation d'échantillon à code-barres dans les données de séquençage ; et éventuellement la réplication de l'au moins un oligonucléotide d'indexation d'échantillon pour générer la pluralité d'oligonucléotides d'indexation d'échantillon répliqués comprenant :
(i) avant de répliquer l'au moins un oligonucléotide d'indexation d'échantillon à code-barres, ligaturer un adaptateur de réplication à l'au moins un oligonucléotide d'indexation d'échantillon à code-barres, et dans lequel la réplication de l'au moins un oligonucléotide d'indexation d'échantillon à code-barres comprend la réplication de l'au moins un oligonucléotide d'indexation d'échantillon à code-barres au moyen de l'adaptateur de réplication ligaturé à l'au moins un oligonucléotide d'indexation d'échantillon à code-barres pour générer la pluralité d'oligonucléotides d'indexation d'échantillon répliqués ; ou
(ii) avant de répliquer l'au moins un oligonucléotide d'indexation d'échantillon à code-barres, mettre en contact une sonde de capture avec l'au moins un oligonucléotide d'indexation d'échantillon afin de générer une sonde de capture hybridée à l'oligonucléotide d'indexation d'échantillon ; et étendre la sonde de capture hybridée à l'oligonucléotide d'indexation d'échantillon pour générer un oligonucléotide d'indexation d'échantillon associé à la sonde de capture, et dans lequel la réplication de l'au moins un oligonucléotide d'indexation d'échantillon comprend la réplication de l'oligonucléotide d'indexation d'échantillon associé à la sonde de capture pour générer la pluralité d'oligonucléotides d'indexation d'échantillon répliqués.

3. Pluralité de compositions d'indexation d'échantillon, dans laquelle chacune de la pluralité de compositions d'indexation d'échantillon comprend une composition d'aptamère comprenant un premier aptamère de liaison à un composant cellulaire et un oligonucléotide d'indexation d'échantillon,
dans laquelle un polynucléotide unique comprend l'oligonucléotide d'indexation d'échantillon et l'aptamère,
dans laquelle l'aptamère de liaison à un composant cellulaire est capable de se lier spécifiquement à au moins une cible de composant cellulaire,
dans laquelle l'oligonucléotide d'indexation d'échantillon comprend une séquence d'indexation d'échantillon pour identifier l'origine de l'échantillon d'une ou plusieurs cellules d'un échantillon,
dans laquelle des séquences d'indexation d'échantillon d'au moins deux compositions d'indexation d'échantillon de la pluralité de compositions d'indexation d'échantillon comprennent des séquences différentes, et dans laquelle la composition d'aptamère comprend :
un second aptamère de liaison à un composant cellulaire capable de se lier spécifiquement à au moins une cible de composant cellulaire, et
un second oligonucléotide d'indexation d'échantillon comprenant une seconde séquence d'indexation d'échantillon,
dans laquelle les cibles de composant cellulaire de l'aptamère de liaison à un composant cellulaire et du second aptamère de liaison à un composant cellulaire sont différentes, et
dans laquelle la séquence d'indexation d'échantillon et la seconde séquence d'indexation d'échantillon sont identiques.

4. Procédé selon l'une quelconque des revendications 1 et 2, ou pluralité de compositions d'indexation d'échantillon selon la revendication 3, dans lequel/laquelle :
(a) la cible de composant cellulaire comprend une cible protéique ;
(b) la séquence d'indexation d'échantillon a une longueur de 6-60 nucléotides ou une longueur de 50-500 nucléotides ;
(c) des séquences d'indexation d'échantillon d'au moins 10, 100 ou 1 000 compositions d'indexation d'échantillon de la pluralité de compositions d'indexation d'échantillon comprennent des séquences différentes ;
(d) l'aptamère est :
(i) en 5' par rapport à l'oligonucléotide d'indexation d'échantillon dans le polynucléotide unique ; ou
(ii) en 3' par rapport à l'oligonucléotide d'indexation d'échantillon dans le polynucléotide unique ; et/ou
(e) l'aptamère comprend un aptamère nucléotidique, éventuellement l'aptamère nucléotidique comprend un acide désoxyribonucléique (ADN), un acide ribonucléique (ARN), un acide xéno nucléique (AXN), un fluorophore, ou une combinaison de ceux-ci, éventuellement dans lequel/laquelle l'aptamère nucléotidique comprend un analogue de base, et éventuellement l'analogue de base comprend un analogue de base fluorescent.

5. Procédé selon l'une quelconque des revendications 1-2 ou 4, ou pluralité de compositions d'indexation d'échantillon selon la revendication 3 ou 4, dans lequel/laquelle :
(a) la composition d'indexation d'échantillon est associée à un premier support ;
(b) la composition d'aptamère est associée à un premier support ; et/ou
(c) dans lequel/laquelle :
(i) l'aptamère et le second aptamère sont associés à un premier support ; ou
(ii) l'aptamère est associé à un premier support et le second aptamère est associé à un second support.

6. Procédé ou pluralité de compositions d'indexation d'échantillon selon la revendication 4 ou 5, dans lequel/laquelle :
(a) le premier support comprend :
(i) un nanomatériau métallique, et éventuellement le nanomatériau métallique comprend une nanostructure métallique, une nanoparticule métallique, ou une combinaison de celles-ci ; et/ou
(ii) un nanomatériau d'or, et éventuellement le nanomatériau d'or est une nanostructure d'or, une nanoparticule d'or, ou une combinaison de celles-ci ; et/ou
(iii) un lysosome, une micelle, une vésicule, une membrane lipidique, une bicouche lipidique, une monocouche lipidique, ou une combinaison de ceux--ci ;
(b) l'aptamère est fixé au premier support et, éventuellement, l'aptamère est fixé de manière covalente au premier support ;
(c) l'aptamère est conjugué au premier support, et éventuellement l'aptamère est conjugué au premier support par l'intermédiaire d'un groupe chimique choisi dans le groupe constitué par un groupe photoclivable par des UV, une streptavidine, une biotine, une amine et une combinaison de ceux-ci ;
(d) l'aptamère est lié de manière non covalente au premier support ;
(e) l'aptamère est associé au premier support par l'intermédiaire d'un lieur ; et/ou
(f) l'aptamère est immobilisé sur le premier support, partiellement immobilisé sur le premier support, immobilisé dans le premier support, partiellement immobilisé dans le premier support, enfermé dans le premier support, partiellement enfermé dans le premier support, intégré dans le premier support, partiellement intégré dans le premier support, ou une combinaison de ceux-ci.

7. Procédé selon l'une quelconque des revendications 5(c) ou 6 :
(a) comprenant la dissociation de l'aptamère du premier support, éventuellement dans lequel la dissociation se produit :
(i) après le codage à barres des oligonucléotides d'indexation d'échantillon ; ou
(ii) avant le codage à barres des oligonucléotides d'indexation d'échantillon ; et/ou
(b) dans lequel la mise en contact de chacun de la pluralité d'échantillons avec la composition d'indexation d'échantillon comprend la mise en contact d'une cellule de la ou des cellules de l'échantillon avec la composition d'indexation d'échantillon, éventuellement dans lequel le premier support est internalisé dans la cellule, éventuellement l'internalisation se fait par endocytose, pinocytose, nanopinocytose, micropinocytose, phagocytose, fusion membranaire, internalisation médicamenteuse à la clathrine, internalisation médiée par la cavéoline, internalisation dépendante du récepteur, internalisation indépendante du récepteur, ou une combinaison de celles-ci.

8. Procédé selon l'une quelconque des revendications 1-2 ou 4-7, dans lequel :
(a) l'au moins une des une ou plusieurs cibles protéiques, ou la ou les cibles de composant cellulaire, se trouve(nt) sur une surface cellulaire ;
(b) le procédé comprend l'élimination de compositions d'indexation d'échantillon non liées parmi la pluralité de compositions d'indexation d'échantillon ;
(c) l'élimination des compositions d'indexation d'échantillon non liées comprend (a) le lavage de la ou des cellules de chacun de la pluralité d'échantillons avec un tampon de lavage, (b) la sélection de cellules liées à au moins un aptamère par cytométrie de flux, ou les deux ; et/ou
(d) le procédé comprend la lyse de la ou des cellules de chacun de la pluralité d'échantillons.

9. Procédé selon l'une quelconque des revendications 1-2 ou 4-8, ou pluralité de compositions d'indexation d'échantillon selon l'une quelconque des revendications 3-6, dans lequel/laquelle :
(a) l'oligonucléotide d'indexation d'échantillon n'est pas homologue à des séquences génomiques d'une quelconque cellule parmi les une ou plusieurs cellules, est homologue à des séquences génomiques d'une espèce, ou une combinaison correspondante, et éventuellement l'espèce est une espèce non mammifère ;
(b) un échantillon de la pluralité d'échantillons comprend une pluralité de cellules, une pluralité de cellules uniques, un tissu, un échantillon de tumeur ou une quelconque combinaison de ceux-ci ;
(c) la pluralité d'échantillons comprend une cellule de mammifère, une cellule bactérienne, une cellule virale, une cellule de levure, une cellule fongique, ou une quelconque combinaison de celles-ci ;
(d) l'oligonucléotide d'indexation d'échantillon comprend une séquence complémentaire d'une séquence de capture configurée pour capturer la séquence de l'oligonucléotide d'indexation d'échantillon, éventuellement dans lequel/laquelle :
(i) le code-barres comprend une région de liaison à une cible qui comprend la séquence de capture, et éventuellement la région de liaison à une cible comprend une région poly(dT) ; ou
(ii) la séquence de l'oligonucléotide d'indexation d'échantillon complémentaire de la séquence de capture comprend une région poly(da), éventuellement dans lequel/laquelle l'oligonucléotide d'indexation d'échantillon comprend une séquence d'alignement adjacente à la région poly(dA), et éventuellement (a) la séquence d'alignement a une longueur de un ou plusieurs nucléotides, ou une longueur de deux nucléotides ou plus, (b) la séquence d'alignement comprend une guanine, une cytosine, une thymine, un uracile ou une combinaison de ceux-ci, et/ou (c) la séquence d'alignement comprend une région poly(dT), une région poly(dG), une région poly(dC), une région poly(dU), ou une combinaison de celles-ci ; et/ou
(e) l'oligonucléotide d'indexation d'échantillon comprend une séquence de marquage moléculaire, un site de liaison pour une amorce universelle, ou les deux, et éventuellement la séquence de marquage moléculaire a une longueur de 2-20 nucléotides ou une longueur de 5-50 nucléotides, et éventuellement l'amorce universelle comprend une amorce d'amplification, une amorce de séquençage, ou une combinaison de celles-ci ;
(f) la cible protéique, ou la cible de composant cellulaire, comprend un glucide, un lipide, une protéine, une protéine extracellulaire, une protéine de surface cellulaire, un marqueur cellulaire, un récepteur de lymphocytes B, un récepteur de lymphocytes T, un complexe majeur d'histocompatibilité, un antigène tumoral, un récepteur, une protéine intracellulaire ou une quelconque combinaison de ceux-ci ;
(g) la cible protéique, ou la cible de composant cellulaire, est sélectionnée dans un groupe comprenant 10-100 cibles protéiques différentes, ou cibles de composant cellulaire différentes ; et/ou
(h) l'aptamère est associé à deux oligonucléotides d'indexation d'échantillon ou plus avec :
(A) une séquence identique ; ou
(B) des séquences d'indexation d'échantillon différentes et/ou
(i) la composition d'indexation d'échantillon de la pluralité de compositions d'indexation d'échantillon comprend un aptamère non associé à l'oligonucléotide d'indexation d'échantillon ; et/ou
(j) chacune de la pluralité de compositions d'indexation d'échantillon comprend l'aptamère ; et/ou
(k) l'aptamère, ou l'aptamère de liaison à un composant cellulaire, est capable de se lier à ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2, ou une quelconque combinaison de ceux-ci ; et/ou
(l) le second aptamère, ou le second aptamère de liaison à un composant cellulaire, est capable de se lier à ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2, ou une quelconque combinaison de ceux-ci.

10. Procédé de mesure d'une expression d'un composant cellulaire dans des cellules, comprenant :
la mise en contact d'une pluralité d'aptamères de liaison à un composant cellulaire avec une pluralité de cellules comprenant une pluralité de cibles de composant cellulaire, chacun de la pluralité d'aptamères de liaison à un composant cellulaire comprenant un oligonucléotide spécifique d'aptamère comprenant une séquence d'identification unique pour l'aptamère de liaison à un composant cellulaire, l'oligonucléotide spécifique d'aptamère et l'aptamère formant un polynucléotide unique, l'aptamère de liaison à un composant cellulaire étant capable de se lier spécifiquement à au moins une de la pluralité de cibles de composant cellulaire, et dans lequel la pluralité d'aptamères de liaison à un composant cellulaire comprend un second aptamère de liaison à un composant cellulaire non conjugué à un oligonucléotide ;
l'extension de sondes oligonucléotidiques hybridées aux oligonucléotides spécifiques d'aptamère pour produire une pluralité d'acides nucléiques marqués, chacun des acides nucléiques marqués comprenant une séquence d'identification unique, ou une séquence complémentaire de celle-ci, et une séquence de code-barres ; et
l'obtention d'informations de séquence de la pluralité d'acides nucléiques marqués, ou d'une partie correspondante pour déterminer la quantité d'un ou plusieurs parmi la pluralité de cibles de composant cellulaire dans une ou plusieurs parmi la pluralité de cellules.

11. Procédé selon la revendication 10, comprenant, avant l'extension des sondes oligonucléotidiques :
le partitionnement de la pluralité de cellules associées à la pluralité d'aptamères de liaison à un composant cellulaire en une pluralité de partitions, une partition de la pluralité de partitions comprenant une cellule unique parmi la pluralité de cellules associées aux aptamères de liaison à un composant cellulaire ;
dans la partition comprenant la cellule unique, la mise en contact d'une particule de codage à barres avec les oligonucléotides spécifiques d'aptamère, la particule de codage à barres comprenant une pluralité de sondes oligonucléotidiques comprenant chacune une région de liaison à une cible et une séquence de code-barres sélectionnée parmi un ensemble diversifié de séquences de code-barres uniques.

12. Composition comprenant :
une pluralité d'aptamères de liaison à un composant cellulaire, chacun de la pluralité d'aptamères de liaison à un composant cellulaire comprenant un oligonucléotide spécifique d'aptamère comprenant une séquence d'identification unique pour l'aptamère de liaison à un composant cellulaire, l'oligonucléotide spécifique d'aptamère et l'aptamère formant un polynucléotide unique, l'aptamère de liaison à un composant cellulaire étant capable de se lier spécifiquement à au moins une cible parmi une pluralité de cibles de composant cellulaire, et dans laquelle la pluralité d'aptamères de liaison à un composant cellulaire comprend un second aptamère de liaison à un composant cellulaire non conjugué à un oligonucléotide.

13. Procédé selon l'une quelconque des revendications 10-11 ou composition selon la revendication 12, dans lequel/laquelle :
(a) la pluralité de cibles de composant cellulaire comprend une pluralité de cibles protéiques, et dans lequel/laquelle l'aptamère de liaison à un composant cellulaire est capable de se lier spécifiquement à au moins une cible de la pluralité de cibles protéiques ; et/ou
(b) l'aptamère est :
(i) en 5' par rapport à l'oligonucléotide spécifique d'aptamère dans le polynucléotide unique ; ou
(ii) en 3' par rapport à l'oligonucléotide spécifique d'aptamère dans le polynucléotide unique ; et/ou
(c) l'aptamère comprend un aptamère nucléotidique, et éventuellement l'aptamère nucléotidique comprend un acide désoxyribonucléique (ADN), un acide ribonucléique (ARN), un acide xénonucléique (AXN), un fluorophore, ou une combinaison de ceux-ci, éventuellement l'aptamère nucléotidique comprenant un analogue de base, éventuellement l'analogue de base comprenant un analogue de base fluorescent.

14. Procédé selon l'une quelconque des revendications 10-11 ou 13, ou composition selon la revendication 12 ou 13, dans lequel/laquelle :
(a) l'aptamère :
(i) et le second aptamère sont associés à un premier support ; ou
(ii) est associé à un premier support, et dans lequel/laquelle le second aptamère est associé à un second support ; et/ou
(b) l'aptamère et le second aptamère sont au moins 60 %, 70 %, 80 %, 90 % ou 95 % identiques en séquence ; et/ou
(c) l'aptamère et le second aptamère de liaison à une protéine sont :
(i) identiques ; ou
(ii) différents, éventuellement dans lequel/laquelle :
les cibles protéiques, ou les cibles de composant cellulaire, de l'aptamère et du second aptamère sont identiques ; et/ou
(d) le premier support comprend
(i) un nanomatériau métallique, et éventuellement le nanomatériau métallique est une nanostructure métallique, une nanoparticule métallique ou une combinaison de celles-ci ; et/ou
(ii) un nanomatériau d'or, et éventuellement le nanomatériau d'or est une nanostructure d'or, une nanoparticule d'or, ou une combinaison de celles-ci ; et/ou
(iii) un lysosome, une micelle, une vésicule, une membrane lipidique, une bicouche lipidique, une monocouche lipidique, ou une combinaison de ceux-ci ; et/ou
(e) l'aptamère est fixé au premier support et, éventuellement, l'aptamère est fixé de manière covalente au premier support ;
(f) l'aptamère est conjugué au premier support, et éventuellement l'aptamère est conjugué au premier support par l'intermédiaire d'un groupe chimique choisi dans le groupe constitué par un groupe photoclivable par des UV, une streptavidine, une biotine, une amine et une combinaison de ceux-ci ;
(g) l'aptamère est lié de manière non covalente au premier support ;
(h) l'aptamère est associé au premier support par l'intermédiaire d'un lieur ; et/ou
(i) l'aptamère est immobilisé sur le premier support, partiellement immobilisé sur le premier support, immobilisé dans le premier support, partiellement immobilisé dans le premier support, enfermé dans le premier support, partiellement enfermé dans le premier support, intégré dans le premier support, partiellement intégré dans le premier support, ou une combinaison de ceux-ci.

15. Procédé ou composition selon la revendication 14, dans lequel/laquelle l'oligonucléotide spécifique d'aptamère comprend une séquence complémentaire d'une séquence de capture configurée pour capturer la séquence de l'oligonucléotide spécifique d'aptamère, éventuellement dans lequel/laquelle :
(a) le code-barres comprend une région de liaison à une cible qui comprend la séquence de capture, et éventuellement la région de liaison à une cible comprend une région poly(dT) ; et/ou
(b) la séquence de l'oligonucléotide spécifique d'aptamère complémentaire de la séquence de capture comprend une région poly(dA), éventuellement dans lequel/laquelle :
(i) l'oligonucléotide spécifique d'aptamère passe d'une première conformation dans laquelle la région poly(dA) est inaccessible à une seconde conformation dans laquelle la région poly(dA) est accessible lorsque l'aptamère entre en contact avec l'au moins une de la pluralité de cibles protéiques, ou cibles de composant cellulaire, éventuellement dans lequel/laquelle la région poly(dA) de l'oligonucléotide spécifique d'aptamère dans la première conformation comprend une structure en épingle à cheveux, et éventuellement la région poly(dA) avec la structure en épingle à cheveux est accessible à la région poly(dT) de la région de liaison à une cible du code-barres;
(ii) les sondes oligonucléotidiques sont hybridées aux oligonucléotides spécifiques d'aptamère par l'hybridation entre les régions poly(dA) des oligonucléotides spécifiques d'aptamère et les régions poly(dT) des sondes oligonucléotidiques ; et/ou
(iii) l'oligonucléotide spécifique d'aptamère comprend une séquence d'alignement adjacente à la région poly(dA), et éventuellement (a) la séquence d'alignement a une longueur de un ou plusieurs nucléotides, ou une longueur de deux nucléotides ou plus, (b) la séquence d'alignement comprend une guanine, une cytosine, une thymine, un uracile, ou une combinaison de ceux-ci, et/ou (c) la séquence d'alignement comprend une région poly(dT), une région poly(dG), une région poly(dC), une région poly(dU) ou une combinaison de celles-ci ; et/ou
(c) l'oligonucléotide spécifique d'aptamère comprend une séquence de marquage moléculaire, un site de liaison pour une amorce universelle, ou les deux, et éventuellement la séquence de marquage moléculaire a une longueur de 2-20 nucléotides, ou l'amorce universelle a une longueur de 5-50 nucléotides, ou les deux, l'amorce universelle comprenant éventuellement une amorce d'amplification, une amorce de séquençage, ou une combinaison de celles-ci.
